# EUROPEAN PATENT SPECIFICATION

(11) **EP 3 455 269 B1**
(45) Date of publication and mention of the grant of the patent: **20.09.2023**
(21) Application number: 17795688.5
(22) Date of filing: 10.05.2017
(51) Int. Cl.: C08G 18/24, C08G 18/40, A61B 5/145, A61B 5/1473, A61L 31/10, A61L 31/12, C08G 18/61, C08G 18/75, C09D 175/08, C08G 18/10, C08G 18/22, C08G 18/28, C08G 18/48, C08G 18/79, C09D 175/04

(54) **IMPLANTABLE GLUCOSE SENSORS HAVING A BIOSTABLE SURFACE**
IMPLANTIERBARE GLUCOSESENSOREN MIT EINER BIOSTABILEN OBERFLÄCHE
CAPTEURS DE GLUCOSE IMPLANTABLES À SURFACE BIOSTABLE

(30) Priority: 10.05.2016 US 201662334188 P
(43) Date of publication of application: 20.03.2019
(73) Proprietor: Evonik Canada Inc., L7N3J5 Burlington, ON (CA)
(72) Inventor: STEEDMAN, Mark, A., Toronto, ON M5M 3L1 (CA); HO, Jeannette, Toronto, ON M4K 1Z1 (CA); SWENOR, Jamie, Robert, Toronto, ON M4Y 2S2 (CA); MULLICK, Sanjoy, Brampton, ON L7A 1Y5 (CA)
(74) Representative: Evonik Patent Association
(86) International application number: PCT/IB2017/000636
(87) International publication number: WO 2017/195035

(56) References cited:
- EP-A1- 2 295 132
- WO-A1-2016/154034
- JP-B1- 5 702 857
- US-A1- 2008 195 232
- US-A1- 2008 275 318
- US-A1- 2011 271 961
- US-A1- 2014 275 900
- US-A1- 2014 275 900
- US-A1- 2015 025 198
- US-A1- 2015 157 248
- US-A1- 2015 157 248
- US-B2- 7 228 159

## Description

### Field of the Invention

The present invention relates to implantable glucose sensors having a biostable surface.

### Background

Numerous techniques for monitoring glucose levels in a subject have been developed. These techniques include implantable, minimally invasive, and non-invasive approaches.

For example, EP 2295132 A1 discloses extracorporeal blood circuits, and components thereof (e.g., hollow fiber membranes, potted bundles, and blood tubing), including 0.005% to 10% (w/w) surface modifying macromolecule including a polyfluoroorgano group. The extracorporeal blood circuits have an antithrombogenic surface and can be used in hemofiltration, hemodialysis, hemodiafiltration, hemoconcentration, blood oxygenation, and related uses.

US 2015/157248 A1 discloses a biointerface membrane for use with an implantable device that interferes with the formation of a barrier cell layer including; a first domain distal to the implantable device wherein the first domain supports tissue attachment and interferes with barrier cell layer formation and a second domain proximal to the implantable device wherein the second domain is resistant to cellular attachment and is impermeable to cells. In addition, the reference discloses sensors including the biointerface membrane, implantable devices including these sensors or biointerface membranes, and methods of monitoring glucose levels in a host utilizing the analyte detection implantable device according to the reference. Other implantable devices which include the biointerface membrane disclosed in the application, such as devices for cell transplantation, drug delivery devices, and electrical signal delivery or measuring devices are also provided.

US 2014/275900 A1 discloses a biointerface membrane for an implantable device including a nonresorbable solid portion with a plurality of interconnected cavities therein adapted to support tissue ingrowth in vivo, and a bioactive agent incorporated into the biointerface membrane and adapted to modify the tissue response. The bioactive agents can be chosen to induce vascularization and/or prevent barrier cell layer formation in vivo, and are advantageous when used with implantable devices wherein solutes are transported across the device-tissue interface.

Among these techniques, the implantable approaches are typically better suited for continuous monitoring of glucose levels in a subject, which allow for alerting a subject of an impending hypoglycemic or hyperglycemic even, thereby enabling the subject to avoid extreme hypoglycemic or hyperglycemic excursions and to minimize deviations outside the normal range of the glucose levels. Such real-time alerts can prevent both life-threatening events and the debilitating complications associated with diabetes.

Electrochemical detection of glucose is a particularly attractive glucose detection technique in the context of implantable glucose sensors, because of its specificity for glucose and high sensitivity.

However, for reasons of biocompatibility, practical implementation of electrochemical detection in implantable glucose sensors is complicated by the necessity, upon implantation into a subject, to shield an electrode and a glucose-oxidizing enzyme, if present, from the intracorporeal environment while maintaining the access of the electrode to glucose and, in some electrochemical detection approaches, oxygen. Typically the electrodes in implantable electrochemical glucose sensors are shielded from the intracorporeal environment through the use of an outer semipermeable membrane. Semipermeable membranes currently used in the implantable electrochemical glucose sensors are often susceptible to accumulation of proteins on the surface and the build-up of a barrier cell layer which hinders diffusion of glucose and oxygen to the electrode of an implantable electrochemical glucose sensor, thereby reducing the accuracy and lifetime of the implantable electrochemical glucose sensor. The reduction in the accuracy of the implantable electrochemical glucose sensors necessitates frequent recalibration of the sensor. Indeed, some manufacturers of commercially available implantable electrochemical glucose sensors recommend as many as three or four sensor recalibrations per day. The accuracy of an implantable electrochemical glucose sensor may be further exacerbated by the working electrode fouling associated with the presence of electrochemical interferents in a body of a subject. For example, agents, such as acetaminophen, salicylic acid, tetracycline, dopamine, ephedrine, ibuprofen, L-DOPA, methyl-DOPA, tolazamide, ascorbic acid, bilirubin, cholesterol, creatinine, triglycerides, and uric acid, are known to undergo oxidation at the working electrode which produces an interfering amperometric signal leads to an elevated glucose reading that does not reflect the actual glucose levels.

Another glucose detection technology currently utilized in implantable glucose sensors involves an optic detection of the glucose levels. Typically, implantable optical glucose sensors can also suffer from a reduction in their accuracy over time due to accumulation of proteins on the surface and the build-up of a barrier cell layer, which reduces the sensor's access to glucose.

Both the electrochemical and optic glucose detection technologies may also be susceptible to glucose detection inaccuracies associated with the reactive oxygen species (ROS) produced in a tissue as part of a foreign body response to the device implantation.

There is a need for implantable glucose sensors having a biostable surface.

### Summary of the Invention

In general, the invention features implantable glucose sensors. The implantable glucose sensors include a glucose detector and an enclosure defining a boundary between an internal space and an external space. The glucose detector is disposed in the internal space. The enclosure includes a semipermeable biointerface film containing a base polymer and a biostabilizing additive. The semipermeable biointerface film has a biostable surface and is permeable to glucose. The biostable surface faces the external space. In some embodiments, both opposing surfaces of the semipermeable biointerface film are biostable. Thus, a biostable surface of the semipermeable biointerface film may face both the internal space and the external space of the glucose sensor.

In a first aspect the present invention refers to a compound of formula (XX):

F_{T}-[B-A]ₙ-B-F_{T} (XX)

wherein
(i) A comprises 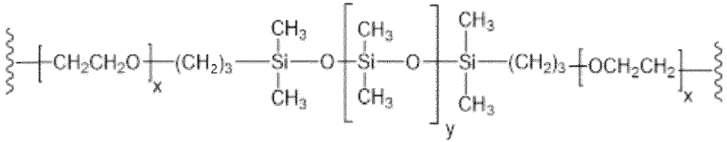
(ii) B is a segment including a urethane formed from 4,4'-methylene bis(cyclohexyl isocyanate);
(iii) F_{T} is a polyfluoroorgano group; and
(iv) x is an integer from 8 to 12, y is an integer from 6-9, and n is an integer from 1 to 10, optionally wherein n is 1 or 2;
a compound of formula (XXI):

F_{T}-[B-A]ₙ-B-F_{T} (XXI)

wherein
(i) A comprises a segment having the formula: 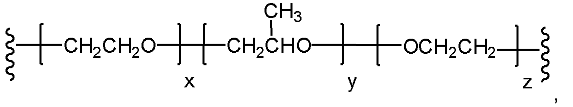 wherein said segment has a MW of 7,000 to 9,000 Da, comprises from 75% to 85% (w/w) polyethylene oxide, and comprises 15% to 25% (w/w) polypropylene oxide;
(ii) B is a segment including a urethane formed from 4,4'-methylene bis(cyclohexyl isocyanate);
(iii) F_{T} is a polyfluoroorgano group; and
(iv) n is an integer from 1 to 10,
   optionally wherein n is 1 or 2; or
a compound of formula (XXII): 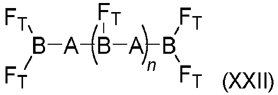 wherein
(i) A comprises a segment having the formula: 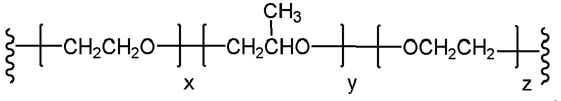 wherein said segment has a MW of 7,000 to 9,000 Da, comprises from 75% to 85% (w/w) polyethylene oxide, and comprises 15% to 25% (w/w) polypropylene oxide;
(ii) B is a segment including an isocyanurate trimer or biuret trimer formed from isophorone diisocyanate (IPDI) trimer;
(iii) F_{T} is a polyfluoroorgano group; and
(iv) n is an integer from 0 to 10.

In a second aspect the present invention relates to an implantable glucose sensor comprising: a glucose detector and an enclosure defining a boundary between an internal space and an external space, said enclosure comprising a semipermeable biointerface film comprising a base polymer and a biostabilizing additive comprising the compound according to the present invention;
wherein said semipermeable biointerface film has a biostable surface and is permeable to glucose; wherein said glucose detector is disposed inside said internal space, and said biostable surface faces said external space or both said internal space and said external space.

Preferred embodiments are set out in the dependent claims.

In some embodiments, the implantable glucose sensors of the invention have an *in vivo* working lifespan that is greater than the working lifespan of a reference sensor that differs from the implantable glucose sensor of the invention only by the absence of the biostabilizing additive in the reference sensor. For example, the working life enhancement the implantable glucose sensors of the invention may be by at least 5%, by at least 10%, by at least 20%, or by at least 50%, as compared to a reference implantable glucose sensor that differs from the implantable glucose sensor of the invention only by the absence of a biostabilizing additive.

In certain embodiments, the implantable glucose sensors of the invention exhibit a reduced mean absolute relative difference (MARD) in comparison to a reference sensor that differs from the implantable glucose sensor of the invention only by the absence of the biostabilizing additive in the reference sensor.

In further embodiments, the biostable surface exhibits reduced protein and cell deposition as compared to a reference film that differs from the semipermeable biointerface film only by the absence of the biostabilizing additive in the reference film.

In particular embodiments, the biostable surface exhibits substantially similar or enhanced aqueous wettability as compared to a reference film that differs from the semipermeable biointerface film only by the absence of the biostabilizing additive in the reference film.

In further embodiments, the semipermeable biointerface film has a thickness of from 1 to 1000 microns (e.g., from 1 to 200 microns, from 1 to 150 microns, from 1 to 100 microns, or from 1 to 50 microns).

In other embodiments, the semipermeable biointerface film contains from 0.05% (w/w) to 15% (w/w) (e.g., from 0.1% (w/w) to 10% (w/w), from 0.5% (w/w) to 10% (w/w), from 1% (w/w) to 10% (w/w), from 0.1% (w/w) to 5% (w/w), from 0.5% (w/w) to 5% (w/w), or from 1% (w/w) to 5% (w/w)) of the biostabilizing additive.

In yet other embodiments, the base polymer is a silicone, polyolefin, polyester, polycarbonate, polysulfone, polyamide, polyether, polyurea, polyurethane, polyetherimide, or cellulosic polymer, or a copolymer thereof or a blend thereof. In certain other embodiments, the base polymer is a silicone, polycarbonate, polypropylene (PP), polyvinylchloride (PVC), polyvinyl alcohol (PVA), polyvinylpyrrolidone (PVP), polyacrylamide (PAAM), polyethylene oxide, polyethylene oxide)-*b-*poly(propylene oxide)-b-poly(ethylene oxide), poly(hydroxyethylmethacrylate) (polyHEMA), polyethylene terephthalate (PET), polybutylene terephthalate (PBT), polymethylmethacrylate (PMMA), polyether ether ketone (PEEK), polyamide, polyurethane, cellulosic polymer, polysulfone, or a copolymer thereof or a blend thereof. In still other embodiments, the base polymer is polyvinylpyrrolidone (PVP), polyacrylamide (PAAM), polyethylene oxide, polyethylene oxide)-*b-*poly(propylene oxide)-*b*-poly(ethylene oxide), poly(hydroxyethylmethacrylate) (polyHEMA), polyether-*b*-polyamide, or polyurethane. In further embodiments, the base polymer is a thermoplastic.

According to the present invention the biostabilizing additive comprises a compound according to formula (XX), (XXI), or (XXII).

In other embodiments, the semipermeable biointerface film further contains one or more biologically active agents selected from the group consisting of anti-inflammatory agents, anti-infective agents, anesthetics, inflammatory agents, growth factors, angiogenic factors, growth factors, immunosuppressive agents, antiplatelet agents, anticoagulants, ACE inhibitors, cytotoxic agents, anti-sense molecules, and mixtures thereof.

In yet other embodiments, the implantable glucose sensor is an implantable electrochemical glucose sensor, and the glucose detector is a working electrode. In still other embodiments, the semipermeable biointerface film has a biostable surface and is permeable to oxygen. In certain other embodiments, the implantable glucose sensor includes a glucose-oxidizing enzyme layer disposed between the working electrode and the semipermeable biointerface film.

In yet other embodiments, the implantable glucose sensor is an implantable optical glucose sensor, and the glucose detector is a glucose recognition element containing a glucose-binding fluorophore.

In some embodiments, the semipermeable biointerface film is a bilayer film containing a biointerface coating and a membrane, where the biointerface coating includes the biostable surface, and the biointerface coating contains the biostabilizing additive. In further embodiments, the biointerface coating contains the base polymer. In certain embodiments, the membrane contains a second base polymer that is same or different as the base polymer in the coating. In particular embodiments, the membrane includes a biostabilizing additive.

In other embodiments, the semipermeable biointerface film is a monolayer membrane including the base polymer and the biostabilizing additive.

In yet other embodiments, the implantable glucose sensor is a subcutaneously implantable glucose sensor.

A method of monitoring glucose levels in a subject by (i) implanting the implantable glucose sensor of the invention into the subject, and (ii) detecting glucose in the subject is disclosed.

Furthermore, a method of preparing the implantable glucose sensor of the invention having a bilayer semipermeable biointerface film by coating a semipermeable membrane with a mixture containing a biostabilizing agent (e.g., containing a biostabilizing agent and a base polymer) is disclosed. The coating step may include, e.g., dip-coating or spray-coating.

Moreover, a method of preparing the implantable glucose sensor having a monolayer semipermeable biointerface film by forming the monolayer membrane from a mixture of a base polymer and a biostabilizing agent is disclosed. The forming step may include, e.g., solvent casting, molding, or spin casting.

The invention features a compound of formula (XX):

F_{T}-[B-A]ₙ-B-F_{T} (XX),

wherein, (i) A comprises 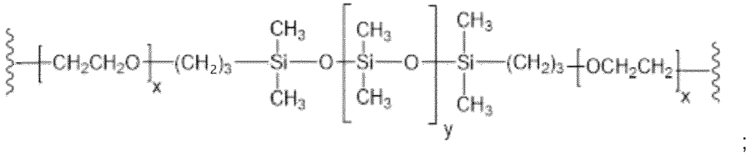 (ii) B is a segment including a urethane formed from 4,4'-methylene bis(cyclohexyl isocyanate); (iii) F_{T} is a polyfluoroorgano group; and (iv) x is an integer from 8 to 12, y is an integer from 6-9, and n is an integer from 1 to 10. In particular embodiments, n is 1 or 2. In still other embodiments, the compound of formula (XX) is compound 37 or compound 38.

The invention features a compound of formula (XXI):

F_{T}-[B-A]ₙ-B-F_{T} (XXI),

wherein, (i) A comprises a segment having the formula: 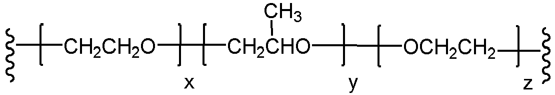 wherein said segment has a MW of 7,000 to 9,000 Da, includes from 75% to 85% (w/w) polyethylene oxide, and includes 15% to 25% (w/w) polypropylene oxide; (ii) B is a segment including a urethane formed from 4,4'-methylene bis(cyclohexyl isocyanate); (iii) F_{T} is a polyfluoroorgano group; and (iv) n is an integer from 1 to 10. In particular embodiments, n is 1 or 2. In some embodiments, A has an average MW of about 8,000 Da and includes about 80%(w/w) polyethylene oxide and about 20% (w/w) polypropylene oxide. In still other embodiments, the compound of formula (XX) is compound 40.

The invention features a compound of formula (XXII): 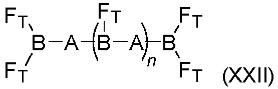 wherein, (i) A comprises a segment having the formula: 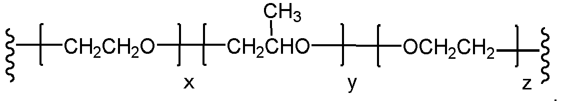 wherein said segment has a MW of 7,000 to 9,000 Da, includes from 75% to 85% (w/w) polyethylene oxide, and includes 15% to 25% (w/w) polypropylene oxide; (ii) B is a segment including an isocyanurate trimer or biuret trimer formed from isophorone diisocyanate (IPDI) trimer; (iii) F_{T} is a polyfluoroorgano group; and (iv) n is an integer from 0 to 10.

In an embodiment of any of the above compounds, F_{T} is selected from the group consisting of radicals of the general formula CHₘF₍₃₋ₘ₎(CF₂)ᵣCH₂CH₂- and CHₘF₍₃₋ₘ₎(CF₂)ₛ(CH₂CH₂O)ₓ-, wherein m is 0, 1, 2, or 3; χ is an integer between 1-10; r is an integer between 2-20; and s is an integer between 1-20. In certain embodiments, m is 0 or 1.

In another embodiment of any of the above compounds, the compound has a theoretical molecular weight of less than 40,000 Da, less than 20,000 Da, or less than 10,000 Da.

### Definitions

The term "about," as used herein, refers to a value that is ±20% of the recited number.

The term "barrier cell layer" as used herein is a broad term and is used in its ordinary sense, including, without limitation, to refer to a part of a foreign body response that can lead to the formation of a cohesive monolayer of cells (e.g., macrophages and foreign body giant cells) that substantially block the transport of molecules and other substances to the implantable device.

The term "base polymer," as used herein, refers to a polymer having a theoretical molecular weight of greater than or equal to 50 kDa (e.g., greater than or equal to 60 kDa, greater than or equal to 75 kDa, greater than or equal to 100 kDa, greater than or equal to 150 kDa, or greater than 200 kDa). Non-limiting examples of base polymers include: silicone, polyolefin, polyester, polycarbonate, polysulfone, polyamide, polyether, polyurea, polyurethane, polyetherimide, cellulosic polymer, and copolymers thereof, and blends thereof. Further non-limiting examples of the base polymers include a silicone, polycarbonate, polypropylene (PP), polyvinylchloride (PVC), polyvinyl alcohol (PVA), polyvinylpyrrolidone (PVP), polyacrylamide (PAAM), polyethylene oxide, polyethylene oxide)-*b*-poly(propylene oxide)-*b*-poly(ethylene oxide), poly(hydroxyethylmethacrylate) (polyHEMA), polyethylene terephthalate (PET), polybutylene terephthalate (PBT), polymethylmethacrylate (PMMA), polyether ether ketone (PEEK), polyamide, polyurethane, cellulosic polymer, polysulfone, and copolymers thereof, and blends thereof. Base polymeric copolymers include, e.g., polyethylene oxide)-*b*-poly(propylene oxide)-*b*-poly(ethylene oxide) and polyether-*b*-polyamide (e.g., PEBAX).

The term "biointerface film," as used herein, refers to a film that functions as an interface between host tissue and the remaining portion of an implantable device. The film may be a monolayer film that is an uncoated semipermeable membrane or a bilayer film that is a coated semipermeable membrane.

The term "biostabilizing additive," as disclosed herein, refers to a segmented compound of any one of formulae (I), (II), (III), (IV), (V), (VI), (VII), (VIII), (IX), (X), (XI), (XII), (XIII), (XIV), (XV), (XVI), and (XVII). Certain biostabilizing additives disclosed herein can have a theoretical molecular weight of less than or equal to 50 kDa (e.g., less than or equal to 10 kDa). Certain biostabilizing additives disclosed herein can have a theoretical molecular weight of greater than or equal to 200 Da (e.g., greater than or equal to 300 Da). Non-limiting examples of biostabilizing additives disclosed herein include those having a theoretical molecular weight of from 500 to 40,000 Daltons, from 500 to 20,000 Daltons, from 500 to 15,000 Daltons, from 1,000 to 12,000 Daltons, from 1,000 to 6,000 Daltons, or from 1,500 to 8,000 Daltons. One of skill in the art will recognize that these structural formulae represent idealized theoretical structures. Specifically, the segments are reacted in specific stoichiometries to furnish a biostabilizing additive as a distribution of molecules having varying ratios of segments. Accordingly, the variable *n* in formulae (I)-(XVII) indicates the theoretical stoichiometry of the segments. The biostabilizing additive according to the present invention comprises a compound according to formula (XX), (XXI), or (XXII).

The term "biostable surface," as used herein, refers to a surface of a semipermeable film that exhibits reduced protein and cell deposition on the surface, as compared to the deposition of proteins and cells under the same conditions on a reference surface of a reference semipermeable film that differs from the semipermeable film having a biostable surface only by the absence of a biostabilizing additive.

As used herein, "C" refers to a chain terminating group. Exemplary chain terminating groups include monofunctional groups containing an amine, alcohol, or carboxylic acid functionality. The term "LinkB," as used herein, refers to a coupling segment linking two oligomeric segments and a surface-active group. Typically, LinkB has a molecular weight ranging from 40 to 700. Preferably, LinkB can be selected from the group of functionalized diamines, diisocyanates, disulfonic acids, dicarboxylic acids, diacid chlorides, and dialdehydes, where the functionalized component has secondary functional group, through which a surface-active group is attached. Such secondary functional groups can be esters, carboxylic acid salts, sulfonic acid salts, phosphonic acid salts, thiols, vinyls, and primary or secondary amines. Terminal hydroxyls, amines, or carboxylic acids of an oligomeric segment intermediate can react with a diamine to form an oligo-amide; react with a diisocyanate to form an oligo-urethane, an oligo-urea, or an oligo-amide; react with a disulfonic acid to form an oligo-sulfonate or an oligo-sulfonamide; react with a dicarboxylic acid to form an oligo-ester or an oligo-amide; react with a diacyl dichloride to form an oligo-ester or an oligo-amide; or react with a dicarboxaldehyde to form an oligo-acetal or an oligo-imine.

The term "linker with two terminal carbonyls," as used herein, refers to a divalent group having a molecular weight of between 56 Da and 1,000 Da, in which the first valency belongs to a first carbonyl, and a second valency belongs to a second carbonyl. Within this linker, the first carbonyl is bonded to a first carbon atom, and the second carbonyl is bonded to a second carbon atom. The linker with two terminal carbonyls can be a small molecule dicarbonyl (e.g., norbornene-dicarbonyl, benzene-dicarbonyl, biphenyl-dicarbonyl, alkylene-dicarbonyl (e.g., succinoyl, glutaryl, adipoyl, pimeloyl, suberoyl, etc.)

The term "molecular weight," as used herein, refers to a theoretical weight of an Avogadro number of molecules of identical composition. As preparation of a biostabilizing additive can involve generation of a distribution of compounds, the term "molecular weight" refers to a molar mass of an idealized structure determined by the stoichiometry of the reactive ingredients. Thus, the term "molecular weight," as used herein, refers to a theoretical molecular weight.

The term "oligomeric linker," as used herein, refers to a divalent group containing from two to fifty bonded to each other identical chemical moieties. The chemical moiety can be an alkylene oxide (e.g., ethylene oxide).

The term "oligomeric segment," as used herein, refers to a relatively short length of a repeating unit or units, generally less than about 50 monomeric units and theoretical molecular weights less than 10,000 Daltons, but preferably <7,000 Daltons and in some examples, <5,000 Daltons. In certain embodiments, oligo is selected from the group consisting of polyurethane, polyurea, polyamide, polyalkylene oxide, polycarbonate, polyester, polylactone, polysilicone, polyethersulfone, polyolefin, polyvinyl, polypeptide, polysaccharide, and ether and amine linked segments thereof.

The term "oxycarbonyl bond," as used herein, refers to a bond connecting an oxygen atom to a carbonyl group. Exemplary oxycarbonyl bonds can be found in esters and urethanes. Preferably, the oxycarbonyl bond is a bond in an ester.

The term "polysulfone," as used herein, refers to a class of polymers that include as a repeating subunit the moiety -aryl-SO₂-aryl-. Polysulfones include, without limitation, polyethersulfones and poly(oxy-1,4-phenylene sulfonyl-1,4-phenyleneoxy-1,4-phenyleneisopropylidene-1,4-phenylene).

The term "polyalkylene," when used herein in reference to a base polymer, refers to a base polymer composed of linear or branched alkylene repeating units having from 2 to 4 carbon atoms and/or optionally a cyclic olefin of 3 to 10 carbon atoms (e.g., norbornene or tetracyclododecene). Each alkylene repeating unit is optionally substituted with one substituent selected from the group consisting of chloro, methoxycarbonyl, ethoxycarbonyl, hydroxyethoxycarbonyl, pyrrolidone, hydroxy, acetoxy, cyano, and phenyl. Non-limiting examples of polyalkylene base polymers include polystyrene, a cyclic olefin polymer (COP), a cyclic olefin copolymer (COC), MABS, SAN, SMMA, MBS, SB, and polyacrylate (e.g., PMMA).

The term "polyfluoroorgano group," as used herein, refers to a hydrocarbon group that may be optionally interrupted by one, two, or three non-contiguous oxygen atoms, in which from two to fifty nine hydrogen atoms were replaced with fluorine atoms. The polyfluoroorgano group contains one to thirty carbon atoms. The polyfluoroorgano group can contain linear alkyl, branched alkyl, or aryl groups, or any combination thereof. The polyfluoroorgano group (e.g., polyfluoroalkyl) can be a "polyfluoroacyl," in which the carbon atom, through which the polyfluoroorgano group (e.g., polyfluoroalkyl) is attached to the rest of the molecule, is substituted with oxo. The alkyl chain within polyfluoroorgano group (e.g., polyfluoroalkyl) can be interrupted by up to nine oxygen atoms, provided that two closest oxygen atoms within polyfluoroorgano are separated by at least two carbon atoms. When the polyfluoroorgano consists of a linear or branched alkyl optionally substituted with oxo and/or optionally interrupted with oxygen atoms, as defined herein, such group can be called a polyfluoroalkyl group. Some polyfluoroorgano groups (e.g., polyfluoroalkyl) can have a theoretical molecular weight of from 100 Da to 1,500 Da. A polyfluoroalkyl can be CF₃(CF₂)ᵣ(CH₂CH₂)ₚ-, where p is 0 or 1, r is from 2 to 20, or CF₃(CF₂)ₛ(CH₂CH₂O)_{χ}-, where χ is from 0 to 10, and s is from 1 to 20. Alternatively, polyfluoroalkyl can be CHₘF(₃₋ₘ)(CF₂)ᵣCH₂CH₂- or CHₘF(₃₋ₘ)(CF₂)ₛ(CH₂CH₂O)_{χ}-, where m is 0, 1, 2, or 3; χ is from 0 to 10; r is an integer from 2 to 20; and s is an integer from 1 to 20. In particular embodiments, χ is 0. In certain embodiments, polyfluoroalkyl is formed from 1H,1H,2H,2H-perfluoro-1-decanol; 1H,1H,2H,2H-perfluoro-1-octanol; 1H,1H,5H-perfluoro-1-pentanol; or 1H,1H, perfluoro-1-butanol, and mixtures thereof. In other embodiments, polyfluoroalkyl is perfluoroheptanoyl. In still other embodiments, polyfluoroalkyl is (CF₃)(CF₂)₅CH₂CH₂O-, (CF₃)(CF₂)₇CH₂CH₂O-, (CF₃)(CF₂)₅CH₂CH₂O-, CHF₂(CF₂)₃CH₂O-, (CF₃)(CF₂)₂CH₂O-, or (CF₃)(CF₂)₅-. In still other embodiments the polyfluoroalkyl group is (CF₃)(CF₂)₅-, e.g., where the polyfluoroalkyl group is bonded to a carbonyl of an ester group. In certain embodiments, polyfluoroorgano is -(O)_{q}-[C(=O)]ᵣ-(CH₂)ₒ(CF₂)ₚCF₃, in which q is 0 and r is 1, or q is 1 and r is 0; o is from 0 to 2; and p is from 0 to 10.

The term "semipermeable," as used herein, refers to a membrane that permits the diffusion of glucose from one side of the membrane to the opposing side of the same membrane.

The term "subject," as used herein, refers to a mammal (e.g., a human) in need of glucose monitoring because of having a disease or condition associated with reduction or loss of control over glucose homeostasis. For example, such a subject may be a diabetic.

The term "substantially similar," as used herein, refers to a measured property being ±20% of a reference measurement.

The term "surface-active group," as used herein, refers to a hydrophobic group bonded to a segment of a biostabilizing additive. For example, the surface-active group can be positioned to cap two, three, or four termini of the central, segmented polymeric portion of the biostabilizing additive and/or can be attached to one or more side chains present in the central polymeric portion of the surface modifier. Examples of surface-active groups include, without limitation, polydimethylsiloxanes, hydrocarbons, polyfluoroalkyl, fluorinated polyethers, and combinations thereof.

Other features and advantages of the invention will be apparent from the Drawings, Detailed Description, and the claims.

### Brief Description of the Drawings

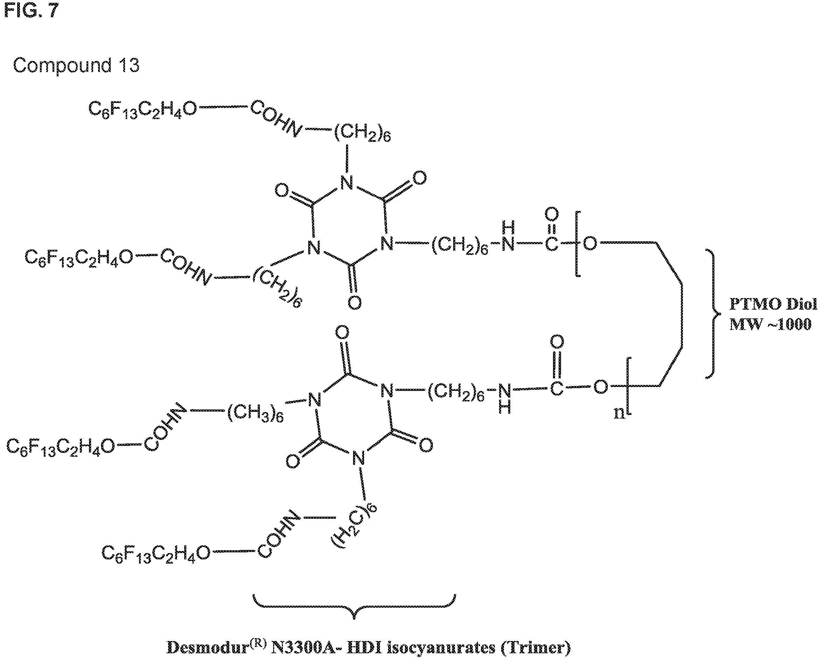

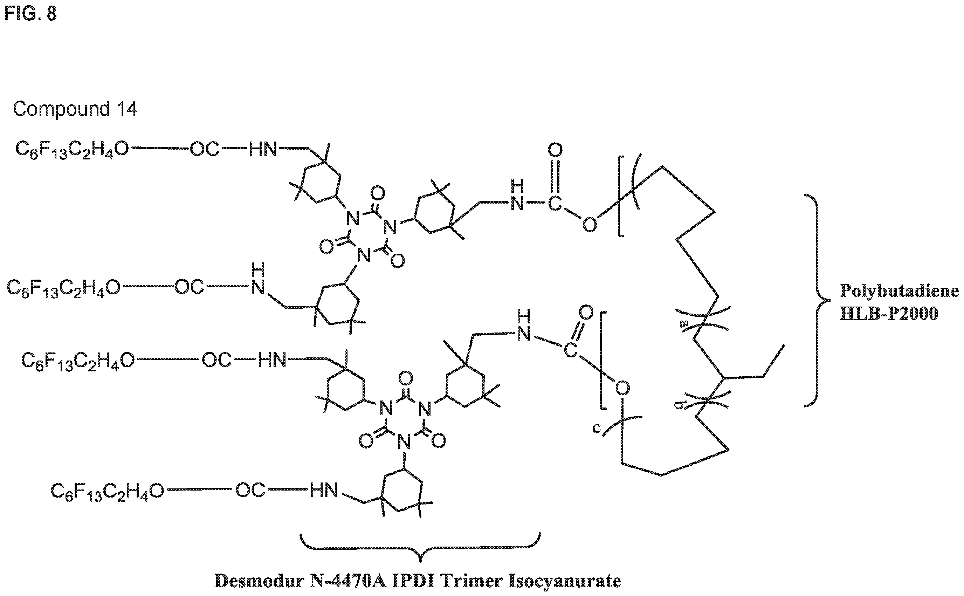

FIGs 1 to 27, 33, and 34 are not according to the present invention.
FIG. 1A shows a structure of compound 1.
FIG. 1B shows a structure of compound 2.
FIG. 2A shows a structure of compound 3.
FIG. 2B shows a structure of compound 4.
FIG. 3A shows a structure of compound 5.
FIG. 3B shows a structure of compound 6.
FIG. 4A shows a structure of compound 7.
FIG. 4B shows a structure of compound 8.
FIG. 5A shows a structure of compound 9.
FIG. 5B shows a structure of compound 10.
FIG. 6A shows a structure of compound 11.
FIG. 6B shows a structure of compound 12.
FIG. 7 shows a structure of compound 13.
FIG. 8 shows a structure of compound 14.
FIG. 9 shows a structure of compound 15.
FIG. 10 shows a structure of compound 16.
FIG. 11 shows a structure of compound 17.
FIG. 12 shows a structure of compound 18.
FIG. 13 shows a structure of compound 19.
FIG. 14 shows a structure of compound 20.
FIG. 15 shows a structure of compound 21.
FIG. 16 shows a structure of compound 22.
FIG. 17shows a structure of compound 23.
FIG. 18 shows a structure of compound 24.
FIG. 19 shows a structure of compound 25.
FIG. 20 shows a structure of compound 26.
FIG. 21A shows a structure of compound 27.
FIG. 21B shows a structure of compound 28.
FIG. 22 shows a structure of compound 29.
FIG. 23A shows a structure of compound 30.
FIG. 23B shows a structure of compound 31.
FIG. 24A shows a structure of compound 32.
FIG. 24B shows a structure of compound 33.
FIG. 25 shows a structure of compound 34.
FIG. 26 shows a structure of compound 35.
FIG. 27 shows a structure of compound 36.
FIG. 28A shows a structure of compound 37.
FIG. 28B shows a structure of compound 38.
FIG. 29 shows a structure of compound 39.
FIG. 30 shows a structure of compound 40.

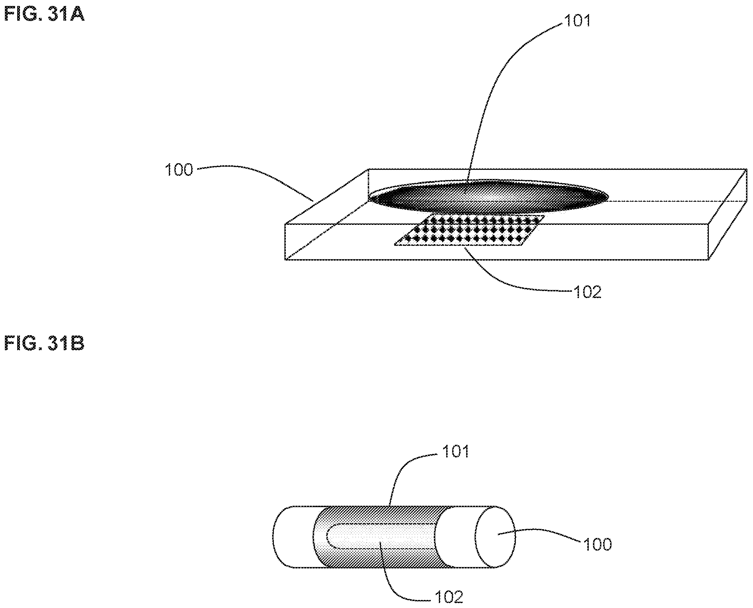

FIG. 31A is a drawing of a non-limiting example of an implantable glucose sensor of the invention. As illustrated in this figure, enclosure (100) includes a semipermeable biointerface film (101), and working electrode (102) is disposed internally within enclosure (100). In this configuration, working electrode (102) can be a metal foil (e.g., a silver foil) or a metallized plastic surface. The drawing is not to scale.
FIG. 31B is a drawing of another non-limiting example of an implantable glucose sensor of the invention. As illustrated in this figure, enclosure (100) includes a semipermeable biointerface film (101), and working electrode (102) is disposed internally within enclosure (100). In this configuration, working electrode (102) can be a wire (e.g., a gold wire) or a metallized plastic thread. The drawing is not to scale.

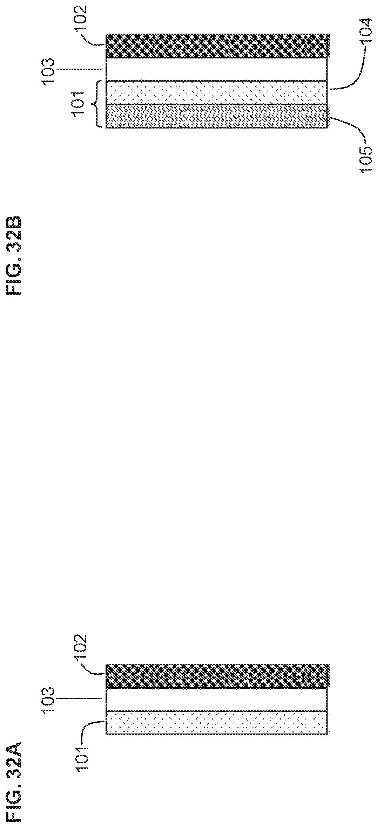

FIG. 32A is a drawing explicating the relative spatial relationship between monolayer semipermeable biointerface film layer (101), working electrode layer (102), and glucose-oxidizing enzyme layer (103). In this arrangement, layer (101) is externally facing and layer (102) is contained within an enclosure. Layer (101) includes a biostabilizing additive. The drawing is not to scale.
FIG. 32B is a drawing explicating the relative spatial relationship between bilayer semipermeable biointerface film (101), working electrode layer (102), and glucose-oxidizing enzyme layer (103). Film (101) includes a semipermeable membrane layer (104) and a coating layer (105). In this arrangement, layer (105) is externally facing and layer (102) is contained within an enclosure. Coating layer (105) includes a biostabilizing additive. Membrane layer (104) may also include a biostabilizing additive. The drawing is not to scale.
FIG. 33 is a chart comparing protein adhesion on rods prepared with and without a biostabilizing additive. Number of samples = 2. The values along the Y axis correspond to the BCA adhesion values normalized to the reference rod.
FIG. 34 is a chart comparing thrombosis observed on rods prepared with and without a biostabilizing additive. Number of samples = 21.

### Detailed Description

The invention features the specific compound of formula (XX), (XXI) or (XXII) as well as a specific implantable glucose sensor inter alia comprising a semipermeable biointerface film comprising a base polymer and a biostabilizing additive comprising at least one of these compounds. The glucose detector is disposed in the internal space. The enclosure includes a semipermeable biointerface film containing a base polymer and a specific biostabilizing additive, where the semipermeable biointerface film has a biostable surface and is permeable to glucose and, optionally, oxygen. The biostable surface faces the external space or both the internal space and the external space.

The implantable glucose sensor of the invention is configured to include the semipermeable biointerface film between a tissue of a subject and the glucose detector, and the glucose detector does not contact a tissue of a subject upon implantation of the implantable glucose sensor into the subject. The implantable glucose sensor of the invention is configured to place the biostable surface in contact with a tissue of a subject upon implantation of the implantable glucose sensor into the subject.

The implantable glucose sensor of the invention may be a subcutaneous, intravascular (e.g., intravenous), or transcutaneous glucose sensor.

Advantageously, the implantable glucose sensors of the invention may have a prolonged *in vivo* lifespan (e.g., at least 5%, at least 10%, at least 20%, or at least 50% longer lifespan) in comparison to a reference implantable glucose sensor that differs from the glucose sensor of the invention only by the absence of the biostabilizing additive in the reference glucose sensor.

The implantable glucose sensors of the invention may also exhibit a reduced mean absolute relative difference (MARD) in comparison to a reference implantable glucose sensor that differs from the glucose sensor of the invention only by the absence of the biostabilizing additive in the reference glucose sensor. Typically, the implantable glucose sensors of the invention may exhibit an initial MARD of less than 13% (e.g., less than 12%, less than 11%, less than 10%, less than 9%, less than 8%, less than 7%, or less than 6%). In some embodiments, the implantable glucose sensor of the invention exhibits an initial MARD of less than 9%. Typically, the implantable glucose sensors of the invention may exhibit an initial MARD of e.g., more than 0.1% (e.g., more than 1%, more than 2%, more than 3%, more than 4%, more than 5%, or more than 6%).

Further, the biostable surface of the semipermeable biointerface film in the glucose sensors of the invention may exhibit reduced protein and cell deposition as compared to a reference film that differs from the semipermeable biointerface film in the glucose sensors of the invention only by the absence of the biostabilizing additive in the reference film. The protein and cell deposition may be measured using methods known in the art. For example, protein deposition may be measured using bicinchoninic acid assay, e.g., as described herein. Cell deposition may be measured by comparing SEM imaged of the surfaces of explanted semipermeable biointerface films that were previously implanted in an animal model.

The biostable surface of the semipermeable biointerface film in the glucose sensors of the invention may exhibit a substantially similar aqueous wettability, as compared to a reference film that differs from the semipermeable biointerface film in the glucose sensors of the invention only by the absence of the biostabilizing additive in the reference film. Typically, aqueous wettability is measured by the diameter of a wet circle produced by placing a predetermined quantity of water as a single drop on a semipermeable biointerface film of a predetermined thickness.

The biostable surface of the semipermeable biointerface film of the invention may exhibit a substantially similar hydration, as compared to a reference surface of a reference membrane that differs from the semipermeable biointerface membrane of the invention only by the absence of a biostabilizing additive. The hydration of a semipermeable biointerface film may be measured as a percentage increase in the mass of a semipermeable biointerface film of a predetermined size after its immersion in water for a predetermined period of time. Typically hydration of the biointerface film of the invention may be at least about 5% (w/w) (e.g., at least about 10% (w/w), at least about 20% (w/w), at least about 50% (w/w), at least about 100% (w/w), at least about 300% (w/w) (e.g., from about 10% (w/w) to about 1000% (w/w), from about 50% (w/w) to about 1000% (w/w), from about 100% (w/w) to about 1000% (w/w), from about 10% (w/w) to about 500% (w/w), from about 50% (w/w) to about 500% (w/w), or from about 100% (w/w) to about 500% (w/w)).

The biostable surface of the semipermeable biointerface membrane of the invention may exhibit a reduction in the inflammatory response in a tissue that is in contact with the biostable surface, as compared to a reference surface of a reference membrane that differs from the semipermeable biointerface membrane of the invention only by the absence of a biostabilizing additive.

Without wishing to be bound by a theory, the inclusion of the biostabilizing additives in the semipermeable biointerface films of the invention may reduce protein and cellular attachment to the semipermeable biointerface films and may reduce the rate of barrier cell layer (e.g., fibrotic capsule) formation, thereby enhancing the overall lifetime of the device without compromising the glucose permeability of the biointerface films.

The semipermeable biointerface membrane of the invention may exhibit a reduced permeability (e.g., by at least about 5%, by at least about 10%, by at least about 20%, by at least about 50%, or by at least about 70% (e.g., by from about 5% to about 80%, by from about 10% to about 80%, by from about 20% to about 80%, or by from about 50% to about 80%)) for certain electrochemical interferents (e.g., acetaminophen), as compared to a reference semipermeable biointerface membrane that differs from the semipermeable biointerface membrane of the invention only by the absence of a biostabilizing additive. Typical electrochemical interferents known in the art include acetaminophen, salicylic acid, tetracycline, dopamine, ephedrine, ibuprofen, L-DOPA, methyl-DOPA, tolazamide, ascorbic acid, bilirubin, cholesterol, creatinine, triglycerides, and uric acid.

### Glucose Detection Approaches

### Electrochemical Glucose Detection

Implantable glucose sensors of the invention may be implantable electrochemical glucose sensors which detect glucose in a subject using enzymatic or non-enzymatic approaches known in the art.

An enzymatic approach typically involves a glucose-oxidizing enzyme-mediated (e.g., glucose oxidase-mediated) oxidation reaction between glucose and an oxidizer (e.g., oxygen) to give gluconolactone and a reduced form of the oxidizer (e.g., hydrogen peroxide). In these approaches, a working electrode of the glucose sensor typically detects an amperometric signal obtained by electrochemical oxidation of the reduced mediator (e.g., electrochemical oxidation of hydrogen peroxide to oxygen). In some enzymatic approaches, the oxidation reaction is a glucose-oxidizing enzyme-mediated (e.g., glucose oxidase-mediated) electrochemical oxidation of glucose to gluconolactone, where the role of an oxidizer is performed by a working electrode linked to the enzyme. In these approaches, a working electrode of the glucose sensor typically detects an amperometric signal obtained by a glucose-oxidizing enzyme-mediated (e.g., glucose oxidase-mediated) electrochemical oxidation of glucose to gluconolactone. Other non-limiting examples of the enzymes that may be used in the enzymatic approaches include glucose dehydrogenases and quinoprotein-based glucose dehydrogenases.

The implantable electrochemical glucose sensors of the invention relying on the enzymatic (e.g., glucose oxidase-mediated) glucose detection approach typically further include a glucose oxidase layer disposed between the working electrode and the semipermeable biointerface film. The working electrodes used in implantable electrochemical glucose sensors utilizing enzymatic approach to glucose detection may be those known in the art as being useful in the field of implantable electrochemical glucose sensors.

A non-enzymatic approach to glucose detection typically involves detecting an amperometric signal obtained by direct electrochemical oxidation of glucose to gluconolactone. A working electrode utilized in these approaches is typically a nanostructured electrode having a high surface area and electrocatalytic activity. Nanostructure electrodes that may be used in the implantable electrochemical glucose sensors are known in the art (e.g., platinum nanoforests, platinum-lead alloy nanowires, gold nanoparticles, or alloy nanostructures (e.g., containing platinum, lead, gold, palladium, and/or rhodium)).

Desirably, the implantable electrochemical glucose sensors of the invention produce a linear response to glucose levels up to at least about 400 mg/dL.

The implantable electrochemical glucose sensors of the invention may be used with data retrievers and processors known in the art for processing amperometric signals produced by implantable glucose sensors. For example, such data retrievers and processors are described in U.S. Patent Nos. 8,844,057 and 8,251,906.

### Optical Glucose Detection

Implantable glucose sensors of the invention may be implantable optical glucose sensors which utilize a glucose recognition element for the detection of glucose. Typically, a glucose recognition element includes a glucose-binding fluorophore. Non-limiting examples of the glucose-binding fluorophores and glucose recognition elements that may be used in the implantable optical glucose sensors of the invention are described in US 2014/0088383.

### Electrode Systems

The implantable electrochemical glucose sensors of the invention include an electrode system capable of producing an amperometric signal allowing for the detection of glucose levels. The electrode systems used in the implantable electrochemical glucose sensors of the invention may be those known in the art. Typical electrode systems include a working electrode (anode), a counter-electrode (cathode), and a reference electrode. Various configurations of electrode systems are known in the art. A non-limiting example of an electrode system configuration is described in US 2005/0245799. Typically, the working electrode and the counter-electrode of a glucose oxidase-based implantable electrochemical glucose sensor require access to intracorporeal oxygen. Accordingly, in these embodiments, the implantable electrochemical glucose sensor includes a counter-electrode that is configured to be in oxygen communication with the external space through a semipermeable biointerface film. In some embodiments of the three-electrode system, all three electrodes are configured to be in glucose and oxygen communication with the external space through a semipermeable biointerface film.

### Glucose-oxidizing Enzyme Layer

The implantable electrochemical glucose sensors of the invention may include a glucose-oxidizing enzyme layer (e.g., a glucose oxidase layer) between the semipermeable biointerface film and a working electrode. The glucose-oxidizing enzyme layer typically contains an effective amount of a glucose-oxidizing enzyme (e.g., glucose oxidase enzyme, a glucose dehydrogenase, or a quinoprotein-based glucose dehydrogenase). The glucose-oxidizing enzyme layer may be formulated as a polymer matrix including an effective amount of a glucose-oxidizing enzyme and an oxygen-solubilizing polymer (e.g., a silicone, fluorocarbon polymer, perfluorocarbon polymer, or perfluoroether polymer). In addition, the polymer matrix may include an additive (e.g., polyethylene glycol, propylene glycol, pyrrolidone, an ester, an amide, or a carbonate). The thickness of the glucose-oxidizing enzyme layer may be from about 0.5 micron (e.g., from about 1 micron) to about 40, 50, 60, 70, 80, 90, or 100 microns. For example, the thickness of the glucose-oxidizing enzyme layer may be between about 1, 2, 3, 4, or 5 microns and 13, 14, 15, 20, 25, or 30 microns. The principles that may be utilized for including a glucose-oxidizing enzyme layer in implantable electrochemical glucose sensors of the invention are known in the art. For example, such principles are described in U.S. Patent No. 8,255,030.

### Glucose-flux Control Layer

The implantable electrochemical glucose sensors of the invention may further include a glucose-flux control layer disposed between the semipermeable biointerface film and the glucose-oxidizing enzyme layer. The glucose-flux control layers are known in the art. See, e.g., U.S. Patent No. 8,744,546. The glucose-flux control layer may be used to control the flux of glucose across the semipermeable biointerface films to reduce the amount of glucose that passes through to the glucose-oxidizing enzyme layer. Desirably, the glucose-flux control is achieved without compromising a linear response of the implantable electrochemical glucose sensors of the invention to glucose up to at least about 400 mg/dL. The inclusion of glucose-flux control layer may be beneficial in the glucose sensors exhibiting insufficient control over glucose flux across the semipermeable biointerface films, which may result in oxygen insufficiency at high glucose concentrations, thereby producing non-linear response at higher glucose levels.

Alternatively, the semipermeable biointerface films of the invention may enhance oxygen flux across the film and/or reduce the flux of glucose, thereby reducing or even negating the need for glucose-flux control layers. Accordingly, some of the implantable electrochemical glucose sensors may be free of glucose-flux control layers.

### Semipermeable Biointerface Films

Semipermeable biointerface films of the invention contain a biostabilizing additive and a base polymer (e.g., the biostabilizing additive is from 0.05% (w/w) to 15% (w/w) (e.g., from 0.05% (w/w) to 10% (w/w)) relative to the total mass of the semipermeable biointerface film). Semipermeable biointerface films are monolayer or bilayer films, where one of the layers is a semipermeable membrane containing a base polymer (e.g., a thermoplastic). In a bilayer film, the second layer may be a coating on the semipermeable membrane. Typically, the biostabilizing additives used in the semipermeable biointerface films of the invention may leave bulk properties of the base polymer material substantially unchanged.

Typically, a semipermeable biointerface film of the invention may have a thickness of from 1 to 200 microns (e.g., from 1 to 150 microns, from 1 to 100 microns, from 1 to 50 microns, from 5 to 150 microns, from 5 to 100 microns, from 5 to 50 microns, from 10 to 150 microns, from 10 to 100 microns, from 10 to 50 microns, from 1 to 20 microns, from 20 to 50 microns, from 50 to 100 microns, from 100 to 150 microns, or from 150 to 200 microns).

Semipermeable biointerface films of the invention may further include a biologically active agent. The biologically active agent may, for example, be included in the coating used in the bilayer semipermeable biointerface films of the invention. The bioactive agents incorporated in the semipermeable biointerface films of the invention may further enhance biostability of the tissue-contacting surface of the semipermeable biointerface film.

### Base Polymers

The base polymer of the semipermeable membrane may be is a silicone, polyolefin, polyester, polycarbonate, polysulfone, polyamide, polyether, polyurea, polyurethane, polyetherimide, or cellulosic polymer, or a copolymer thereof or a blend thereof (e.g., a silicone, polycarbonate, polypropylene (PP), polyvinylchloride (PVC), polyvinyl alcohol (PVA), polyvinylpyrrolidone (PVP), polyacrylamide (PAAM), polyethylene oxide, polyethylene oxide)*-b*-poly(propylene oxide)-*b-*poly(ethylene oxide), poly(hydroxyethylmethacrylate) (polyHEMA), polyethylene terephthalate (PET), polybutylene terephthalate (PBT), polymethylmethacrylate (PMMA), polyether ether ketone (PEEK), polyamide, polyurethane, cellulosic polymer, polysulfone, or a copolymer thereof or a blend thereof). A base polymer used in the semipermeable biointerface films of the invention may be, e.g., polyvinylpyrrolidone (PVP), polyacrylamide (PAAM), polyethylene oxide, polyethylene oxide)-*b*-poly(propylene oxide)-*b*-poly(ethylene oxide), poly(hydroxyethylmethacrylate) (polyHEMA), polyether-*b*-polyamide (e.g., PEBAX), or polyurethane. A base polymer used in the semipermeable biointerface films of the invention may be a thermoplastic polymer (e.g., a thermoplastic polyurethane). The base polymers of the semipermeable membrane may also be cross-linked.

### Biostabilizing Additives

The biostabilizing additives comprised in the implantable glucose sensor of the present invention comprise any compound of formulae (XX), (XXI), and (XXII). The implantable glucose sensors of the invention may further comprise a compound described by the structure of any one of formulae (I), (II), (III), (IV), (V), (VI), (VII), (VIII), (IX), (X), (XI), (XII), (XIII), (XIV), (XV), (XVI), and (XVII) shown below.
(1) Formula (I):

   F_{T}-[B-A]ₙ-B-F_{T} (I)

   where
   (i) A includes hydrogenated polybutadiene, poly((2,2-dimethyl)-1,3-propylene carbonate), polybutadiene, poly(diethylene glycol)adipate, poly(hexamethylene carbonate), poly(ethylene-co-butylene), (neopentyl glycol-ortho phthalic anhydride) polyester, (diethylene glycol-ortho phthalic anhydride) polyester, (1,6-hexanediol-ortho phthalic anhydride) polyester, or bisphenol A ethoxylate;
   (ii) B is a segment including a urethane; and
   (iii) F_{T} is a polyfluoroorgano group, and
   (iv) n is an integer from 1 to 10.
(2) Formula (II):

   F_{T}-[B-A]ₙ-B-F_{T} (II)

   where
   (i) B includes a urethane;
   (ii) A includes polypropylene oxide, polyethylene oxide, or polytetramethylene oxide;
   (iii) F_{T} is a polyfluoroorgano group; and
   (iv) n is an integer from 1 to 10.
(3) Formula (III) or Formula (IV): 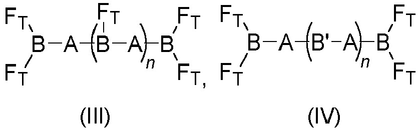 where
   (i) A is an oligomeric segment containing an ether linkage, an ester linkage, a carbonate linkage, or a polyalkylene and having a theoretical molecular weight of from 500 to 3,500 Daltons (e.g., from 500 to 2,000 Daltons, from 1,000 to 2,000 Daltons, or from 1,000 to 3,000 Daltons);
   (ii) B is a segment including a isocyanurate trimer or biuret trimer; B', when present, is a segment including a urethane;
   (iii) each F_{T} is a polyfluoroorgano group; and
   (iv) n is an integer between 0 to 10.
(4) Formula (V):

   F_{T}-[B-A]ₙ-B-F_{T} (V)

   where
   (i) A is an oligomeric segment including polypropylene oxide, polyethylene oxide, or polytetramethylene oxide and having a theoretical molecular weight of from 500 to 3,000 Daltons (e.g., from 500 to 2,000 Daltons, from 1,000 to 2,000 Daltons, or from 1,000 to 3,000 Daltons);
   (ii) B is a segment formed from a diisocyanate;
   (iii) F_{T} is a polyfluoroorgano group; and
   (iv) n is an integer from 1 to 10.
(5) Formula (VI): 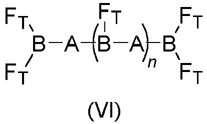 where
   (i) A is an oligomeric segment including polyethylene oxide, polypropylene oxide, polytetramethylene oxide, or a mixture thereof, and having a theoretical molecular weight of from 500 to 3,000 Daltons (e.g., from 500 to 2,000 Daltons, from 1,000 to 2,000 Daltons, or from 1,000 to 3,000 Daltons);
   (ii) B is a segment including an isocyanurate trimer or biuret trimer;
   (iii) F_{T} is a polyfluoroorgano group; and
   (iv) n is an integer from 0 to 10.
(6) Formula (VII):

   F_{T}-[B-A]ₙ-B-F_{T} (VII)

   where
   (i) A is a polycarbonate polyol having a theoretical molecular weight of from 500 to 3,000 Daltons (e.g., from 500 to 2,000 Daltons, from 1,000 to 2,000 Daltons, or from 1,000 to 3,000 Daltons);
   (ii) B is a segment formed from a diisocyanate;
   (iii) F_{T} is a polyfluoroorgano group; and
   (iv) n is an integer from 1 to 10.
(7) Formula (VIII): 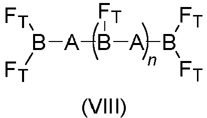 where
   (i) A is an oligomeric segment including a polycarbonate polyol having a theoretical molecular weight of from 500 to 3,000 Daltons (e.g., from 500 to 2,000 Daltons, from 1,000 to 2,000 Daltons, or from 1,000 to 3,000 Daltons);
   (ii) B is a segment including an isocyanurate trimer or biuret trimer;
   (iii) F_{T} is a polyfluoroorgano group; and
   (iv) n is an integer from 0 to 10.
(8) Formula (IX): 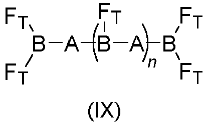 where
   (i) A includes a first block segment selected from polypropylene oxide, polyethylene oxide, polytetramethylene oxide, or a mixture thereof, and a second block segment including a polysiloxane or polydimethylsiloxane, where A has a theoretical molecular weight of from 1,000 to 5,000 Daltons (e.g., from 1,000 to 3,000 Daltons, from 2,000 to 5,000 Daltons, or from 2,500 to 5,000 Daltons);
   (ii) B is a segment including an isocyanurate trimer or biuret trimer;
   (iii) F_{T} is a polyfluoroorgano group; and
   (iv) n is an integer from 0 to 10.
(9) Formula (X):

   F_{T}-[B-A]ₙ-B-F_{T} (X)

   where
   (i) A is a segment selected from the group consisting of hydrogenated polybutadiene (e.g., HLBH), polybutadiene (e.g., LBHP), hydrogenated polyisoprene (e.g., HHTPI), polysiloxane-polyethylene glycol block copolymer, and polystyrene and has a theoretical molecular weight of from 750 to 3,500 Daltons (e.g., from 750 to 2,000 Daltons, from 1,000 to 2,500 Daltons, or from 1,000 to 3,500 Daltons);
   (ii) B is a segment formed from a diisocyanate;
   (iii) F_{T} is a polyfluoroorgano group; and
   (iv) n is an integer from 1 to 10.
(10) Formula (XI): 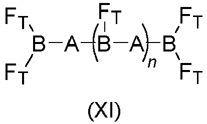 where
   (i) A is hydrogenated polybutadiene (e.g., HLBH), polybutadiene (e.g., LBHP), hydrogenated polyisoprene (e.g., HHTPI), or polystyrene and has a theoretical molecular weight of from 750 to 3,500 Daltons (e.g., from 750 to 2,000 Daltons, from 1,000 to 2,500 Daltons, or from 1,000 to 3,500 Daltons);
   (ii) B is a segment including an isocyanurate trimer or biuret trimer;
   (iii) F_{T} is a polyfluoroorgano group; and
   (iv) n is an integer from 0 to 10.
(11) Formula (XII): 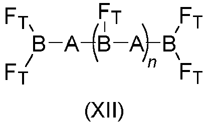 where
   (i) A is a polyester having a theoretical molecular weight of from 500 to 3,500 Daltons (e.g., from 500 to 2,000 Daltons, from 1,000 to 2,000 Daltons, or from 1,000 to 3,000 Daltons);
   (ii) B is a segment including an isocyanurate trimer or biuret trimer;
   (iii) F_{T} is a polyfluoroorgano group; and
   (iv) n is an integer from 0 to 10.
(12) Formula (XIII):

   F_{T}-A-F_{T} (XIII)

   where F_{T} is a polyfluoroorgano group and A is an oligomeric segment.
(13) Formula (XIV): 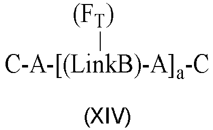 where
   (i) F_{T} is a polyfluoroorgano group covalently attached to LinkB;
   (ii) C is a chain terminating group;
   (iii) A is an oligomeric segment;
   (iv) LinkB is a coupling segment; and
   (v) a is an integer greater than 0.
(14) Formula (XV): 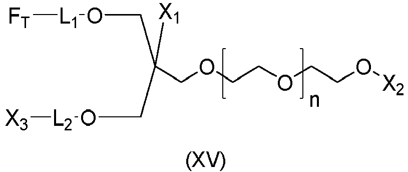 where
   (i) each F_{T} is independently a surface-active group selected from polydimethylsiloxanes, hydrocarbons, and polyfluoroorgano groups, and combinations thereof (e.g., each F_{T} is independently a polyfluoroorgano);
   (ii) X₁ is H, CH₃, or CH₂CH₃;
   (iii) each of X₂ and X₃ is independently H, CH₃, CH₂CH₃, or F_{T};
   (iv) each of L₁ and L₂ is independently a bond, an oligomeric linker, or a linker with two terminal carbonyls; and
   (v) n is an integer from 5 to 50.
(15) Formula (XVI): 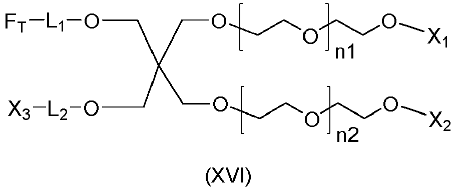 where
   (i) each F_{T} is independently a surface-active group (e.g., a polyfluoroorgano);
   (ii) each of X₁, X₂, and X₃ is independently H, CH₃, CH₂CH₃, or F_{T};
   (iii) each of L₁ and L₂ is independently a bond, an oligomeric linker, a linker with two terminal carbonyls, or is formed from a diisocyanate; and
   (iv) each of n1 and n2 is independently an integer from 5 to 50.
(16)

   Formula (XVII): G - Aₘ - [B - A]ₙ - B - G (XVII)

   where
   (i) each A includes hydrogenated polybutadiene, poly ((2,2-dimethyl)-1,3-propylene carbonate), polybutadiene, poly (diethylene glycol)adipate, poly (hexamethylene carbonate), poly (ethylene-co-butylene), (diethylene glycol-ortho phthalic anhydride) polyester, (1,6-hexanediol-ortho phthalic anhydride) polyester, (neopentyl glycol-ortho phthalic anhydride) polyester, a polysiloxane, or bisphenol A ethoxylate;
   (ii) each B is independently a bond, an oligomeric linker, or a linker with two terminal carbonyls;
   (iii) each G is H or a polyfluoroograno, provided that at least one G is a polyfluoroorgano;
   (iv) n is an integer from 1 to 10; and
   (v) m is 0 or 1.

The biostabilizing additive of formula (I) or formula (II) can include B formed from a diisocyanate (e.g., 3-isocyanatomethyl-3,5,5-trimethyl-cyclohexylisocyanate; 4,4'-methylene bis(cyclohexyl isocyanate); 4,4'-methylene bis(phenyl isocyanate); toluene-2,4-diisocyanate; m-tetramethylxylene diisocyanate; or hexamethylene diisocyanate). The variable n may be 1 or 2. The implantable glucose sensors of the invention may include a semipermeable biointerface film (e.g., a monolayer or a bilayer film) containing a base polymer and the biostabilizing additive of formula (I) or formula (II).

The biostabilizing additive of formulae (III) and (IV) can include A that is an oligomeric segment containing hydrogenated polybutadiene (HLBH), poly((2,2-dimethyl)-1,3-propylene carbonate) (PCN), polybutadiene (LBHP), polytetramethylene oxide (PTMO), polypropylene oxide (PPO), (diethyleneglycol-orthophthalic anhydride) polyester (PDP), hydrogenated polyisoprene (HHTPI), poly(hexamethylene carbonate), poly((2-butyl-2-ethyl)-1,3-propylene carbonate), or hydroxylterminated polydimethylsiloxane (C22). In the biostabilizing additive of formulae (III) and (IV), B is formed by reacting a triisocyanate (e.g., hexamethylene diisocyanate (HDI) biuret trimer, isophorone diisocyanate (IPDI) trimer, or hexamethylene diisocyanate (HDI) trimer) with a diol including the oligomeric segment A. The implantable glucose sensors of the invention may include a semipermeable biointerface film (e.g., a monolayer or a bilayer film) containing a base polymer and the biostabilizing additive of formula (III). The implantable glucose sensors of the invention may include a semipermeable biointerface film (e.g., a monolayer or a bilayer film) containing a base polymer and the biostabilizing additive of formula (IV).

In the biostabilizing additive of formula (V), B may be a segment formed from 3-isocyanatomethyl-3,5,5-trimethyl-cyclohexylisocyanate; 4,4'-methylene bis(cyclohexyl isocyanate); 4,4'-methylene bis(phenyl isocyanate); toluene-2,4-diisocyanate; m-tetramethylxylene diisocyanate; and hexamethylene diisocyanate. In the biostabilizing additive of formula (V), segment A can be polyethylene oxide)-*b*-poly(propylene oxide)-*b*-poly(ethylene oxide). The variable n may be an integer from 1 to 3. The implantable glucose sensors of the invention may include a semipermeable biointerface film (e.g., a monolayer or a bilayer film) containing a base polymer and the biostabilizing additive of formula (V).

In the biostabilizing additive of formula (VI), B is a segment formed by reacting a triisocyanate with a diol of A. The triisocyanate may be hexamethylene diisocyanate (HDI) biuret trimer, isophorone diisocyanate (IPDI) trimer, or hexamethylene diisocyanate (HDI) trimer. In the biostabilizing additive of formula (VI), segment A can be polyethylene oxide)-*b*-poly(propylene oxide)-*b-*poly(ethylene oxide). The variable n may be 0, 1, 2, or 3. The implantable glucose sensors of the invention may include a semipermeable biointerface film (e.g., a monolayer or a bilayer film) containing a base polymer and the biostabilizing additive of formula (VI).

In the biostabilizing additive of formula (VII), Oligo can include poly((2,2-dimethyl)-1 ,3-propylene carbonate) (PCN). B may be a segment formed from 3-isocyanatomethyl-3,5,5-trimethyl-cyclohexylisocyanate; 4,4'-methylene bis(cyclohexyl isocyanate); 4,4'-methylene bis(phenyl isocyanate); toluene-2,4-diisocyanate; m-tetramethylxylene diisocyanate; and hexamethylene diisocyanate. The variable n may be 1, 2, or 3. The implantable glucose sensors of the invention may include a semipermeable biointerface film (e.g., a monolayer or a bilayer film) containing a base polymer and the biostabilizing additive of formula (VII).

In the biostabilizing additive of formula (VIII), B is a segment formed by reacting a triisocyanate with a diol of A (e.g., the oligomeric segment). The triisocyanate may be hexamethylene diisocyanate (HDI) biuret trimer, isophorone diisocyanate (IPDI) trimer, or hexamethylene diisocyanate (HDI) trimer. The segment A can include poly((2,2-dimethyl)-1,3-propylene carbonate) (PCN) or poly(hexamethylene carbonate) (PHCN). The variable n may be 0, 1, 2, or 3. The implantable glucose sensors of the invention may include a semipermeable biointerface film (e.g., a monolayer or a bilayer film) containing a base polymer and the biostabilizing additive of formula (VIII).

In the biostabilizing additive of formula (IX), B is a segment formed by reacting a triisocyanate with a diol of A. In segment A, the number of first block segments and second block segments can be any integer or non-integer to provide the approximate theoretical molecule weight of the segment. The segment A can include polypropylene oxide and polydimethylsiloxane. The triisocyanate may be hexamethylene diisocyanate (HDI) biuret trimer, isophorone diisocyanate (IPDI) trimer, or hexamethylene diisocyanate (HDI) trimer. The variable n may be 0, 1, 2, or 3. The implantable glucose sensors of the invention may include a semipermeable biointerface film (e.g., a monolayer or a bilayer film) containing a base polymer and the biostabilizing additive of formula (IX).

In biostabilizing additive of formula (X), B is a segment formed from a diisocyanate. The segment A can include hydrogenated polybutadiene. Alternatively, the segment A can include polysiloxane-polyethylene glycol block copolymer (e.g., PEG-PDMS-PEG). The segment B may be formed from 3-isocyanatomethyl-3,5,5-trimethy-cyclohexylisocyanate; 4,4'-methylene bis(cyclohexyl isocyanate); 4,4'-methylene bis(phenyl isocyanate); toluene-2,4-diisocyanate; m-tetramethylxylene diisocyanate; and hexamethylene diisocyanate. The variable n may be 1, 2, or 3. The implantable glucose sensors of the invention may include a semipermeable biointerface film (e.g., a monolayer or a bilayer film) containing a base polymer and the biostabilizing additive of formula (X).

In the biostabilizing additive of formula (XI), B is a segment formed by reacting a triisocyanate with a diol of A. The segment A may be hydrogenated polybutadiene (HLBH) or hydrogenated polyisoprene (HHTPI). The triisocyanate may be hexamethylene diisocyanate (HDI) biuret trimer, isophorone diisocyanate (IPDI) trimer, or hexamethylene diisocyanate (HDI) trimer. The variable n may be 0, 1, 2, or 3. The implantable glucose sensors of the invention may include a semipermeable biointerface film (e.g., a monolayer or a bilayer film) containing a base polymer and the biostabilizing additive of formula (XI).

In the biostabilizing additive of formula (XII), B is a segment formed by reacting a triisocyanate with a diol of A (e.g., polyester). The segment A may be poly(diethylene glycol)adipate, (neopentyl glycol-ortho phthalic anhydride) polyester, (diethylene glycol-ortho phthalic) anhydride polyester, or (1,6-hexanediol-ortho phthalic anhydride) polyester. The triisocyanate may be hexamethylene diisocyanate (HDI) biuret trimer, isophorone diisocyanate (IPDI) trimer, and hexamethylene diisocyanate (HDI) trimer. The variable n may be 0, 1, 2, or 3. The implantable glucose sensors of the invention may include a semipermeable biointerface film (e.g., a monolayer or a bilayer film) containing a base polymer and the biostabilizing additive of formula (XII).

The biostabilizing additive of formula (XIII) can include a segment A that is a branched or non-branched oligomeric segment of fewer than 20 repeating units (e.g., from 2 to 15 units, from 2 to 10 units, from 3 to 15 units, and from 3 to 10 units). In certain embodiments, the biostabilizing additive of formula (XIII) include an oligomeric segment selected from polyurethane, polyurea, polyamide, polyalkylene oxide, polycarbonate, polyester, polylactone, polysilicone, polyethersulfone, polyolefin, polyvinyl derivative, polypeptide, polysaccharide, polysiloxane, polydimethylsiloxane, polyethylenebutylene, polyisobutylene, polybutadiene, polypropylene oxide, polyethylene oxide, polytetramethylene oxide, or polyethylenebutylene segments. The implantable glucose sensors of the invention may include a semipermeable biointerface film (e.g., a monolayer or a bilayer film) containing a base polymer and the biostabilizing additive of formula (XIII).

The biostabilizing additive of formula (XIV) can include a segment A that is a branched or non-branched oligomeric segment of fewer than 20 repeating units (e.g., from 2 to 15 units, from 2 to 10 units, from 3 to 15 units, and from 3 to 10 units). In certain embodiments, the biostabilizing additive of formula (XIV) include an oligomeric segment selected from polyurethane, polyurea, polyamide, polyalkylene oxide, polycarbonate, polyester, polylactone, polysilicone, polyethersulfone, polyolefin, polyvinyl derivative, polypeptide, polysaccharide, polysiloxane, polydimethylsiloxane, polyethylenebutylene, polyisobutylene, polybutadiene, polypropylene oxide, polyethylene oxide, or polytetramethylene oxide. The implantable glucose sensors of the invention may include a semipermeable biointerface film (e.g., a monolayer or a bilayer film) containing a base polymer and the biostabilizing additive of formula (XIV).

The biostabilizing additive of formula (XV) can include a segment L₁ that is an oligomeric linker (e.g., of fewer than 50 repeating units (e.g., from 2 to 40 units, from 2 to 30 units, from 3 to 20 units, or from 3 to 10 units)). In some embodiments of formula (XV), L₂ is an oligomeric linker (e.g., of fewer than 50 repeating units (e.g., from 2 to 40 units, from 2 to 30 units, from 3 to 20 units, or from 3 to 10 units)). In particular embodiments of formula (XV), each of L₁ and L₂ is a bond. In certain embodiments of formula (XV), the biostabilizing additive includes an oligomeric segment (e.g., in any one of L₁ and L₂) selected from the group consisting of polyurethane, polyurea, polyamide, polyalkylene oxide (e.g., polypropylene oxide, polyethylene oxide, or polytetramethylene oxide), polyester, polylactone, polysilicone, polyethersulfone, polyolefin, polyvinyl derivative, polypeptide, polysaccharide, polysiloxane, polydimethylsiloxane, poly(ethylene-co-butylene), polyisobutylene, and polybutadiene. In some embodiments of formula (XV), the biostabilizing additive is a compound of formula (XV-A): 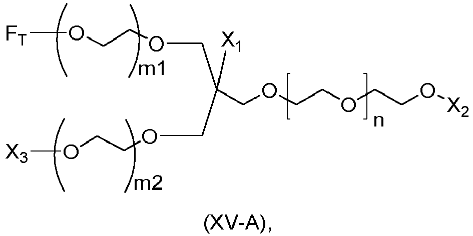 where each of m1 and m2 is independently an integer from 0 to 50. In particular embodiments of formula (XV-A), m1 is 5, 6, 7, 8, 9, or 10 (e.g., m1 is 6). In some embodiments of formula (XV-A), m2 is 5, 6, 7, 8, 9, or 10 (e.g., m2 is 6).

In certain embodiments of formula (XV) or (XV-A), X₂ is F_{T}. In other embodiments, X₂ is CH₃ or CH₂CH₃. In particular embodiments of formula (XV) or (XV-A), X₃ is F_{T}. In other embodiments, each F_{T} is independently a polyfluoroorgano (e.g., a polyfluoroacyl, such as -(O)_{q}-[C(=O)]ᵣ-(CH₂)ₒ(CF₂)ₚCF₃, in which q is 0, r is 1; o is from 0 to 2; and p is from 0 to 10). In certain embodiments of formula (XV) or (XV-A), n is an integer from 5 to 40 (e.g., from 5 to 20, such as from 5, 6, 7, 8, 9, or 10). In some embodiments of formula (XV) or (XV-A), each F_{T} includes (CF₂)₅CF₃. The implantable glucose sensors of the invention may include a semipermeable biointerface film (e.g., a monolayer or a bilayer film) containing a base polymer and the biostabilizing additive of formula (XV). The implantable glucose sensors of the invention may include a semipermeable biointerface film (e.g., a monolayer or a bilayer film) containing a base polymer and the biostabilizing additive of formula (XV-A).

The biostabilizing additive of formula (XVI) can include a segment L₁ that is an oligomeric linker (e.g., of fewer than 50 repeating units (e.g., from 2 to 40 units, from 2 to 30 units, from 3 to 20 units, or from 3 to 10 units)). In some embodiments of formula (XVI), L₂ is an oligomeric linker (e.g., of fewer than 50 repeating units (e.g., from 2 to 40 units, from 2 to 30 units, from 3 to 20 units, or from 3 to 10 units)). In particular embodiments of formula (XVI), each of L₁ and L₂ is a bond. In certain embodiments of formula (XVI), the biostabilizing additive includes an oligomeric segment (e.g., in any one of L₁ and L₂) selected from polyurethane, polyurea, polyamide, polyalkylene oxide (e.g., polypropylene oxide, polyethylene oxide, or polytetramethylene oxide), polyester, polylactone, polysilicone, polyethersulfone, polyolefin, polyvinyl derivative, polypeptide, polysaccharide, polysiloxane, polydimethylsiloxane, poly(ethylene-co-butylene), polyisobutylene, or polybutadiene. In some embodiments of formula (XVI), the biostabilizing additive is a compound of formula (XVI-A): 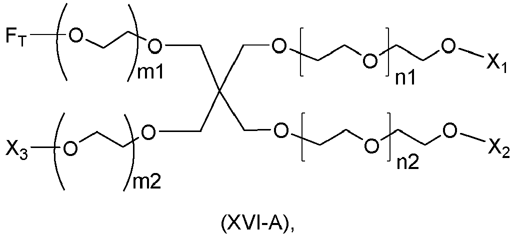 where each of m1 and m2 is independently an integer from 0 to 50. In particular embodiments of formula (XV-A), m1 is 5, 6, 7, 8, 9, or 10 (e.g., m1 is 6). In some embodiments of formula (XV-A), m2 is 5, 6, 7, 8, 9, or 10 (e.g., m2 is 6).

In certain embodiments of formula (XVI) or (XVI-A), X₂ is F_{T}. In other embodiments of formula (XVI) or (XVI-A), X₂ is CH₃ or CH₂CH₃. In particular embodiments of formula (XVI) or (XVI-A), X₃ is F_{T}. In other embodiments of formula (XVI) or (XVI-A), each F_{T} is independently a polyfluoroorgano (e.g., a polyfluoroacyl, such as -(O)_{q}-[C(=O)]ᵣ-(CH₂)ₒ(CF₂)ₚCF₃, in which q is 0, r is 1; o is from 0 to 2; and p is from 0 to 10). In some embodiments of formula (XVI) or (XVI-A), each F_{T} includes (CF₂)₅CF₃. The implantable glucose sensors of the invention may include a semipermeable biointerface film (e.g., a monolayer or a bilayer film) containing a base polymer and the biostabilizing additive of formula (XVI). The implantable glucose sensors of the invention may include a semipermeable biointerface film (e.g., a monolayer or a bilayer film) containing a base polymer and the biostabilizing additive of formula (XVI-A).

In some embodiments of formula (XVII), m is 1. The biostabilizing additive of formula (XVII) can be a compound of formula (XVII-A):

G - A - [B - A]ₙ - G (XVII-A).

In other embodiments of formula (XVII), m is 0. The biostabilizing additive of formula (XVII) can be a compound of formula (XVII-B):

G - [B - A]ₙ - B - G (XVII-B).

In particular embodiments of formula (XVII), (XVII-A), or (XVII-B), each B is a linker with two terminal carbonyls. In certain embodiments of formula (XVII), (XVII-A), or (XVII-B), each B is a bond. In some embodiments of Formula (XVII) , (XVII-A), or (XVII-B), the bond connecting G and B is an oxycarbonyl bond (e.g., an oxycarbonyl bond in an ester). In other embodiments of formula (XVII), (XVII-A), or (XVII-B), n is 1 or 2.

The biostabilizing additive of formula (XVII) can be a compound of formula (XVII-C):

G-A-G (XVII-C).

In formula (XVII), (XVII-A), (XVII-B), or (XVII-C), G can be a polyfluoroorgano group (e.g., a polyfluoroalkyl). In some embodiments of formula (XVII) , (XVII-A), (XVII-B), or (XVII-C), G is F_{T} (e.g., each F_{T} is independently a polyfluoroorgano (e.g., a polyfluoroacyl, such as -(O)_{q}-[C(=O)]ᵣ-(CH₂)ₒ(CF₂)ₚCF₃, in which q is 0, r is 1; o is from 0 to 2; and p is from 0 to 10). In some embodiments of formula (XVII), (XVII-A), (XVII-B), or (XVII-C), each F_{T} includes (CF₂)₅CF₃. The implantable glucose sensors of the invention may include a semipermeable biointerface film (e.g., a monolayer or a bilayer film) containing a base polymer and the biostabilizing additive of formula (XVII). The implantable glucose sensors of the invention may include a semipermeable biointerface film (e.g., a monolayer or a bilayer film) containing a base polymer and the biostabilizing additive of formula (XVII-A). The implantable glucose sensors of the invention may include a semipermeable biointerface film (e.g., a monolayer or a bilayer film) containing a base polymer and the biostabilizing additive of formula (XVII-B). The implantable glucose sensors of the invention may include a semipermeable biointerface film (e.g., a monolayer or a bilayer film) containing a base polymer and the biostabilizing additive of formula (XVII-C).

For any of the biostabilizing additives of the invention formed from a diisocyanate, the diisocyanate may be 3-isocyanatomethyl-3,5,5-trimethyl-cyclohexylisocyanate; 4,4'-methylene bis(cyclohexyl isocyanate) (HMDI); 2,2'-, 2,4'-, and 4,4'-methylene bis(phenyl isocyanate) (MDI); toluene-2,4-diisocyanate; aromatic aliphatic isocyanate, such 1,2-, 1,3-, and 1,4-xylene diisocyanate; meta-tetramethylxylene diisocyanate (m-TMXDI); para-tetramethylxylene diisocyanate (p-TMXDI); hexamethylene diisocyanate (HDI); ethylene diisocyanate; propylene-1,2-diisocyanate; tetramethylene diisocyanate; tetramethylene-1,4-diisocyanate; octamethylene diisocyanate; decamethylene diisocyanate; 2,2,4-trimethylhexamethylene diisocyanate; 2,4,4-trimethylhexamethylene diisocyanate; dodecane-1,12-diisocyanate; dicyclohexylmethane diisocyanate; cyclobutane-1,3-diisocyanate; cyclohexane-1,2-diisocyanate; cyclohexane-1,3-diisocyanate; cyclohexane-1,4-diisocyanate; methyl-cyclohexylene diisocyanate (HTDI); 2,4-dimethylcyclohexane diisocyanate; 2,6-dimethylcyclohexane diisocyanate; 4,4'-dicyclohexyl diisocyanate; 2,4'-dicyclohexyldiisocyanate; 1,3,5-cyclohexane triisocyanate; isocyanatomethylcyclohexane isocyanate; 1-isocyanato-3,3,5-trimethyl-5-isocyanatomethylcyclohexane; isocyanatoethylcyclohexane isocyanate; bis(isocyanatomethyl)-cyclohexane; 4,4'-bis(isocyanatomethyl) dicyclohexane; 2,4'-bis(isocyanatomethyl) dicyclohexane; isophoronediisocyanate (IPDI); 2,4-hexahydrotoluene diisocyanate; 2,6-hexahydrotoluene diisocyanate; 3,3'-dimethyl-4,4'-biphenylene diisocyanate (TODI); polymeric MDI; carbodiimide-modified liquid 4,4'-diphenylmethane diisocyanate; para-phenylene diisocyanate (PPDI); meta-phenylene diisocyanate (MPDI); naphthylene-1,5-diisocyanate; 2,4'-, 4,4'-, or 2,2'-biphenyl diisocyanate; polyphenyl polymethylene polyisocyanate (PMDI); mixtures of MDI and PMDI; mixtures of PMDI and TDI; dimerized uretdione of any isocyanate described herein, such as uretdione of toluene diisocyanate, uretdione of hexamethylene diisocyanate, or a mixture thereof; or a substituted or isomeric mixture thereof.

For any of the biostabilizing additives of the invention formed from an isocyanate trimer, the isocyanate trimer can be hexamethylene diisocyanate (HDI) biuret or trimer, isophorone diisocyanate (IPDI) trimer, hexamethylene diisocyanate (HDI) trimer; 2,2,4-trimethyl-1,6-hexane diisocyanate (TMDI) trimer; a trimerized isocyanurate of any isocyanates described herein, such as isocyanurate of toluene diisocyanate, trimer of diphenylmethane diisocyanate, trimer of tetramethylxylene diisocyanate, or a mixture thereof; a trimerized biuret of any isocyanates described herein; modified isocyanates derived from the above diisocyanates; or a substituted or isomeric mixture thereof.

The biostabilizing additive can include the group F_{T} that is a polyfluoroorgano group having a theoretical molecular weight of from 100 Da to 1,500 Da. For example, F_{T} may be CF₃(CF₂)ᵣ(CH₂CH₂)ₚ- wherein p is 0 or 1, r is 2-20, and CF₃(CF₂)ₛ(CH₂CH₂O)_{χ}, where χ is from 0 to 10 and s is from 1 to 20. Alternatively, F_{T} may be CHₘF₍₃₋ₘ₎(CF₂)ᵣCH₂CH₂- or CHₘF₍₃₋ₘ₎(CF₂)ₛ(CH₂CH₂O)_{χ}-, where m is 0, 1, 2, or 3; χ is an integer from 0 to 10; r is an integer from 2 to 20; and s is an integer from 1 to 20. In certain embodiments, F_{T} is 1H,1H,2H,2H-perfluoro-1-decanol; 1H,1H,2H,2H-perfluoro-1-octanol; 1H,1H,5H-perfluoro-1-pentanol; or 1H,1H-perfluoro-1-butanol, or a mixture thereof. In particular embodiments, F_{T} is (CF₃)(CF₂)₅CH₂CH₂O-, (CF₃)(CF₂)₇CH₂CH₂O-, (CF₃)(CF₂)₅CH₂CH₂O-, CHF₂(CF₂)₃CH₂O-, (CF₃)(CF₂)₂CH₂O-, or (CF₃)(CF₂)₅-. In still other embodiments the polyfluoroalkyl group is (CF₃)(CF₂)₅-, e.g., where the polyfluoroalkyl group is bonded to a carbonyl of an ester group. In certain embodiments, polyfluoroorgano is -(O)_{q}-[C(=O)]ᵣ-(CH₂)ₒ(CF₂)ₚCF₃, in which q is 0 and r is 1, or q is 1 and r is 0; o is from 0 to 2; and p is from 0 to 10.

### Biologically Active Agents

The semipermeable biointerface film of the invention (e.g., a coating in the bilayer semipermeable biointerface film of the invention) may include one or more biologically active agents. Non-limiting examples of the biologically active agents that may be included in the semipermeable biointerface films of the invention include anti-inflammatory agents, anti-infective agents, anesthetics, inflammatory agents, growth factors, angiogenic factors, growth factors, immunosuppressive agents, antiplatelet agents, anticoagulants, ACE inhibitors, cytotoxic agents, anti-sense molecules, and mixtures thereof.

Non-limiting examples of the biologically active agents that may be used in the semipermeable biointerface films of the invention include: anti-inflammatory agents (e.g., corticosteroids and NSAIDs), anti-infective agents, anti-proliferative agents, anesthetics, angiogenic agents, and antispasmodics.

Exemplary anti-inflammatory agents include but are not limited to, for example, nonsteroidal anti-inflammatory drugs (NSAIDs) such as acetometaphen, aminosalicylic acid, aspirin, celecoxib, choline magnesium trisalicylate, diclofenac potasium, diclofenac sodium, diflunisal, etodolac, fenoprofen, flurbiprofen, ibuprofen, indomethacin, interleukin (IL)-10, IL-6 mutein, anti-IL-6 iNOS inhibitors (for example, L-NAME or L-NMDA), Interferon, ketoprofen, ketorolac, leflunomide, melenamic acid, mycophenolic acid, mizoribine, nabumetone, naproxen, naproxen sodium, oxaprozin, piroxicam, rofecoxib, salsalate, sulindac, and tolmetin; and corticosteroids such as cortisone, hydrocortisone, methylprednisolone, prednisone, prednisolone, betamethesone, beclomethasone dipropionate, budesonide, dexamethasone sodium phosphate, flunisolide, fluticasone propionate, paclitaxel, tacrolimus, tranilast, triamcinolone acetonide, betamethasone, fluocinolone, fluocinonide, betamethasone dipropionate, betamethasone valerate, desonide, desoximetasone, fluocinolone, triamcinolone, triamcinolone acetonide, clobetasol propionate, and dexamethasone.

Exemplary immunosuppressive and/or immunomodulatory agents include anti-proliferative, cell-cycle inhibitors, (for example, paclitaxel, cytochalasin D, infiximab), taxol, actinomycin, mitomycin, thospromote VEGF, estradiols, NO donors, QP-2, tacrolimus, tranilast, actinomycin, everolimus, methothrexate, mycophenolic acid, angiopeptin, vincristing, mitomycine, statins, C MYC antisense, sirolimus (and analogs), RestenASE, 2-chloro-deoxyadenosine, PCNA Ribozyme, batimstat, prolyl hydroxylase inhibitors, PPARγ ligands (for example troglitazone, rosiglitazone, pioglitazone), halofuginone, C-proteinase inhibitors, probucol, BCP671, EPC antibodies, catchins, glycating agents, endothelin inhibitors (for example, Ambrisentan, Tesosentan, Bosentan), Statins (for example, Cerivasttin), *E. coli* heat-labile enterotoxin, and advanced coatings.

Exemplary anti-infective agents include, but are not limited to, anthelmintics (mebendazole), antibiotics including aminoclycosides (gentamicin, neomycin, tobramycin), antifungal antibiotics (amphotericin b, fluconazole, griseofulvin, itraconazole, ketoconazole, nystatin, micatin, tolnaftate), cephalosporins (cefaclor, cefazolin, cefotaxime, ceftazidime, ceftriaxone, cefuroxime, cephalexin), beta-lactam antibiotics (cefotetan, meropenem), chloramphenicol, macrolides (azithromycin, clarithromycin, erythromycin), penicillins (penicillin G sodium salt, amoxicillin, ampicillin, dicloxacillin, nafcillin, piperacillin, ticarcillin), tetracyclines (doxycycline, minocycline, tetracycline), bacitracin; clindamycin; colistimethate sodium; polymyxin b sulfate; vancomycin; antivirals including acyclovir, amantadine, didanosine, efavirenz, foscarnet, ganciclovir, indinavir, lamivudine, nelfinavir, ritonavir, saquinavir, silver, stavudine, valacyclovir, valganciclovir, zidovudine; quinolones (ciprofloxacin, levofloxacin); sulfonamides (sulfadiazine, sulfisoxazole); sulfones (dapsone); furazolidone; metronidazole; pentamidine; sulfanilamidum crystallinum; gatifloxacin; and sulfamethoxazole/trimethoprim.

Exemplary angiogenic agents which can be used in the methods and compositions of the invention include, without limitation, Sphingosine-1-Phosphate (S1P), Basic Fibroblast Growth Factor (bFGF), (also known as Heparin Binding Growth Factor-II and Fibroblast Growth Factor II), Acidic Fibroblast Growth Factor (aFGF), (also known as Heparin Binding Growth Factor-I and Fibroblast Growth Factor-I), Vascular Endothelial Growth Factor (VEGF), Platelet Derived Endothelial Cell Growth Factor BB (PDEGF-BB), Angiopoietin-1, Transforming Growth Factor Beta (TGF-Beta), Transforming Growth Factor Alpha (TGF-Alpha), Hepatocyte Growth Factor, Tumor Necrosis Factor-Alpha (TNF-Alpha), Placental Growth Factor (PLGF), Angiogenin, Interleukin-8 (IL-8), Hypoxia Inducible Factor-I (HIF-1), Angiotensin-Converting Enzyme (ACE) Inhibitor Quinaprilat, Angiotropin, Thrombospondin, Peptide KGHK, Low Oxygen Tension, Lactic Acid, Insulin, Copper Sulphate, Estradiol, prostaglandins, cox inhibitors, endothelial cell binding agents (e.g., decorin or vimentin), glenipin, nicotine, and Growth Hormone.

The general principles according to which one or more biologically active agents may be included in a semipermeable biointerface film of the invention are described in U.S. Patent No. 7,875,293.

### Preparation of Implantable Glucose Sensors

The implantable glucose sensors may be prepared and used according to the principles known in the art for the assembly of implantable glucose sensors and for their use. For example, such principles are described in U.S. Patent Nos. 6,702,857; 6,413,393; 6,368,274; 5,786,439; 5,777,060; 5,391,250; 5,390,671; 5,322,063; 5,165,407; 4,890,620; 4,484,987; 5,390,671; 5,390,691; 5,391,250; 5,482,473; 5,299,571; 5,568,806; 7,310,544; 7,379,765; 7,875,293; 7,882,611; 8,050,731; 8,251,906; 8,255,030; and 8,844,057; U.S. Pre-grant Publication No. 2002/0090738, 2005/0245799, 2014/0088383 2015/0182115, and 2015/0025631; and International Patent Application Publication Nos. WO 2001/58348, WO 2003/034902, WO 2003/035117, WO 2003/035891, WO 2003/023388, WO 2003/022128, WO 2003/022352, WO 2003/023708, WO 2003/036255, WO 2003/036310, WO 2003/074107, and WO 2006/018425. Principles typically utilized for monitoring glucose concentrations in a subject are further described in Shichiri et al., Horm. Metab. Res., Suppl. Ser. 20:17-20, 1988; Bruckel et al., Klin. Wochenschr. 67:491-495, 1989; Pickup et al., Diabetologia 32:213-217, 1989; and Vaddiraju et al., J. Diabetes Sci. Tech. 4:1540-1562, 2010.

Semipermeable biointerface films for use in the glucose sensors of the invention can be prepared according to methods known in the art for preparation of coated or uncoated membranes from base polymers with additives. A membrane adapted for use in the glucose sensors of the invention can be produced using processes known for the manufacture of semipermeable membranes useful in the manufacture of continuous glucose monitors. Such membranes are often made from natural cellulose, cellulose derivatives (e.g. cellulose acetates), or synthetic polymers (e.g., silicone, polycarbonate, polypropylene (PP), polyvinylchloride (PVC), polyvinyl alcohol (PVA), polyvinylpyrrolidone (PVP), polyacrylamide (PAAM), polyethylene oxide, polyethylene oxide)-*b-*poly(propylene oxide)-*b*-poly(ethylene oxide), poly(hydroxyethylmethacrylate) (polyHEMA), polyethylene terephthalate (PET), polybutylene terephthalate (PBT), polymethylmethacrylate (PMMA), polyether ether ketone (PEEK), polyamide, polyurethane, polysulfone, or a copolymer thereof or a blend thereof). Typical membrane fabrication techniques (e.g., solvent casting, molding, or spin casting) may be utilized in the preparation of a semipermeable biointerface film for use in the glucose sensors of the invention.

When the semipermeable biointerface film is a monolayer film (an uncoated semipermeable biointerface membrane), the monolayer film may be prepared from a liquid mixture (e.g., a melt or a solution, suspension, or emulsion in a solvent) of one or more base polymers and one or more biostabilizing additives described herein. Non-limiting examples of the methods for preparation of membranes of the invention include solvent casting, molding, or spin casting.

When the semipermeable biointerface film is a bilayer film (a coated semipermeable membrane), the bilayer film may be formed by coating a semipermeable membrane with a coating composition containing one or more biostabilizing additives described herein. Typical coating techniques that may be used for the preparation of bilayer semipermeable biointerface films of the invention include those known in the art. Non-limiting examples of coating techniques include solid deposition, spray coating, printing, and dip coating.

The following examples are meant to illustrate the invention. They are not meant to limit the invention in any way.

### Examples

### Example 1. Preparation of Biostabilizing Additives

The biostabilizing additives used in the glucose sensors of the invention can be prepared using methods known in the art from the appropriately selected reagents, such as diisocyanates/triisocyanates, dicarboxylic acids, diols, and fluorinated alcohols to form a wide range of biostabilizing additives. The reagents include but are not limited to the component reagents mentioned below.

### Diisocyanates

HMDI = 4,4'-methylene bis(cyclohexyl isocyanate)
IPDI = Isophorone Diisocyanate
TMXDI = m-tetramethylenexylene diisocyanate
HDI = Hexamethylene Diisocyanate

### Triisocyanates

Desmodur N3200 or Desmodur N-3200 = hexamethylene diisocyanate (HDI) biuret trimer Desmodur Z4470A or Desmodur Z-4470A = isophorone diisocyanate (IPDI) trimer
Desmodur N3300 = hexamethylene diisocyanate (HDI) trimer

### Diols/Polyols

HLBH = Hydrogenated-hydroxyl terminated polybutadiene,
PCN = Poly(2,2-dimethyl-1-3-propylenecarbonate) diol
PHCN = Poly(hexamethylene carbonate)diol
PEB = Poly(Ethylene-co-Butylene)diol
LBHP = Hydroxyl terminated polybutadiene polyol
PEGA = Poly(diethylene glycol)adipate
PTMO = Poly(tetramethylene oxide) diol
PDP = Diethylene Glycol-Ortho phthalic Anhydride polyester polyol
HHTPI = hydrogenated hydroxyl terminated polyisoprene
C22 = hydroxylterminated polydimethylsiloxanes block copolymer
C25 (Diol) = Hydroxy Terminated Polidimethylsiloxane (Ethylene Oxide-PDMS-Ethylene Oxide) block copolymer
C10 (Diol) = Hydroxy Terminated Polidimethylsiloxane (Ethylene Oxide-PDMS-Ethylene Oxide) block copolymer
PLN = Polyethylene glycol)-*block*-poly(propylene glycol))-*block*-poly(ethylene glycol) polymer
(PEO-PPO-PEO Pluronic polymers)
PLN8K = Polyethylene glycol)-*block*-poly(propylene glycol))-*block*-poly(ethylene glycol) polymer (PEO-PPO-PEO Pluronic polymers)
DDD = 1,12-dodecanediol
SPH = 1,6-hexanediol-Ortho Phthalic anhydride polyester polyol
SPN = Neopentyl glycol-Ortho Phthalic Anhydride polyester polyol
BPAE = Bisphenol A Ethoxylate diol
YMer (Diol) = Hydroxy Terminated Polyethylene glycol monomethyl ether
YMerOH(Triol) = Trimethylolpropane Ethoxylate
XMer (Tetraol) = Pentaerythritol Ethoxylate

### FLUORINATED END-CAPPING GROUPS

C6-FOH = (CF₃)(CF₂)₅CH₂CH₂OH (1H,1H,2H,2H Perfluorooctanol)
C8-FOH = 1H,1H,2H,2H Perfluorooctanol
C6-C8 FOH = (CF₃)(CF₂)₇CH₂CH₂OH and (CF₃)(CF₂)₅CH₂CH₂OH (Mixtures of C6- FOH and C8-FOH; also designated as BAL-D)
C10-FOH = 1H,1H,2H,2H Perfluorodecanol
C8-C10 FOH = mixtures of C8-FOH and C10-FOH
C5-FOH = 1 H,1H,5H-perfluoro-1-pentanol
C4-FOH = 1H,1H-perfluorobutanol
C3-FOH = (CF₃)(CF₂)₂CH₂OH (1H,1H perfluorobutanol)

### NON-TIN BASED CATALYST

Bi348 - Bismuth Carboxylate Type 1
Bi221- Bismuth Carboxylate Type 2
Bi601- Bismuth Carboxylate Type 3

The bismuth catalysts listed above can be purchased from King Industries (Norwalk CT). Any bismuth catalyst known in the art can be used to synthesize the biostabilizing additives described herein. Also, tin-based catalysts useful in the synthesis of polyurethanes may be used instead of the bismuth-based catalysts for the synthesis of the biostabilizing additives described herein.

Compounds 1 to 36 are only reference compounds and not according to the present invention.

### Compound 1

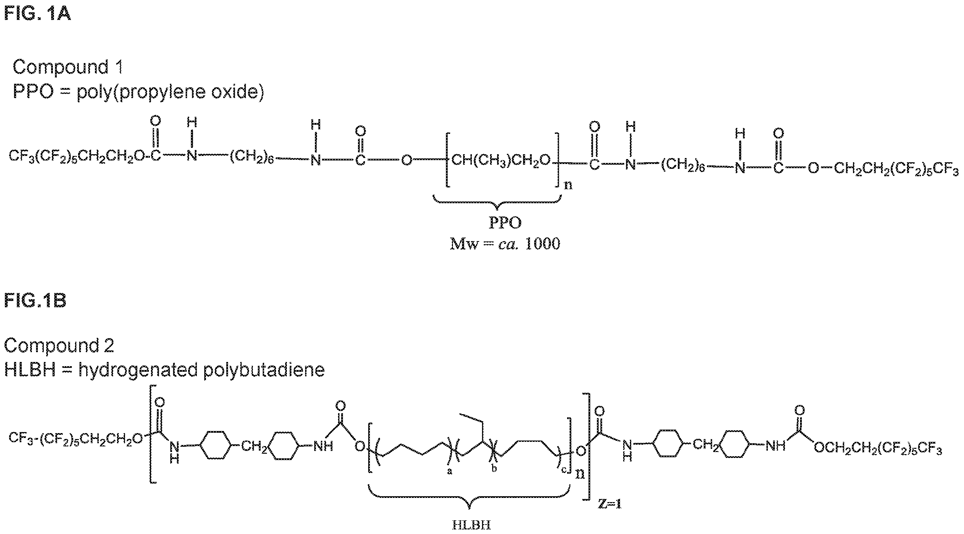

Compound 1 was synthesized with PPO diol of molecular weight 1000, 1,6-hexamethylene diisocyanate (HDI), and the low boiling fraction of the fluoroalcohol (BA-L). The conditions of the synthesis were as follows: 10 grams of PPO were reacted with 3.36 grams of HDI for two hours, and then 5 grams of BA-L (low boiling fraction) were added to the reaction. The mixture was reacted with 42.5 mg of the catalyst, dibutyltin dilaurate, in 130 mL of dimethylacetamide, and the reaction temperature for the prepolymer step was maintained within 60-70° C. The polystyrene equivalent weight average molecular weight is 1.6+/-0.2×10⁴ and its total fluorine content is 18.87+/-2.38% by weight. Thermal transitions for compound 1 are detectable by differential scanning calorimetry. Two higher order thermal transitions at approximately 14° C. and 85° C were observed. The theoretical chemical structure of the compound 1 is shown Figure 1A.

### Compound 2

All glassware used for the synthesis was dried in an oven at 110°C overnight. To a 3- necked 1000 mL oven dried flask equipped with a stir bar was added 175 g (72 mmol) of hydrogenated-hydroxyl terminated polybutadiene (HLBH polyol, MW = 2000). The flask with the polyol was degassed overnight and then purged with dry N₂. A 1000 mL graduated cylinder was filled with 525 mL anhydrous Toluene, sealed by a rubber septa and purged with dry N₂. The toluene was transferred to the 3-necked flask via a double-edged needle and the polyol stirred vigorously to dissolve in the solvent. The flask was placed in an oil bath at 65-70 °C. 39.70 g (151 mmol) of 4,4'-methylene bis(cyclohexyl isocyanate) (HMDI) was added to a degassed 250 mL flask equipped with a stir bar. To this flask was added 150 mL of anhydrous toluene from a degassed, N₂ purged 250 mL septa-sealed cylinder also using a double-edged needle and the mixture was stirred to dissolve the HMDI in the solvent. To a degassed 50 mL round bottom flask was added 8.75 g (5.00 % w/w based on diol) of the bismuth carboxylate catalyst followed by 26 mL of toluene to dissolve the catalyst. The HMDI solution was transferred to the 1000 mL flask containing the polyol. The bismuth catalyst solution was added (20 mL) immediately following the addition of the HMDI. The reaction mixture was allowed to stir for 5 h at 70 °C to produce a HMDI-HLBH prepolymer.

In another 50 mL round bottom flask 74.95 g (180 mmol) of C8-C10 FOH (mixture of C8-FOH and C10-FOH) was added, capped with a septa, degassed and then purged with N₂. This was added to the 1000 mL flask containing prepolymer. All additions and transfers were conducted carefully in an atmosphere of dry N₂ to avoid any contact with air. The resulting mixture was heated to 45 °C for 18 hours to produce SMM (**1**) with the end-capped C8-C10 FOH. The SMM solution was allowed to cool to ambient temperature and formed a milky solution. The milky solution was precipitated in MeOH (methanol) and the resulting precipitate was washed repeatedly with MeOH to form a white viscous material with dough-like consistency. This viscous, semi-solid material was washed twice in THF/EDTA (Ethylene Diamine Tetraacetic Acid) to remove residual catalyst followed by two more successive washes in THF/MeOH to remove unreacted monomers, low molecular weight byproducts, and catalyst residues. The SMM was first dried in a flow oven from at 40-120 °C in a period of 10 hours gradually raising the temperature and finally dried under vacuum at 120 °C (24 hours) and stored in a desiccator as a colorless rubbery semi-solid. The theoretical chemical structure of compound 2 is shown Figure 1B.

### Compound 3

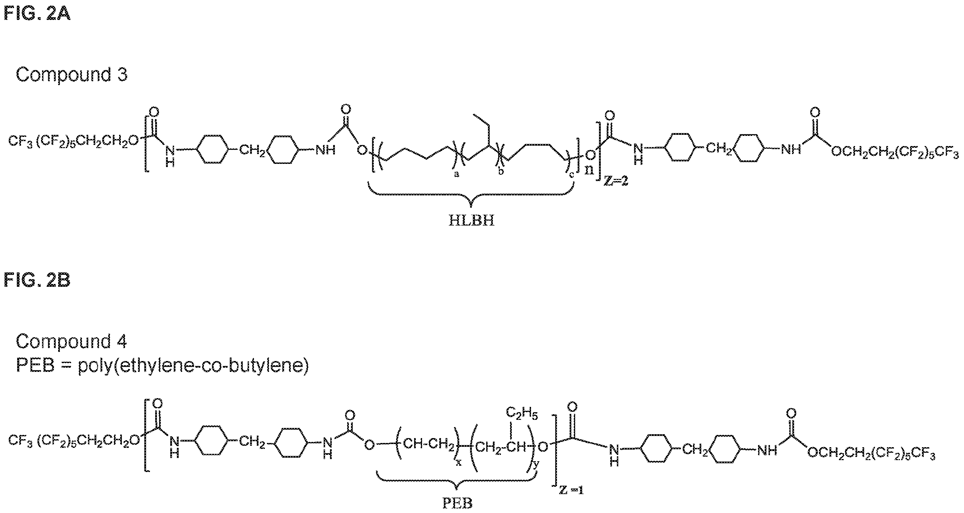

The reaction was carried out as described for compound 2 using 180 g (74 mmol) hydrogenated-hydroxyl terminated polybutadiene (HLBH polyol, MW = 2000) and 30.14 g (115 mmol) of 4,4'-methylene-bis(cyclohexyl isocyanate) (HMDI) to form the prepolymer. The prepolymer was end-capped with 40.48 g (111.18 mmol) of 1H,1H,2H,2H-perfluoro-1-octanol (C8-FOH ) to form compound 3 as a colorless rubbery semi-solid. As described above, the couplings were carried out in the presence of bismuth carboxylate catalyst, and compound 3 was washed similarly to compound 2 and dried prior to use. The theoretical chemical structure of compound 3 is shown in Figure 2a.

### Compound 4

The reaction was carried out as described for compound 3 using 10 g (4 mmol) poly(ethylene-co-butylene (PEB polyol, MW= 2500) and 2.20 g (8.4 mmol) of 4,4'-methylene-bis(cyclohexyl isocyanate) (HMDI) to form the prepolymer. The prepolymer was capped with 3.64 g (10 mmol) of 1H, 1H, 2H, 2H-perfluoro-1-octanol (C8-FOH) to form compound 4. As described above, the couplings were carried out in the presence of bismuth carboxylate catalyst, and the compound 4 was washed similarly to compound 2 and dried prior to use. The theoretical chemical structure of compound 4 is shown in Figure 2B.

### Compound 5

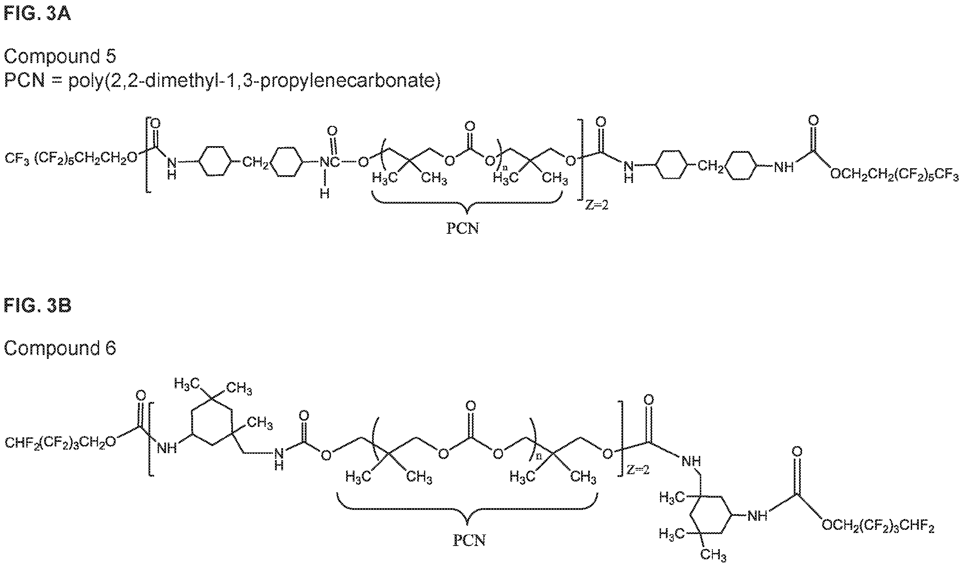

The reaction was carried out as described for compound 4, except the solvent was changed from toluene to DMAc. Here, 100 g (100 mmol) poly(2,2-dimethyl-1,3-propylenecarbonate) diol (PCN, MW 1000) and 40.7 g (155 mmol) of 4,4'-methylene-bis(cyclohexyl isocyanate) (HMDI) to form a prepolymer. The prepolymer was end-capped with 45.5 g (125 mmol) of 1H,1H,2H,2H-perfluoro-1-octanol (C8-FOH) to form compound 5. The work-up after the reaction and the subsequent washing procedures are modified from the compound 4 synthesis as follows. Compound 5 from the reaction mixture in DMAc was precipitated in distilled water and washed successively in IPA/EDTA (Isopropanol/Ethylene Diamine Tetraacetic Acid) solution followed by another wash in IPA/hexanes to remove unreacted monomers, low molecular weight byproducts, and catalyst residues to yield compound 5 as a white amorphous powder. As described above, the couplings were carried out in the presence of bismuth carboxylate catalyst and dried under vacuum prior to use. The theoretical chemical structure of compound 5 is shown in Figure 3A.

### Compound 6

The reaction was carried out as described for compound 5 using 6.0 g (6.0 mmol) poly(2,2 dimethyl-1,3-propylenecarbonate) diol (MW 1000) and 1.90 g (8.5 mmol) of isophorone diisocyanate (IPDI) to form the prepolymer. The prepolymer was end-capped with 1.4 g (6.0 mmol) of 1H,1H,5H-perfluoro-1-pentanol (C5-FOH) to form compound 6 as a white amorphous solid. As described above, the couplings were carried out in the presence of bismuth carboxylate catalyst, and compound 6 was washed similarly to compound 5 and dried prior to use. The theoretical chemical structure of compound 6 is shown in Figure 3B.

### Compound 7

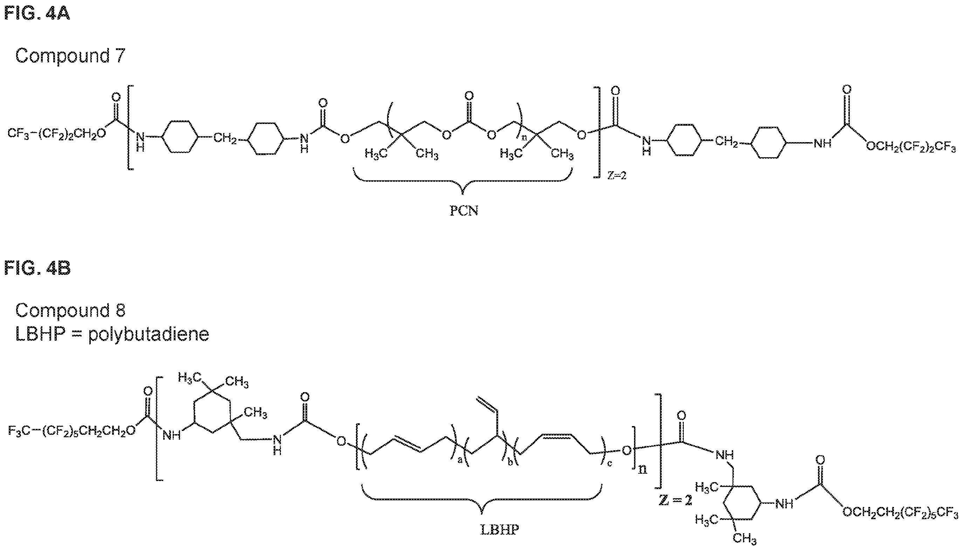

The reaction was carried out as described for compound 5 using 10.0 g (10.0 mmol) poly(2,2-dimethyl-1 ,3-propylenecarbonate) diol (MW 1000) and 4.07 g (15.5 mmol) of 4,4'-methylene-bis(cyclohexyl isocyanate) (HMDI) to form the prepolymer. The prepolymer was capped with 2.5 g (12.5 mmol) of 1H, 1H-Perfluoro-1-butanol (C4-FOH) to form compound 8 as a white amorphous solid. As described above, the couplings were carried out in the presence of bismuth carboxylate catalyst, and compound 7 was washed similar to compound 5 and dried prior to use. The theoretical chemical structure of compound 7 is shown in Figure 4A.

### Compound 8

The reaction was carried out as described for compound 5 using 180 g (84.8 mmol) hydroxylterminated polybutadiene (LBHP polyol, MW= 2000) and 29.21 g (131.42 mmol) of isophorone diisocyanate (IPDI) to form the prepolymer. The prepolymer was capped with 46.31 g (127.18 mmol) of 1H,1H,2H,2H-perfluoro-1-octanol (C8-FOH) to form compound 8 as an off-white opaque viscous liquid. As described above, the couplings were carried out in the presence of bismuth carboxylate catalyst, and compound 8 was washed similarly to compound 5 and dried prior to use. The theoretical chemical structure of compound 8 is shown in Figure 4B.

### Compound 9

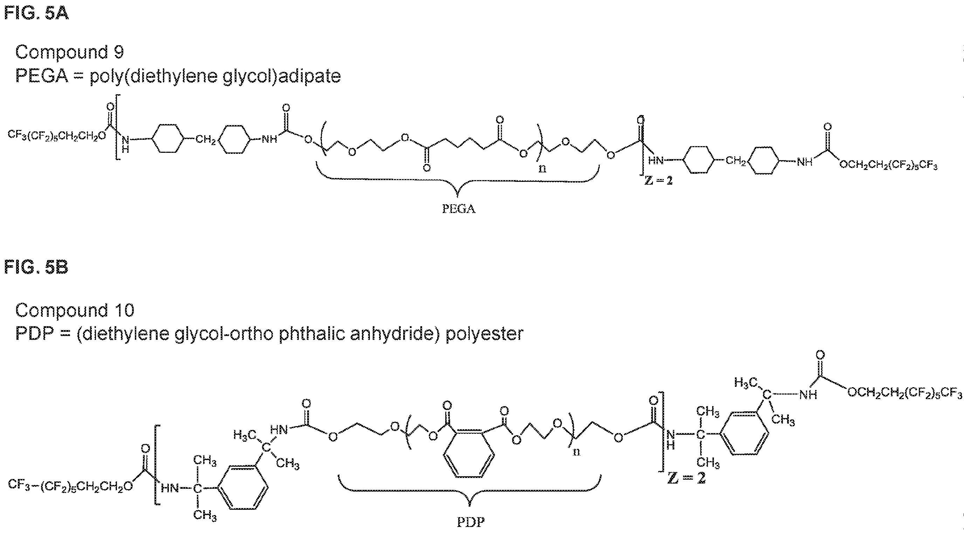

The reaction was carried out as described for compound 5 using 10 g (3.92 mmol) poly(diethyhlene glycol adipate) (PEGA polyol, MW= 2500) and 1.59 g (6.08 mmol) of 4,4'-methylene-bis(cyclohexyl isocyanate) (HMDI) to form a prepolymer. The prepolymer was capped with 2.14 g (5.88 mmol) of 1H,1H,2H,2H-perfluoro-1-octanol (C8-FOH) to form compound 9 as an off-white opaque viscous liquid. As described above, the couplings were carried out in the presence of bismuth carboxylate catalyst, and compound 9 was washed similarly to compound 5 and dried prior to use. The theoretical chemical structure of compound 9 is shown in Figure 5A.

### Compound 10

The reaction was carried out as described for compound 5 using 10 g (5.06 mmol), ortho phthalate-diethylene glycol-based polyester polyol (PDP polyol, MW = 2000) and 1.92 g (7.85 mmol) of m-tetramethylenexylene diisocyanate (TMXDI) to form a prepolymer. The prepolymer was capped with 2.76 g (7.59 mmol) of 1H,1H,2H,2H-perfluoro-1-octanol (C8-FOH) to form compound 10 as a colorless solid. As described above, the couplings were carried out in the presence of bismuth carboxylate catalyst, and compound 10 was washed similarly to compound 5 and dried prior to use. The theoretical chemical structure of compound 10 is shown in Figure 5B.

### Compound 11

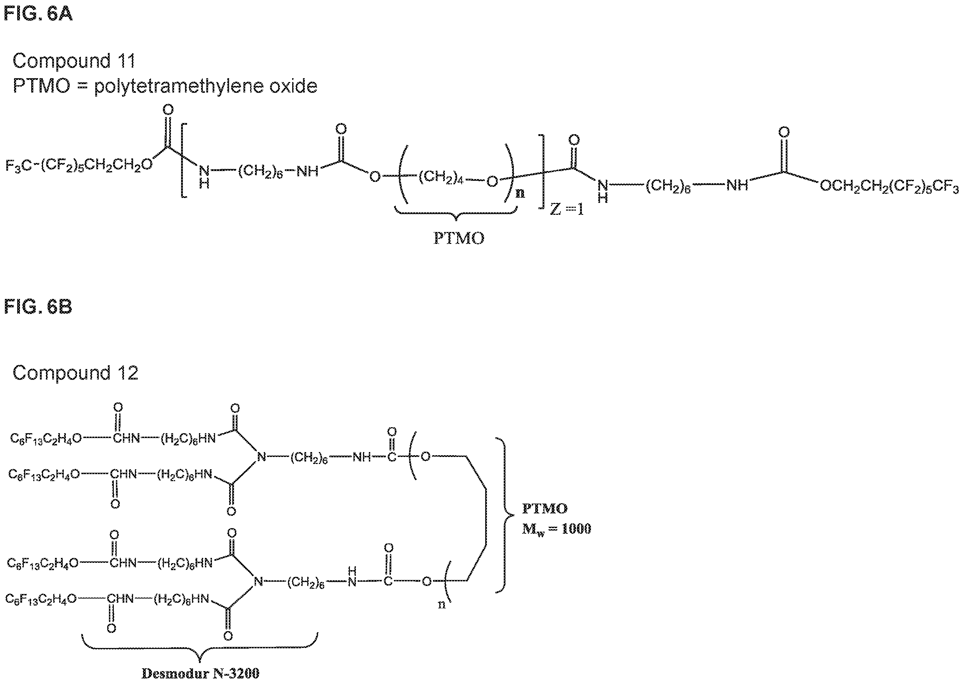

Compound 11 was synthesized with PTMO diol of molecular weight 1000, 1,6-hexamethylene diisocyanate (HDI), and the low boiling fraction of the fluoroalcohol (BA-L). The conditions of the synthesis were as follows: 10 grams of PTMO were reacted with 3.36 grams of HDI for two hours and then 9 grams of BA-L (low boiling fraction) were added to the reaction. The mixture was reacted with 60 mL of the catalyst, dibutyltin dilaurate, in 70 mL of dimethyl-acetamide (DMAc), and the reaction temperature for the prepolymer step was maintained within 60-70° C. The polystyrene equivalent weight average molecular weight is 3.0×10⁴ and its total fluorine content is 7.98% by weight. The theoretical chemical structure of compound 11 is shown in Figure 6A.

### Compounds 12-26

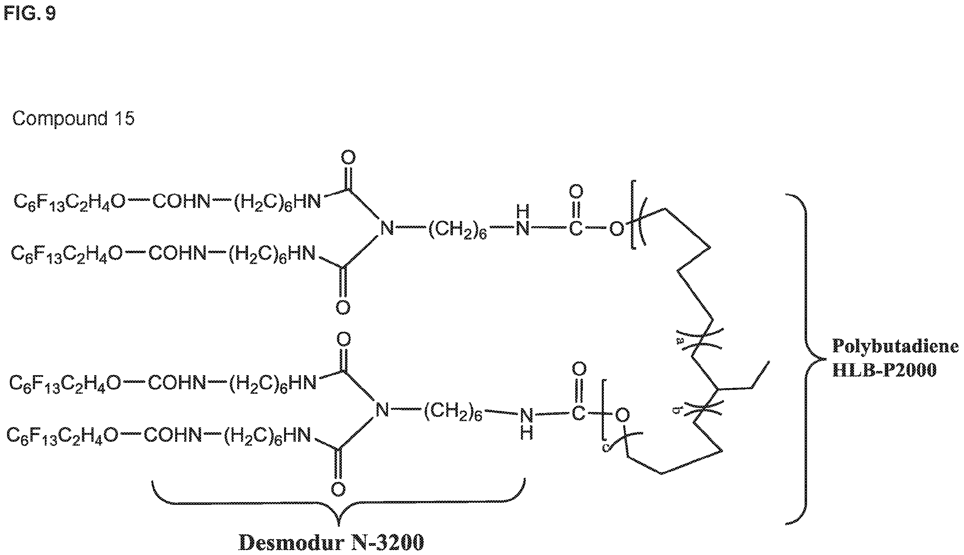

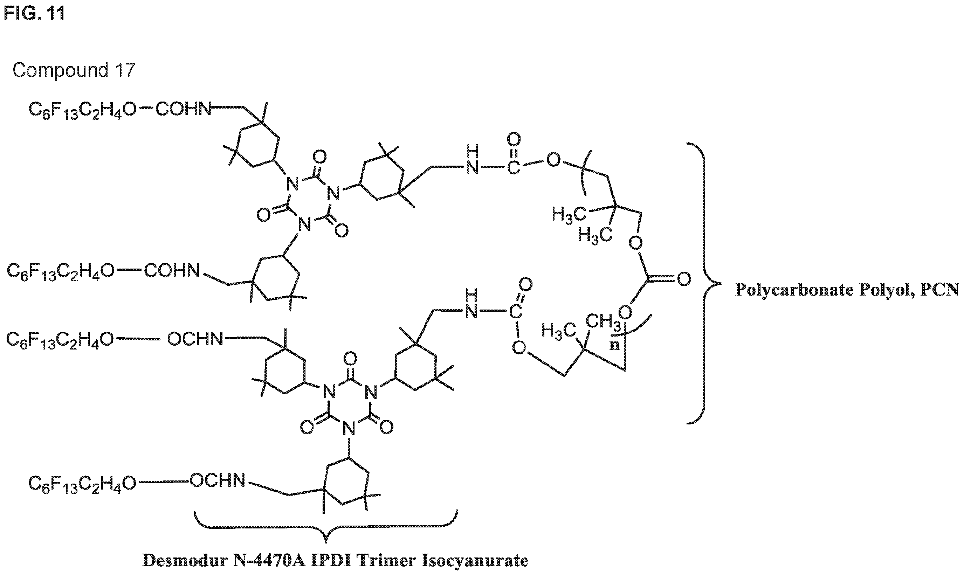

Surface modifiers of the invention such as compound 15 and compound 17 may be synthesized by a 2-step convergent method according to the schemes depicted in schemes 1 and 2. Briefly, the polyisocyanate such as Desmodur N3200 or Desmodur 4470 is reacted dropwise with the surface-active group (e.g., a fluoroalcohol) in an organic solvent (e.g. anhydrous THF or dimethylacetamide (DMAc)) in the presence of a catalyst at 25 °C for 2 hours. After addition of the fluoroalcohol, stirring is continued for 1 hour at 50 °C and for a further 1 hour at 70 °C. These steps lead to the formation of a partially fluorinated intermediate that is then coupled with the polyol (e.g., hydrogenated-hydroxyl terminated polybutadiene, or poly(2,2-dimethyl-1,3-propylenecarbonate)diol) at 70 °C over a period of 14 hours to provide the SMM. Because the reactions are moisture sensitive, they are carried out under an inert N₂ atmosphere and anhydrous conditions. The temperature profile is also maintained carefully, especially during the partial fluorination, to avoid unwanted side reactions. The reaction product is precipitated in MeOH and washed several times with additional MeOH. The catalyst residues are eliminated by first dissolving the biostabilizing additive in hot THF or in hot IPA followed by reacting the biostabilizing additive with EDTA solution, followed by precipitation in MeOH. Finally, the biostabilizing additive is dried in a rotary evaporator at 120-140 °C prior to use. The theoretical chemical structure of compounds 15 and 17 is shown in Figures 9 and 11, respectively.

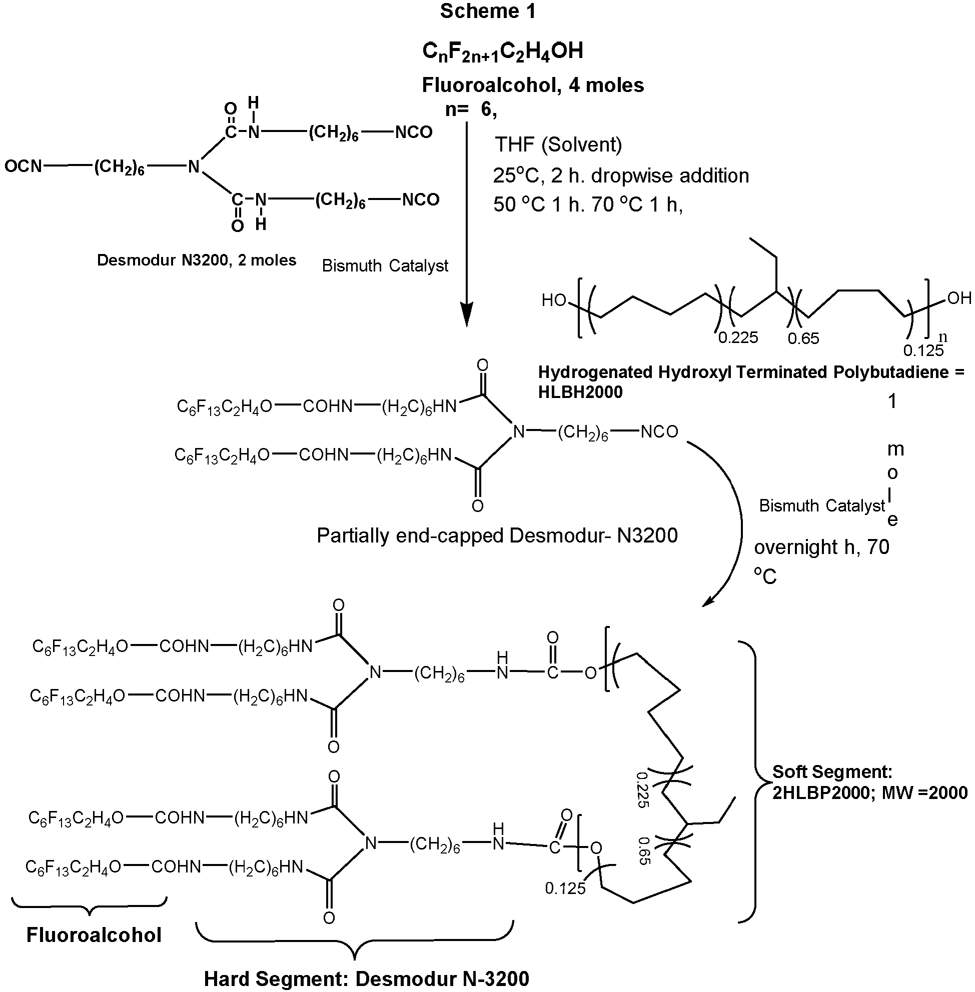

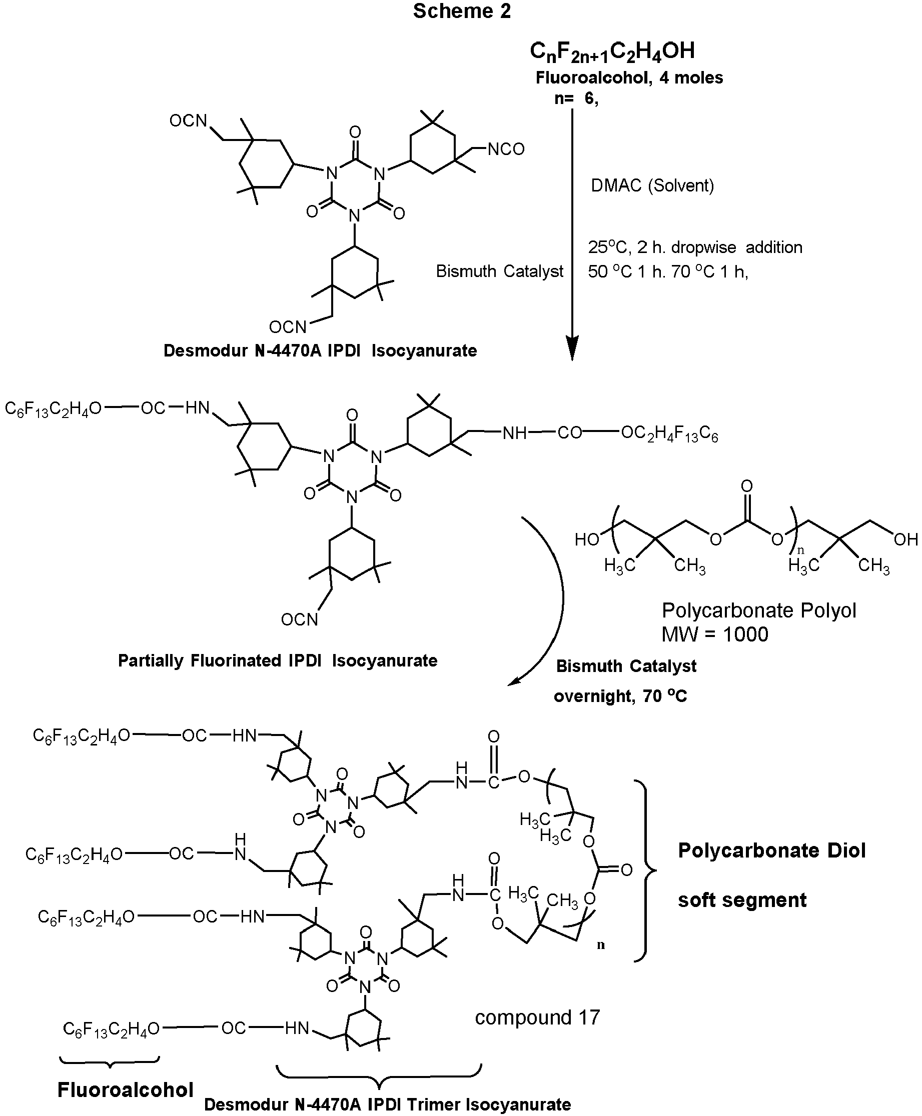

All glassware were dried in the oven overnight at 110 °C. To a 3-necked 5000 mL reactor equipped with a stir bar and a reflux condenser was added 300 g (583 mmol) of Desmodur N3300. The mixture was degassed overnight at ambient temperature. Hydrogenated-hydroxyl terminated polybutadiene (HLBH polyol MW = 2000) was measured into a 2000 mL flask and degassed at 60 °C overnight. The bismuth catalyst K-Kat 348 (a bismuth carboxylate; available from King Industries) was measured out into a 250 mL flask and degassed overnight at ambient temperature. The perfluorinated alcohol was measured into a 1000 mL flask and degassed for 30 minutes at ambient temperature. After degassing, all the vessels were purged with Nitrogen.

300 mL of THF (or DMAc) was then added to the Desmodur N3300 contaning vessel, and the mixture was stirred to dissolve the polyisocyanate. Similarly, 622 mL of THF was added to the HLBH polyol, and the mixture was stirred to dissolve the polyol. Likewise, 428 mL of THF (or DMAC) was added to the perfluorinated alcohol and the mixture was stirred to dissolve. Similarly for K-Kat 348 which was dissolved in 77 mL of THF or DMAC. Stirring was continued to ensure all the reagents were dissolved in their respective vessels.

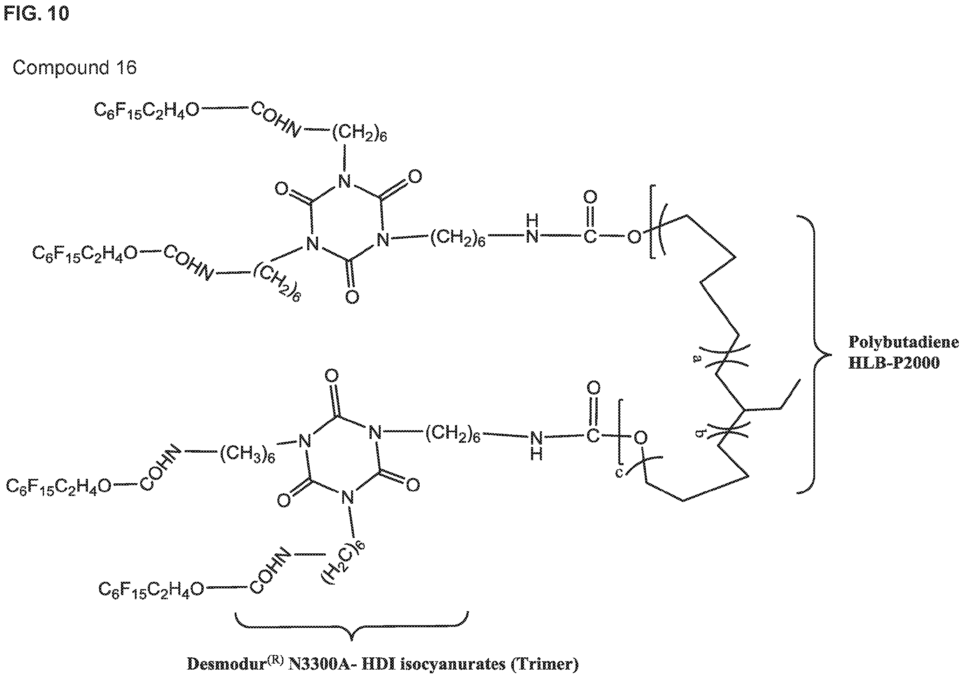

Half the K-Kat solution was transferred to the perfluorinated solution which was stirred for 5 minutes. This solution was added to the reaction vessel containing the Desmodur N3300 solution dropwise over a period of 2 hours at ambient (25 °C) temperature through a cannula (double ended needle) under positive nitrogen pressure. After addition, the temperature was raised to 50 °C for 1 hour and 70 °C for another 1 hour. Proper stirring was maintained throughout. The remaining K-Kat 348 catalyst was transferred to the HLBH-2000 flask; after stirring to dissolve, this was added to the reactor containing the N3300. The reaction mixture was allowed to react overnight for 14 hours at 70 °C to produce compound 16 with four fluorinated end groups. The theoretical chemical structure of compound 16 is shown in Figure 10.

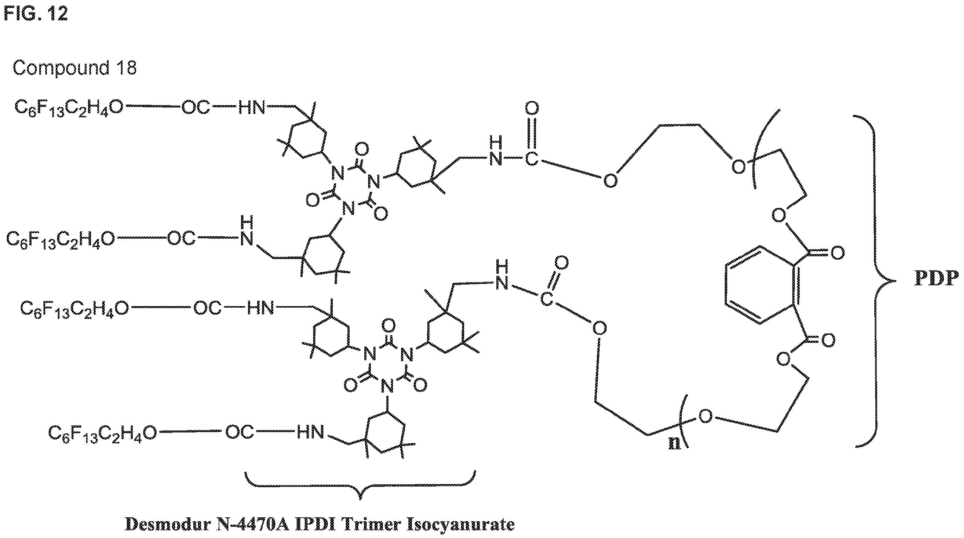

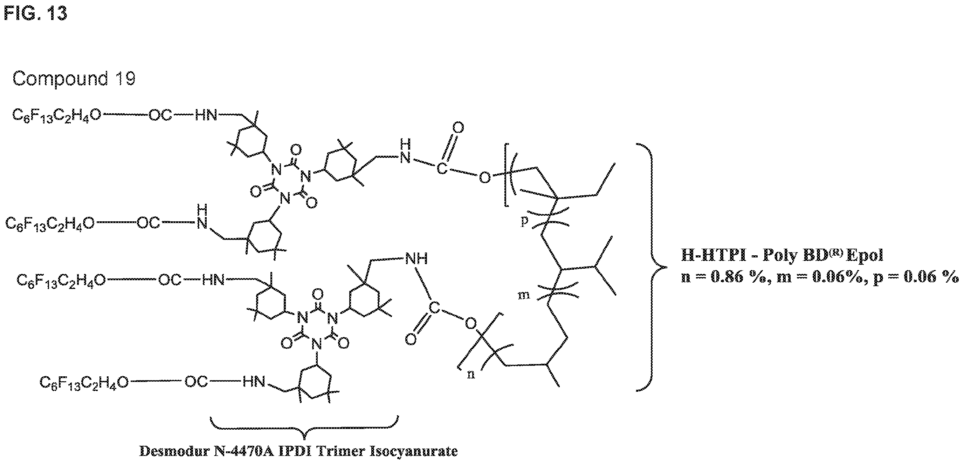

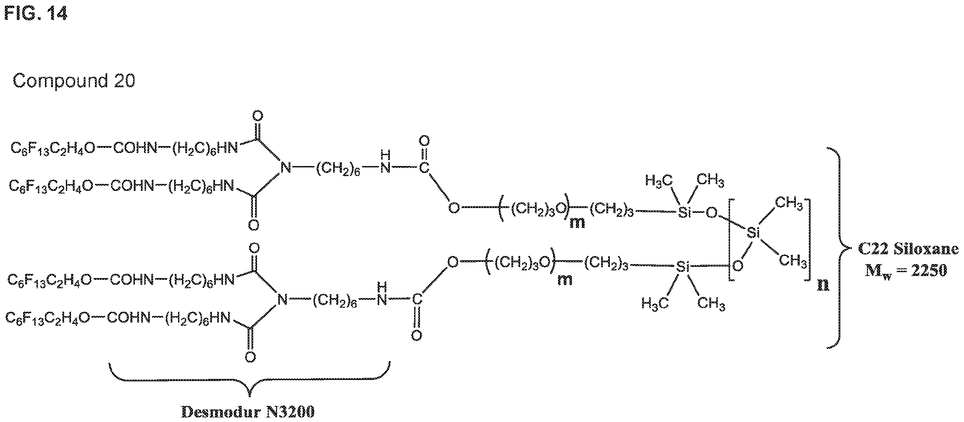

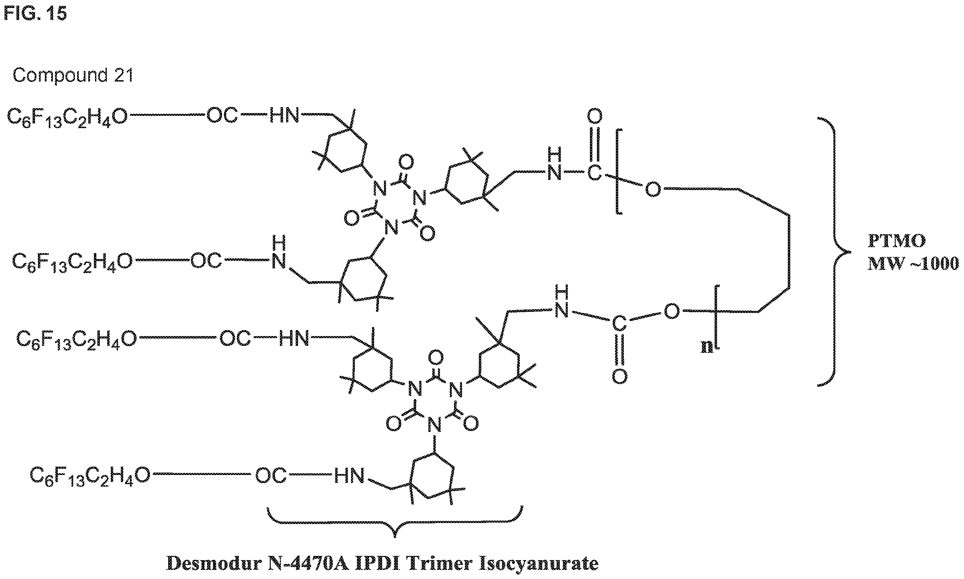

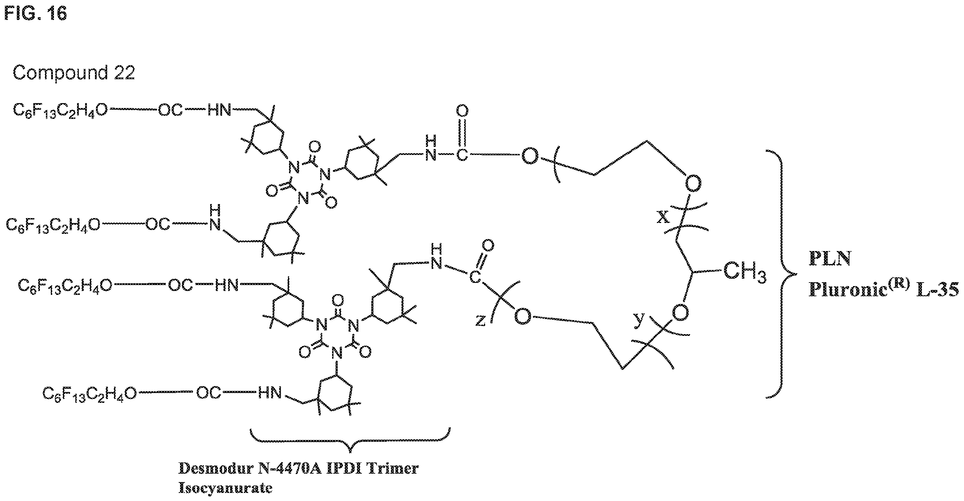

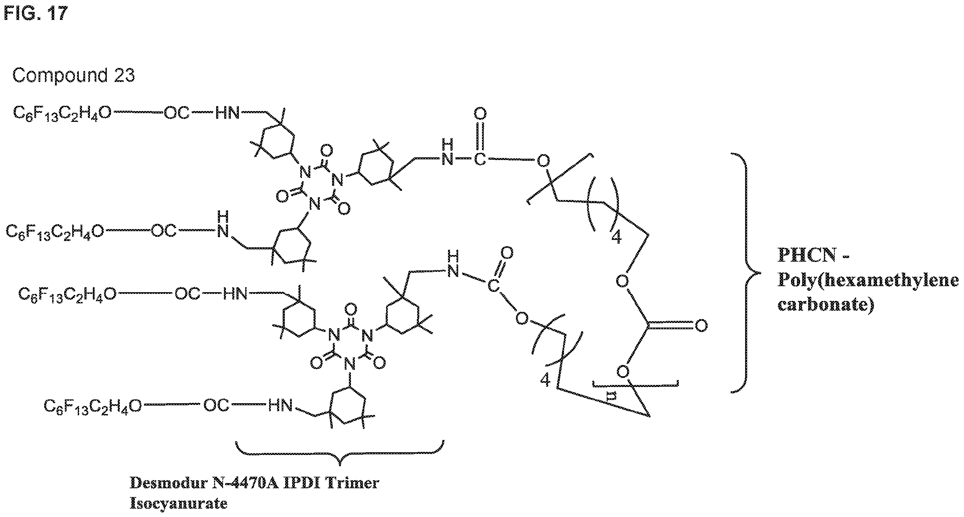

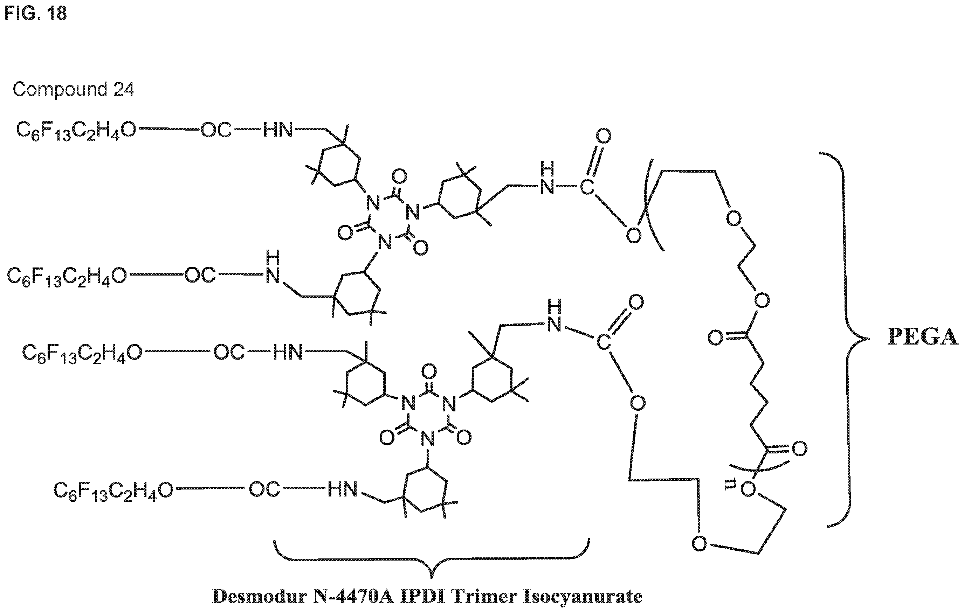

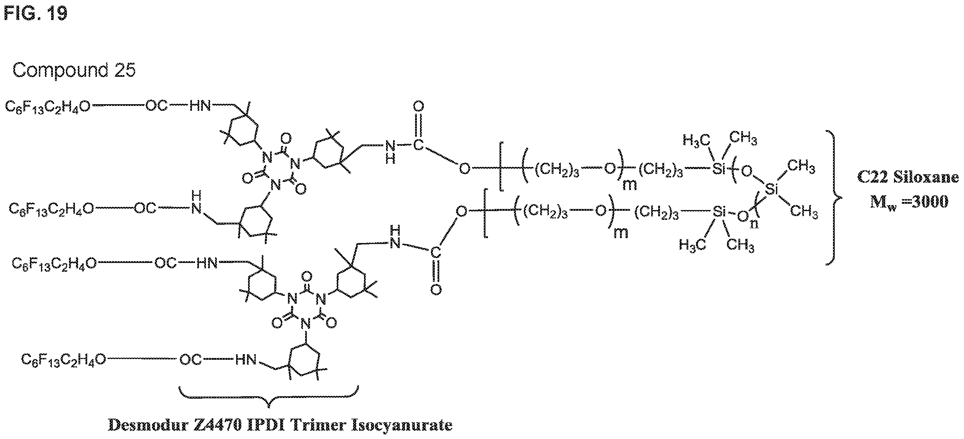

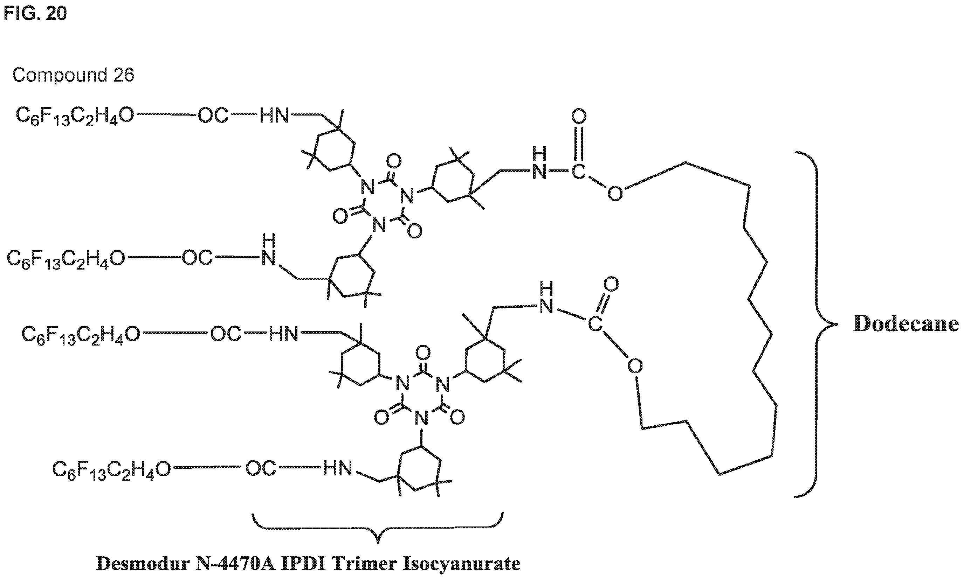

Exemplary biostabilizing additives that can be prepared according to the procedures described for compounds 15-17 are illustrated in Figures 6B and 11-20.

### General Synthesis Description for Ester-based Biostabilizing Additives

A diol such as Ymer diol, hydroxyl terminated polydimethylsiloxane, or polyols such as trimethylolpropane ethoxylate or pentaerythritol ethoxylate are reacted in a one-step reaction with a surface-active group precursor (e.g., perfluoroheptanoyl chloride) at 40 °C in a chlorinated organic solvent e.g. chloroform or methylene chloride in the presence of an acid scavenger like pyridine or triethylamine for 24 h. This reaction end-caps the hydroxyl groups with polyfluoroorgano groups. Because the reactions are moisture sensitive, the reactions are carried out under a nitrogen atmosphere using anhydrous solvents. After the reaction the solvent is rotary evaporated and the product is dissolved in Tetrahydrofuran (THF) which dissolves the product and precipitates the pyridine salts which are filtered off and the filtrate rotary evaporated further to dryness. The product is then purified by dissolving in minimum THF and precipitating in hexanes. This is performed 3 times and after which the final product is again rotary evaporated and finally dried in a vacuum oven at 60 °C overnight.

### Compound 27

Glassware used for the synthesis was dried in an oven at 110°C overnight. To a 2- necked 1000 mL oven dried round bottom flask equipped with a stir bar was added 85 g (24 mmol) of C25-Diol (MW = 3500). The flask with the diol was degassed overnight at 60 °C with gentle stirring and then purged with dry N₂ the following day. The heating was turned off. A 1000 mL graduated cylinder was charged with 320 mL anhydrous CHCl₃, sealed by a rubber septa and purged with dry N₂. The CHCl₃ was transferred to the 2-necked flask via a cannula and the diol stirred vigorously to dissolve in the solvent. Anhydrous pyridine (11.53 g, 146 mmol) was added to the C25-Diol solution using a plastic syringe, and the resulting mixture was stirred to dissolve all materials. Another oven dried 2-necked 1000 mL flask was charged with 32.51 g (85 mmol) of perfluoroheptanoyl chloride. The flask was sealed with rubber septa and degassed for 5 minutes, then purge with nitrogen. At this time 235 mL of anhydrous CHCl₃ were added via cannula to the 1000 mL 2-necked flask containing the perfluoroheptanoyl chloride. Stir at room temperature to dissolve the acid chloride. This flask was fitted with an addition funnel and the C25-Diol -pyridine solution in CHCl₃ was transferred via a cannula into the addition funnel. N₂ flow through the reactor was adjusted to a slow and steady rate. Continuous drop-wise addition of C25-Diol -pyridine solution to the acid chloride solution was started at room temperature and was continued over a period of ~ 4 hours. Stirring was maintained at a sufficient speed to achieve good mixing of reagents. After completing addition of the C25-Diol-pyridine solution, the addition funnel was replaced with an air condenser, and the 2-neck flask was immerses in an oil bath placed on a heater fitted with a thermocouple unit. The temperature was raised to 40 °C, and the reaction continued at this temperature under N₂ for 24 h.

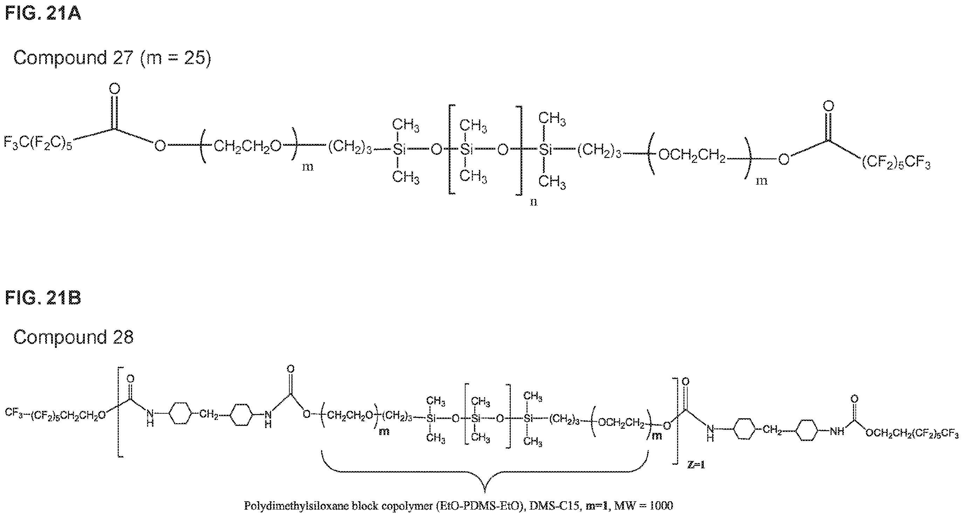

The product was purified by evaporating CHCl₃ in a rotary evaporator and by filtering the pyridine salts after addition of THF. The crude product was then precipitated in isopropanol/hexanes mixture twice. The oil from the IPA/Hexane that precipitated was subjected to further washing with hot hexanes as follows. About 500 mL of Hexanes was added to the oil in a 1 L beaker with a stir bar. The mixture was stirred while the Hexanes was heated to boiling. The heating was turned off, and the mixture was allowed to cool for 5 minutes. The oil settles at the bottom at which point the Hexane top layer is decanted. The isolated oil is further dissolved in THF, transferred to a round bottom flask and then the solvents rotary evaporated. The oil is finally dried in a vacuum oven at 40 °C for 24 h. The purified product (a mixture of di- and mono-substituted products) was characterized by GPC (Molecular Weight based on Polystyrene Standards), elemental analysis for fluorine, ¹⁹F NMR, ¹H NMR, FTIR, and TGA. Appearance: viscous oil. Weight Average molecular weight (polystyrene equivalent) = 5791 g/mol. Polydispersity: 2.85. Elemental analysis: F: 7.15% (theory: 10.53%). ¹⁹F NMR (CDCl₃, 400 MHz. ppm): δ -80.78 (m, CF₃), -118.43 (m, CF₂), -121.85 (m, CF₂), -122.62 (m, CF₂), -126.14 (m, CF₂). ¹H NMR (CDCl₃, 400 MHz): δ ppm = 0.0 (m, **CH₃**Si), 0.3 (br m, **CH₂**Si), 1.4 (br m, CH₂), 3.30 (m, CH₂'s), 4.30 (m, **CH₂**COO-). FTIR, neat (cm⁻¹): 3392 (OH), 2868 (CH₂), 1781 (O-C=O, ester), 1241, 1212, 1141, 1087 (CF₃, CF₂,). The theoretical chemical structure of compound 27 is shown in Figure 21A. Compound 28 can be synthesized using a different ratio of reactants under conditions similar to those described above.

### Compound 29

Glassware used for the synthesis was dried in an oven at 110°C overnight. To a 2- necked 100 mL oven dried round bottom flask equipped with a stir bar was added 10 g (5 mmol) of PDMS C22 - Diol (C22 diol, MW = 3000). The flask with the diol was degassed overnight at 60 °C with gentle stirring and then purged with dry N₂ the following day. Heating was turned off. A 100 mL graduated cylinder was filled with 50 mL anhydrous CHCl₃, sealed with a rubber septum, and purged with dry N₂. The CHCl₃ was transferred to the 2-necked flask via a cannula, and the diol was stirred vigorously to dissolve in the solvent. Anhydrous pyridine (0.53 g, 7 mmol) was then added to the C22-Diol solution using a plastic syringe, and the resulting mixture was stirred to dissolve all materials. Another oven-dried 2-necked 250 mL flask was charged with 3.19 g (8 mmol) perfluoroheptanoyl chloride. The flask was then sealed with a rubber septum, and the mixture in the flask was degassed for 5 minutes and purged with nitrogen. Then, 22 mL of anhydrous CHCl₃ were added using a graduated cylinder and a cannula to transfer the solvent to the 250 mL 2-necked flask containing the perfluoroheptanoyl chloride. The resulting mixture was stirred at room temperature to dissolve the acid chloride. The flask was then equipped with an addition funnel, and the C22 diol/pyridine solution in CHCl₃ was transferred to the addition funnel using a cannula. N₂ flow through the reactor was adjusted to a slow and steady rate. C22 diol/pyridine solution was then added continuously drop-wise to the acid chloride solution at room temperature over a period of ~ 4 hours. Stirring was maintained at a sufficient speed to achieve good mixing of reagents. After completing the addition of the C22 diol, the addition funnel was replaced with an air condenser, and the 2-necked flask was immersed in an oil bath placed on a heater fitted with a thermocouple unit. The temperature was raised to 50 °C, and the reaction mixture was left at this temperature under N₂ for 24 h.

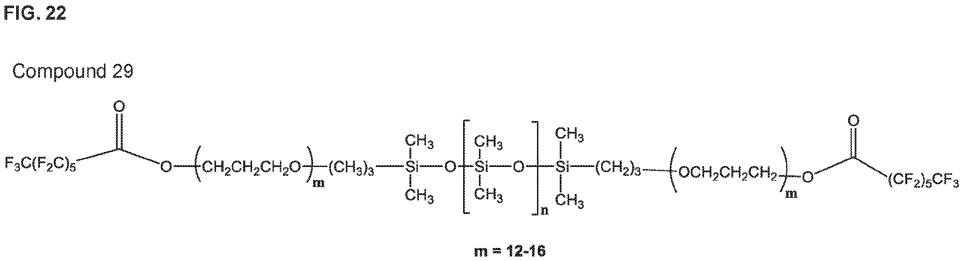

Then, heating and stirring were turned off. The flask was removed and its contents were poured into a round bottom flask. Volatiles were removed by rotary evaporation. Upon concentration, a dense precipitate (pyridine salts) formed. THF was added to dissolve the product, and the precipitated pyridine salts were removed by filtration using a coarse Whatman Filter paper (No 4), as the pyridine salts are insoluble in THF. Volatiles were removed by rotary evaporation. The crude product was then dissolved in 100 mL of CHCl₃ and poured into a separatory funnel. 150 mL of water and 5 mL of 5N HCI were added to neutralize any remaining pyridine. The funnel was shaken, and the product was extracted into CHCl₃. The bottom CHCl₃ layer containing product was then washed in a separatory funnel sequentially with water, 5 mL of 5% (w/v) NaHCO₃ solution to neutralize any remaining HCI, and with distilled water. The CHCl₃ layer was separated and concentrated by rotary evaporation to obtain crude product, which was then dissolved in 10 mL of isopropanol. The resulting solution was added dropwise to a 1 L beaker containing 200 mL of DI Water with 1% (v/v) MeOH with continuous stirring. The product separated out as oil, at which time the solution was kept in an ice bath for 20 minutes, and the top aqueous layer was decanted. The oil was dissolved in THF and transferred into a 200 mL round bottom flask. The volatiles were removed by rotary evaporation at a maximum of 80 °C and 4 mbar to remove residual solvents. The resulting product was dried in a vacuum oven at 60 °C for 24 h to give a purified product as a light yellow, clear oil (~64 % yield). The purified product was characterized by GPC (Molecular Weight based on Polystyrene Standards), and elemental analysis (for fluorine). Appearance: Light Yellow clear oil. Weight Average Molecular Weight (Polystyrene equivalent) Mw = 5589, Polydispersity PD = 1.15. Elemental Analysis F: 12.86 % (theory: 13.12 %). The theoretical chemical structure of compound 29 is shown in Figure 22.

### Compound 30

Glassware used for the synthesis was dried in an oven at 110°C overnight. To a 2-necked 250 mL oven dried round bottom flask equipped with a stir bar was added 20 g (8.0 mmol) of hydrogenated-hydroxyl terminated polybutadiene (HLBH diol, MW= 2000). The flask with the diol was degassed overnight at 60 °C with gentle stirring and then purged with dry N₂ the following day. At this time, the heating was turned off. A 200 mL graduated cylinder was charged with 104 mL anhydrous CHCl₃, sealed by a rubber septa, and purged with dry N₂. The CHCl₃ was transferred to the 2-necked flask via a cannula, and the diol was stirred vigorously to dissolve in the solvent. At this time, anhydrous pyridine (3.82 g, 48 mmol) was added to the HLBH diol solution using a plastic syringe, and the resulting mixture was stirred to dissolve all materials. Another oven dried 2-necked 100 mL flask was charged with trans-5-norbornene-2,3-dicarbonyl chloride ("NCI"; 3.70 g, 17 mmol), sealed with rubber septa, and degassed for 5 minutes, and then purged with nitrogen. At this time, 52 mL of anhydrous CHCl₃ were added using a graduated cylinder and a cannula to transfer the solvent to the 100 mL 2-necked flask containing NCI. The resulting mixture was stirred to dissolve NCI. The 250 mL 2-necked flask was then fitted with an addition funnel, and the solution of NCI in CHCl₃ was transferred to the addition funnel using a cannula. N₂ flow was adjusted through the reactor to a slow and steady rate. The solution of NCI was added continuously drop-wise to the HLBH-pyridine solution at room temperature over a period of ~ 1 hour to form a pre-polymer. Stirring was maintained at a sufficient speed to achieve good mixing of reagents.

In parallel, another oven-dried 50 mL flask was charged with Capstone^{™} AI-62 perfluorinated reagent (5.45 g, 15 mmol). The flask was sealed with rubber septa, degassed for 15 minutes, and purged with N₂. Anhydrous CHCl₃ (17 mL) and anhydrous pyridine (1.9 g, 24 mmol) were added. The mixture was stirred to dissolve all reagents. After the addition of the NCI solution to the 250 mL 2-necked flask was complete, the Capstone^{™} AI-62 perfluorinated reagent solution was added to this flask using a cannula with stirring. The addition funnel was replaced with an air condenser, and the 250-mL 2-necked flask was immersed in an oil bath placed on a heater fitted with a thermocouple unit. The temperature was raised to 50 °C, and the reaction continued at this temperature under N₂ for 24 h.

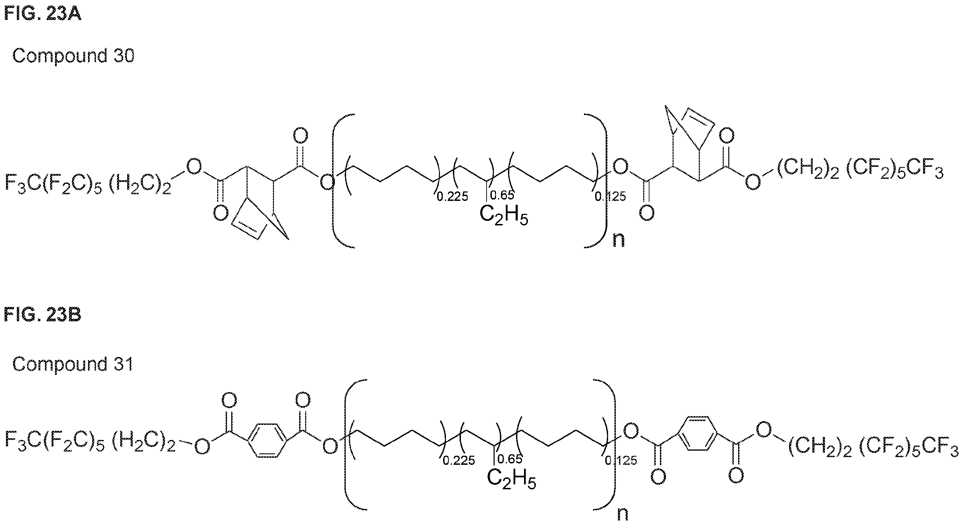

After the reaction, heating and stirring were turned off. The reaction flask was removed, and its contents were poured into a round bottom flask. CHCl₃ was removed by rotary evaporation. Upon concentration, a dense precipitate (pyridine salts) formed. THF was added to dissolve the product, and the precipitated pyridine salts were removed by filtration using a coarse Whatman Filter paper (No 4). Pyridine salts are insoluble in THF. THF was removed by rotary evaporation. The crude product was dissolved in 100 mL of CHCl₃ and was poured into a separatory funnel. 100 mL of water were added, followed by the addition of 5 mL of (5N) HCl to neutralize any remaining pyridine. The funnel was shaken, and the product was extracted into CHCl₃. The bottom CHCl₃ layer containing product was isolated and washed in a separatory funnel with water (5 mL of 5 % NaHCO₃ solution were added to neutralize any remaining HCI). The organic layer was then washed once more with plain distilled water. Isolated CHCl₃ layer was concentrated by rotary evaporation to obtain crude product. The crude product was dissolved in 10 mL of isopropanol (IPA) and was then added dropwise to a beaker containing 200 mL of deionized water containing 1% (v/v) MeOH with continuous stirring. Product separated out as an oil. The mixture was kept in ice bath for 20 minutes, and the top water layer was decanted. The oil was dissolved in THF and transferred into 200 mL round bottom flask. THF was removed by rotary evaporation at a maximum temperature of 80 °C and 4 mbar to remove all residual solvents. The resulting product was dried in a vacuum oven at 60 °C for 24 h to give a purified product as a viscous oil (~55 % yield). The purified product (a mixture of di- and mono-substituted products) was characterized by GPC, elemental analysis,for fluorine, and Hi-Res TGA. Appearance: light yellow viscous liquid. Weight Average molecular weight (polystyrene equivalent) = 12389 g/mol. Polydispersity, PD: 1.43. Elemental analysis: F: 10.6% (theory: 14.08%). The theoretical chemical structure of compound 30 is shown in Figure 23A.

### Compound 31

Compound 31 was prepared according to a procedure similar to compound 30. Glassware used for the synthesis was dried in an oven at 110°C overnight. To a 2-necked 250 mL oven dried round bottom flask equipped with a stir bar was added 15 g (6.0 mmol) of hydrogenated-hydroxyl terminated polybutadiene (HLBH diol, MW = 2000). The flask with the diol was degassed overnight at 60 °C with gentle stirring and then purged with dry N₂ the following day. At this time, the heating was turned off. A 100 mL graduated cylinder was charged with 12 mL anhydrous CHCl₃, sealed by a rubber septa, and purged with dry N₂. The CHCl₃ was transferred to the 2-necked flask via a cannula, and the diol was stirred vigorously to dissolve in the solvent. At this time, anhydrous pyridine (0.95 g, 12 mmol) was added to the HLBH diol solution using a plastic syringe, and the resulting mixture was stirred to dissolve all materials. Another oven dried 2-necked 100 mL flask was charged with terephthaloyl chloride (2.57 g, 13 mmol), sealed with rubber septa, and degassed for 5 minutes, and then purged with nitrogen. At this time, 85 mL of anhydrous CHCl₃ were added using a graduated cylinder and a cannula to transfer the solvent to the 100 mL 2-necked flask. The resulting mixture was stirred to dissolve terephthaloyl chloride. The 250 mL 2-necked flask was then fitted with an addition funnel, and the solution of terephthaloyl chloride in CHCl₃ was transferred to the addition funnel using a cannula. N₂ flow was adjusted through the reactor to a slow and steady rate. The solution of terephthaloyl chloride was added continuously drop-wise to the HLBH-pyridine solution at room temperature over a period of ~ 1 hour to form a pre-polymer. Stirring was maintained at a sufficient speed to achieve good mixing of reagents.

In parallel, another oven-dried 50 mL flask was charged with Capstone^{™} AI-62 perfluorinated reagent (5.45 g, 15 mmol). The flask was sealed with rubber septa, degassed for 15 minutes, and purged with N₂. Anhydrous CHCl₃ (12 mL) and anhydrous pyridine (0.95 g, 12 mmol) were added. The mixture was stirred to dissolve all reagents. After the addition of the terephthaloyl chloride solution to the 250 mL 2-necked flask was complete, the Capstone^{™} AI-62 perfluorinated reagent solution was added to this flask with stirring. The addition funnel was replaced with an air condenser, and the 250-mL 2-necked flask was immersed in an oil bath placed on a heater fitted with a thermocouple unit. The temperature was raised to 50 °C, and the reaction continued at this temperature under N₂ for 24 h.

After the reaction, heating and stirring were turned off. The reaction flask was removed, and its contents were poured into a round bottom flask. CHCl₃ was removed by rotary evaporation. Upon concentration, a dense precipitate (pyridine salts) formed. THF was added to dissolve the product, and the precipitated pyridine salts were removed by filtration using a coarse Whatman Filter paper (No 4). Pyridine salts are insoluble in THF. THF was removed by rotary evaporation. The crude product was dissolved in 100 mL of CHCl₃ and was poured into a separatory funnel. 100 mL of water were added, followed by the addition of 5 mL of (5N) HCI to neutralize any remaining pyridine. The funnel was shaken, and the product was extracted into CHCl₃. The bottom CHCl₃ layer containing product was isolated and washed in a separatory funnel with water (5 mL of 5 % NaHCO₃ solution were added to neutralize any remaining HCI). The organic layer was then washed once more with plain distilled water. Isolated CHCl₃ layer was concentrated by rotary evaporation to obtain crude product. The crude product was dissolved in 10 mL of isopropanol (IPA) and was then added dropwise to a beaker containing 200 mL of deionized water containing 1% (v/v) MeOH with continuous stirring. Product separated out as an oil. The mixture was kept in ice bath for 20 minutes, and the top water layer was decanted. The oil was dissolved in THF and transferred into 200 mL round bottom flask. THF was removed by rotary evaporation at a maximum temperature of 80 °C and 4 mbar to remove all residual solvents. The resulting product was dried in a vacuum oven at 60 °C for 24 h to give a purified product as a viscous oil (~87 % yield). The purified product (a mixture of di- and mono-substituted products) was characterized by GPC, elemental analysis,for fluorine, and Hi-Res TGA. Appearance: off-white viscous liquid. Weight Average molecular weight (polystyrene equivalent) = 10757 g/mol. Polydispersity, PD: 1.33. Elemental analysis: F: 11.29% (theory: 14.21%). The theoretical chemical structure of compound 31 is shown in Figure 23B.

### Compound 32

Glassware used for the synthesis was dried in an oven at 110°C overnight. To a 2- necked 100 mL oven dried round bottom flask equipped with a stir bar was added 10 g (5 mmol) of hydrogenated-hydroxyl terminated polyisoprene (HHTPI diol, MW = 2000). The flask with the diol was degassed overnight at 60 °C with gentle stirring and then purged with dry N₂ the following day. At this time, the heating was turned off. A 100 mL graduated cylinder was charged with 50 mL anhydrous CHCl₃, sealed by a rubber septa, and purged with dry N₂. The CHCl₃ was transferred to the 2-necked flask via a cannula, and the diol was stirred vigorously to dissolve in the solvent. At this time, excess anhydrous pyridine (0.75 g, 9 mmol) was added to the HHTPI diol solution using a plastic syringe, and the resulting mixture was stirred to dissolve all materials. Another oven dried 2-necked 250 mL flask was charged with perfluoroheptanoyl chloride (4.51 g, 12 mmol), sealed with rubber septa, and degassed for 5 minutes, and then purged with nitrogen. At this time, 22 mL of anhydrous CHCl₃ was added using a graduated cylinder and a cannula to transfer the solvent to the 250 mL 2-necked flask containing the perfluoroheptanoyl chloride. The resulting mixture was stirred at room temperature to dissolve the acid chloride. An addition funnel was fitted to this flask, and the HHTPI-pyridine solution in CHCl₃ was added into the addition funnel. N₂ flow was adjusted through the reactor to a slow and steady rate. HHTPI-Pyridine solution was added continuously drop-wise to the acid chloride solution at room temperature over a period of ~ 4 hours. Stirring was maintained at a sufficient speed to achieve good mixing of reagents. After completing addition of the HHTPI diol, the addition funnel was replaced with an air condenser, and the 2-necked flask was immersed in an oil bath on a heater fitted with a thermocouple unit. The temperature was raised to 50 °C, and the reaction continued at this temperature under N₂ for 24 h.

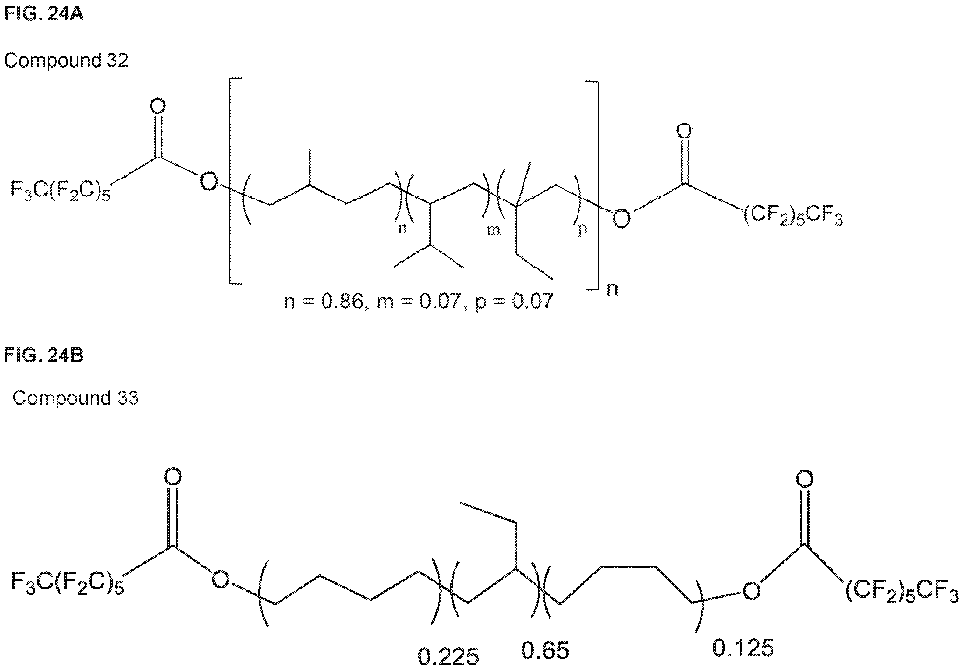

After the reaction, heating and stirring were turned off. The reaction flask was removed, and its contents were poured into a round bottom flask. CHCl₃ was removed by rotary evaporation. Upon concentration, a dense precipitate (pyridine salts) formed. THF was added to dissolve the product, and the precipitated pyridine salts were removed by filtration using a coarse Whatman Filter paper (No 4). Pyridine salts are insoluble in THF. THF was removed by rotary evaporation. The crude product was dissolved in 100 mL of CHCl₃ and was poured into a separatory funnel. 150 mL of water were added, followed by the addition of 5 mL of (5N) HCI to neutralize any remaining pyridine. The funnel was shaken, and the product was extracted into CHCl₃. The bottom CHCl₃ layer containing product was isolated and washed in separatory funnel with water (5 mL of 5 % NaHCO₃ solution were added to neutralize any remaining HCI). The organic layer was then washed once more with plain distilled water. Isolated CHCl₃ layer was concentrated by rotary evaporation to obtain crude product. The crude product was dissolved in 10 mL of isopropanol (IPA) and was added dropwise to a 1L beaker containing 200 mL of deionized water containing 1% (v/v) MeOH with continuous stirring. Product separated out as an oil. The mixture was kept in ice bath for 20 minutes, and the top water layer was decanted. The oil was dissolved in THF and transferred into 200 mL round bottom flask. THF was removed by rotary evaporation at a maximum temperature of 80 °C and 4 mbar to remove all residual solvents. The resulting product was dried in a vacuum oven at 60 °C for 24 h to give a purified product as a colorless viscous oil (~99.9 % yield). The purified product (a mixture of di- and mono-substituted products) was characterized by GPC, elemental analysis,for fluorine, and Hi-Res TGA. Appearance: colorless viscous liquid. Weight Average molecular weight (polystyrene equivalent) = 12622 g/mol. Polydispersity, PD: 1.53. Elemental analysis: F: 13.50% (theory: 17.13%). The theoretical chemical structure of compound 32 is shown in Figure 24A.

### Compound 33

Glassware used for the synthesis was dried in an oven at 110°C overnight. To a 2- necked 1000 mL oven dried round bottom flask equipped with a stir bar was added 100 g (40 mmol) of Hydrogenated-hydroxyl terminated polybutadiene (HLBH diol, MW = 2000). The flask with the diol was degassed overnight at 60 °C with gentle stirring and then purged with dry N₂ the following day. At this time, the heating was turned off. A 1000 mL graduated cylinder was charged with 415 mL anhydrous CHCl₃, sealed by a rubber septa, and purged with dry N₂. The CHCl₃ was transferred to the 2-necked flask via a cannula, and the diol was stirred vigorously to dissolve in the solvent. Now excess anhydrous pyridine (19.08 g, 241 mmol) was added to the HLBH diol solution using a plastic syringe, and the resulting mixture was stirred to dissolve all materials. Another oven dried 2-necked 1000 mL flask was charged with 38.45 g, (101 mmol) perfluoroheptanoyl chloride, sealed with rubber septa, and degassed for 5 minutes, and then purged with nitrogen. At this time, 277 mL of anhydrous CHCl₃ was added using a graduated cylinder and a cannula to transfer the solvent to the 1000 mL 2-necked flask containing the perfluoroheptanoyl chloride. The resulting mixture was stirred at room temperature to dissolve the acid chloride. An addition funnel was fitted to this flask, and the HLBH-pyridine solution in CHCL₃ was added into the addition funnel using a cannula. N₂ flow was adjusted through the reactor to a slow and steady rate. Continuous drop-wise addition of HLBH-Pyridine solution to the acid chloride solution was started at room temperature over a period of ~ 4 hours. Stirring was maintained at a sufficient speed to achieve good mixing of reagents. After completing addition of the HLBH, the addition funnel was replaced with an air condenser, and the 2-necked flask was immersed in an oil bath on a heater fitted with a thermocouple unit. The temperature was raised to 50 °C, and the reaction continued at this temperature under N₂ for 24 h.

After the reaction, heating and stirring were turned off. The reaction flask was removed, and its contents were poured into a round bottom flask. CHCl₃ was removed by rotary evaporation. Upon concentration, a dense precipitate (pyridine salts) formed. THF was added to dissolve the product, and the precipitated pyridine salts were removed by filtration using a coarse Whatman Filter paper (No 4). Pyridine salts are insoluble in THF. THF was removed by rotary evaporation. The crude product was dissolved in 400 mL of CHCl₃ and was poured into a separatory funnel. 500 mL of water were added, followed by the addition of 20 mL of (5N) HCI to neutralize any remaining pyridine. The funnel was shaken, and the product was extracted into CHCl₃. The bottom CHCl₃ layer containing product was isolated, and washed in a separatory funnel with water (20 mL of 5 % NaHCO₃ solution were added to neutralize any remaining HCl). The organic layer was then washed once more with plain distilled water. Isolated CHCl₃ layer was concentrated by rotary evaporation to obtain crude product. The crude product was dissolved in 20 mL of THF and was then added dropwise to a 4 L beaker containing 1200 mL of deionized water containing 1% (v/v) MeOH with continuous stirring. Product separated out as an oil. The mixture was kept in ice bath for 20 minutes, and the top hexane layer was decanted. The oil was dissolved in THF and transferred into 500 mL round bottom flask. THF was removed by rotary evaporation at a maximum temperature of 80 °C and 4 mbar to remove all residual solvents. The resulting product was dried in a vacuum oven at 60 °C for 24 h to give a purified product as a yellow viscous oil (~80 % yield). The purified product (a mixture of di- and mono-substituted products) was characterized by GPC, elemental analysis for fluorine and Hi-Res TGA. Appearance: light yellow viscous liquid. Weight Average molecular weight (polystyrene equivalent) = 6099 g/mol. Polydispersity, PD: 1.08. Elemental analysis: F: 12.84% (theory: 15.54%). The theoretical chemical structure of compound 33 is shown in Figure 24B.

### Compound 34

Glassware used for the synthesis was dried in an oven at 110°C overnight. To a 2- necked 1000 mL oven dried round bottom flask equipped with a stir bar was added 65 g (63 mmol) of YMer-diol (MW = 1000). The flask with the diol was degassed overnight at 60 °C with gentle stirring and then purged with dry N₂ the following day. At this time, heating was turned off. A 1000 mL graduated cylinder was charged with 374 mL anhydrous CHCl₃, sealed by rubber septa, and purged with dry N₂. The CHCl₃ was transferred to the 2-necked flask via a cannula, and the diol was stirred vigorously to dissolve in the solvent. Excess anhydrous pyridine (30 g, 375 mmol) was added to the YMer-diol solution using a plastic syringe, the resulting stir to dissolve all materials. Another oven dried 2-necked 1000 mL flask was charged with 59.82 g (156 mmol) of perfluoroheptanoyl chloride, sealed with rubber septa, and degassed for 5 minutes, then purged with nitrogen. At this time 250 mL of anhydrous CHCl₃ were added using a graduated cylinder and cannula to transfer the solvent to the 1000 mL 2-necked flask containing the perfluoroheptanoyl chloride. The resulting mixture was stirred at room temperature to dissolve the acid chloride. An addition funnel was fitted to this flask and using a cannula transfer the YMer-diol-pyridine solution in CHCl₃ into the addition funnel. N₂ flow through the reactor was adjusted to a slow and steady rate. YMer-diol-pyridine solution was added drop-wise, continuously to the acid chloride solution at room temperature over a period of ~ 4 hours. Stirring was maintained at a sufficient speed to achieve good mixing of reagents. After completing the addition of the YMer-diol-pyridine solution, the addition funnel was replaced with an air condenser, and the 2-necked flask was immersed in an oil bath placed on a heater fitted with a thermocouple unit. The temperature was raised to 40 °C, and the reaction continued at this temperature under N₂ for 24 h.

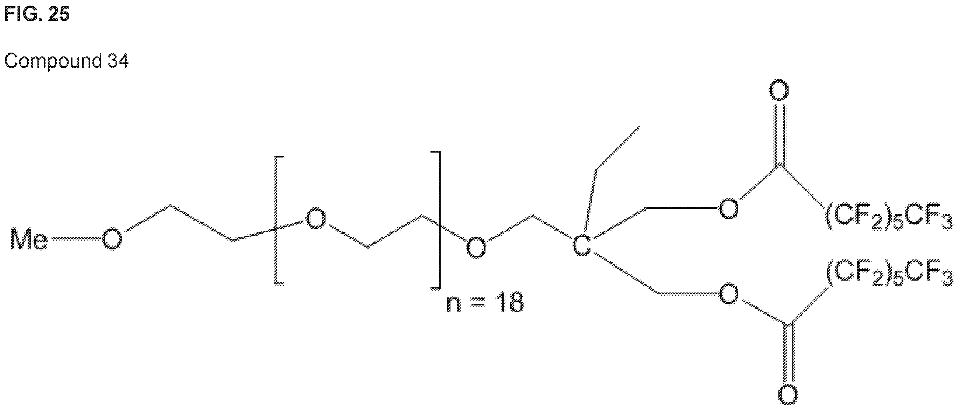

After the reaction, heating and stirring were turned off. The reaction flask was removed, and the contents were poured into a round bottom flask. CHCl₃ was removed by rotary evaporation. Upon concentration, a dense precipitate (pyridine salts) formed. THF was added to dissolve the product. The flask was cooled in an ice bath for 20 minutes, at which time, the precipitated pyridine salts were removed by gravity filtration using a coarse Whatman Filter paper (No 4). Pyridine salts are insoluble in THF. THF was removed by rotary evaporation. The resulting crude product was dissolved in a minimum quantity of Isopropanol (IPA), and this solution was added to 700 mL of hexanes in a beaker with a stir bar. An oil separated out. The top layer was decanted and washed once with 200 mL of hexanes. The residue was then dissolved in 200 mL of THF and transferred to a 500 mL round bottom flask. Rotary evaporation of the solvents at a maximum temperature of 75 °C and 4 mbar vacuum furnished an oil, which was then transferred to a wide mouth jar and further dried for 24 h at 60 °C under vacuum to yield the pure product which solidifies upon cooling at room temperature to an off white waxy semi-solid (Yield 82 %). The purified product was characterized by GPC (Molecular Weight based on Polystyrene Standards), elemental analysis for fluorine, ¹⁹F NMR, ¹H NMR, FTIR and TGA. Appearance: waxy semi-solid. Weight Average molecular weight (polystyrene equivalent) = 2498 g/mol. Polydispersity: 1.04. Elemental Analysis: F: 27.79% (theory: 28.54%). ¹⁹F NMR (CDCl₃, 400 MHz): δ ppm -81.3 (m, CF₃), -118.88 (m, CF₂), -122.37 (m, CF₂), -123.28 (m, CF₂), -126 (m, CF₂). ¹H NMR (CDCl₃, 400 MHz): δ ppm 0.83 (t, **CH₃**CH₂), 1.44 (q, **CH₂**CH₃), 3.34 (m, CH₂), 3.51 (m, CH₂), 3.54 (m, CH₂), 4.30 (m, **CH₂**COO-). FTIR, neat (cm⁻¹): 2882 (CH2), 1783 (O-C=O, ester), 1235, 1203, 1143, 1104 (CF₃, CF₂). The theoretical chemical structure of compound 34 is shown in Figure 25.

### Compound 35

Compound 35 was prepared according to a procedure similar to that used for the preparation of compound 34.

Glassware used for the synthesis was dried in an oven at 110°C overnight. To a 2- necked 1000 mL oven dried round bottom flask equipped with a stir bar was added 60 g (59 mmol) of YMerOH-triol (MW = 1014). The flask with the triol was degassed overnight at 60 °C with gentle stirring and then purged with dry N₂ the following day. Heating was turned off. A 1000 mL graduated cylinder was charged with 435 mL anhydrous CHCl₃, sealed with rubber septa, and purged with dry N₂. The CHCl₃ liquid was transferred to the 2-necked flask via a cannula, and the triol was stirred vigorously to dissolve in the solvent. Excess anhydrous pyridine (37 g, 473 mmol) was added to the YMer-triol solution using a plastic syringe, the resulting mixture was stirred to dissolve all materials. Another oven dried 2-necked 1000 mL flask was charged with 84.88 g (222 mmol) of perfluoroheptanoyl chloride, sealed with rubber septa, and degassed for 5 minutes, then purged with nitrogen. 290 mL of anhydrous CHCl₃ were added using a graduated cylinder and cannula to transfer the solvent to the 1000 mL 2-necked flask containing the perfluoroheptanoyl chloride. The mixture was stirred at room temperature to dissolve the acid chloride. An addition funnel was fitted to this flask, and the YMerOH-triol-pyridine solution in CHCL₃ was transferred to the addition funnel using a cannula. N₂ flow through the reactor was adjusted to a slow and steady rate. YMerOH-triol-pyridine solution was added continuously drop-wise to the acid chloride solution at room temperature over a period of ~ 4 hours. Stirring was maintained at a sufficient speed to achieve good mixing of reagents. After completing the addition of the YMer-triol-pyridine solution, the addition funnel was replaced with an air condenser, and the 2-necked flask was immersed in an oil bath placed on a heater fitted with a thermocouple unit. The temperature was raised to 40 °C, and the reaction was continued at this temperature under N₂ for 24 h.

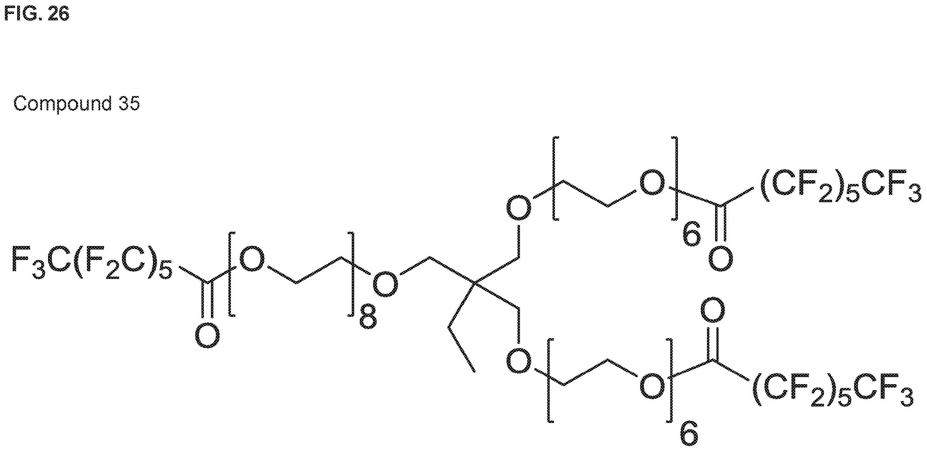

The resulting product was purified in a similar manner to compound **7** described above. The purification involved rotary evaporation of CHCl₃, addition of THF, and separation of the pyridine salts by filtration. The product was then precipated in isopropanol (IPA)/Hexanes, washed as described above for compound **7**, and dried at 75 °C and 4 mbar. Final drying was also done under vacuum at 60 °C for 24 h to yield an oil (Yield 78.2 %). The purified product was characterized by GPC (Molecular Weight based on Polystyrene Standards), elemental analysis for fluorine, ¹⁹F NMR, ¹H NMR, FTIR, and TGA. Appearance: light yellow, viscous oil. Weight Average molecular weight (polystyrene equivalent) = 2321g/mol. Polydispersity: 1.06. Elemental Analysis: F: 35.13% (theory: 36.11%). ¹⁹F NMR (CDCl₃, 400 MHz): δ ppm - 81.30 (m, CF₃), - 118.90 (m, CF₂), -122.27 (m, CF₂), -123.07 (m, CF₂), -126.62 (m, CF₂). ¹H NMR (CDCl₃, 400 MHz): δ ppm 0.83 (t, **CH₃**CH₂), 1.44 (q, **CH₂**CH₃), 3.34 (m, CH₂O), 3.41 (m, CH₂'s), 3.74 (m, CH₂), 4.30 (m, **CH₂**COO-). FTIR, neat (cm⁻¹): 2870 (CH₂), 1780 (O-C=O, ester), 1235, 1202, 1141, 1103 (CF₃, CF₂). The theoretical chemical structure of compound 35 is shown in Figure 26.

### Compound 36

Compound 36 was prepared according to a procedure similar to that used for the preparation of compound 34.

Glassware used for the synthesis was dried in an oven at 110°C overnight. To a 2- necked 1000 mL oven dried round bottom flask equipped with a stir bar was added 50 g (65 mmol) of XMer-Tetraol (MW = 771). The flask with the tetraol was degassed overnight at 60 °C with gentle stirring and then purged with dry N₂ the following day. Heating was turned off. A 1000 mL graduated cylinder was charged with 400 mL anhydrous CHCl₃, sealed with rubber septa, and purged with dry N₂. CHCl₃ was transferred to the 2-necked flask via a cannula, and the tetraol was stirred vigorously to dissolve in the solvent. Excess anhydrous pyridine (51.30 g, 649 mmol) was added to the XMer-Tetraol solution using a plastic syringe, and the resulting mixture was stirred to dissolve all materials. Another oven dried 2-necked 1000 mL flask was charged with 111.63 g (292 mmol) of perfluoroheptanoyl chloride, sealed with rubber septa, and degassed for 5 minutes, and then purged with nitrogen. 300 mL of anhydrous CHCl₃ were added using a graduated cylinder and cannula to transfer the solvent to the 1000 mL 2-necked flask containing perfluoroheptanoyl chloride. The resulting mixture was stirred at room temperature to dissolve the acid chloride. An addition funnel was attached to this flask, and the XMer-tetraol-pyridine solution in CHCL₃ was transferred into the addition funnel via a cannula. N₂ flow through the reactor was adjusted to a slow and steady rate. XMer-tetraol-pyridine solution was added continuously drop-wise to the acid chloride solution at room temperature over a period of ~ 4 hours. Stirring was maintained at a sufficient speed to achieve good mixing of reagents. After completing addition of the XMer-tetraol-pyridine solution, the addition funnel was replaced with an air condenser, and the 2-necked flask was immersed in an oil bath placed on a heater fitted with a thermocouple unit. The temperature was raised to 40 °C, and the reaction continued at this temperature under N₂ for 24 h.

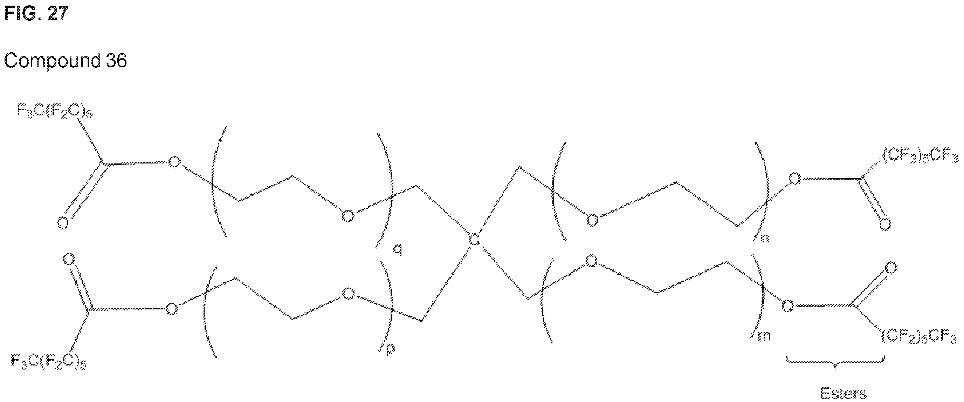

The resulting product was purified in a similar manner to compound 7 described above, where the CHCl₃ was removed by rotary evaporation, addition of THF, and the separation of pyridine salts by filtration after adding THF. The product was then precipitated in isopropanol (IPA)/hexanes, washed as described for compound **7,** and dried at 75 °C and 4 mbar. Final drying was also done under vacuum at 60 °C for 24 h to yield an oil (Yield 80.9 %). The purified product was characterized by GPC (Molecular Weight based on Polystyrene Standards), elemental analysis for fluorine, ¹⁹F NMR, ¹H NMR, FTIR, and TGA. Appearance: light yellow, viscous oil. Weight Average molecular weight (polystyrene equivalent) = 2410 g/mol. Polydispersity: 1.04. Elemental Analysis: F: 44.07% (theory: 45.85%). ¹⁹F NMR (CDCl₃, 400 MHz): δ ppm -81.37 (m, CF₃), -118.89 (m, CF₂), -122.27 (m, CF₂), -123.06 (m, CF₂), -26.64 (m, CF₂). ¹H NMR (CDCl₃, 400 MHz): δ ppm 3.36 (m, CH₂'s), 3.75 (m, CH₂O), 4.39 (m, CH₂O), 4.49 (m, **CH₂**COO-). FTIR, neat (cm⁻¹): 2870 (CH₂), 1780 (O-C=O, ester), 1235, 1202, 1141, 1103 (CF₃, CF₂). Thermal decomposition temperature (TGA), N₂, at *ca.* 10 % (w/w) loss = 327 °C. The theoretical chemical structure of compound 36 is shown in Figure 27.

### Compounds 37 and 38

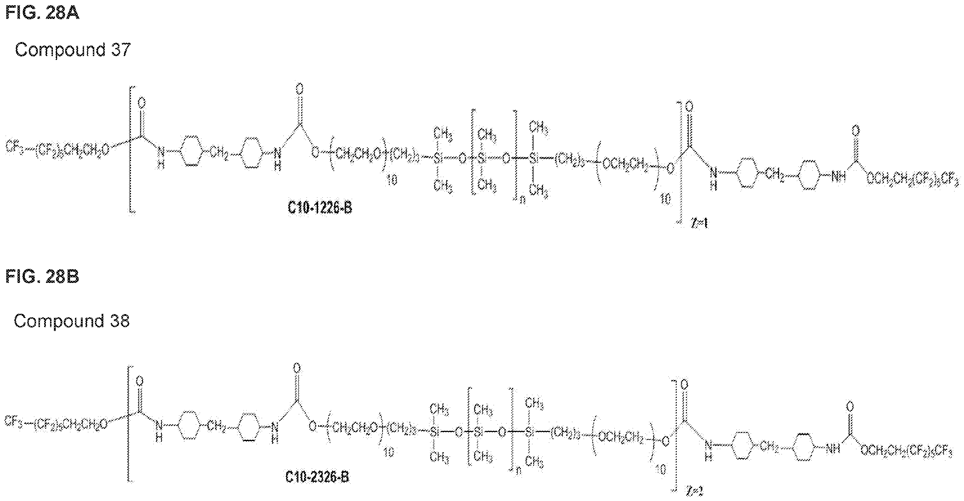

Glassware used for the synthesis was dried in an oven at 110°C overnight. 25.04 g (9.7 mmol) of pegylated polydimethylsiloxane diol (C10-Diol) was weighed out in a 250 mL 2-necked flask, heated to 50 °C, and degassed overnight with stirring. The diol was then purged with nitrogen and dissolved in 25 mL of anhydrous THF. To the resulting mixture was added 36 mg of bismuth carboxylate catalyst in THF (concentration of 0.02 g/mL) followed by a solution of HMDI diisocyanate in THF (5.34 g, 20.4 mmol) which was previously degassed for 30 minutes followed by nitrogen purge. The addition was performed using a syringe. The reaction vessel was fitted with an air condenser, and the mixture was allowed to react at 60 °C with stirring for 4 h. While the pre-polymer reaction was underway, capstone C6-FOH (fluoroalcohol) (8,82 g, 24.2 mmol) was degassed for 15 minutes in a separate flask and then purged with nitrogen. The fluoroalcohol was dissolved in THF, and a further 24 mg of bismuth carboxylate catalyst in THF was added to it. This mixture was then added to the prepolymer reaction vessel via syringe. After the addition was completed, the reaction mixture was allowed to react overnight at 45 °C under a nitrogen atmosphere. After the reaction, the THF solvent was removed on a rotary evaporator, and the crude residue was dissolved in chloroform. The bismuth catalyst residues were extracted using EDTA solution (pH ~ 9). The solution containing EDTA was washed with DI water in a separatory funnel, and the organic layer was concentrated in a rotary evaporator to give the product as an amber viscous liquid. Final drying was done under vacuum at 60 °C for 24 h to yield a viscous oil (Yield 74 %). The purified product was characterized by GPC (Molecular Weight based on Polystyrene Standards), elemental analysis for fluorine, and TGA. Appearance: amber, viscous oil. Weight Average molecular weight (polystyrene equivalent) = 13583 g/mol. Polydispersity: 1.73. Elemental Analysis: F: 12.20% (theory: 12.88%). Thermal decomposition temperature (TGA), N₂, at *ca.* <5 % (w/w) loss = 231 °C. The theoretical chemical structure of compound 37 is shown in Figure 28A.

### Compound 38

Compound 38 is synthesized following a procedure similar to that which was used in the preparation of compound 37. Thus, 25.01 g (9.7 mmol) of C10-Diol was reacted with 4.07 g (15.5 mmol) of HMDI in THF in the presence of Bismuth Carboxylate catalyst to form the prepolymer. The prepolymer was then endcapped with 5.29 g (14.5 mmol) Capstone C6-FOH (fluoroalcohol) to yield the product as a viscous oil (Yield, 59 %). The purified product was characterized by GPC (Molecular Weight based on Polystyrene Standards), elemental analysis for fluorine, and TGA. Appearance: amber, viscous oil. Weight Average molecular weight (polystyrene equivalent) = 19279 g/mol. Polydispersity: 1.79. Elemental Analysis: F: 6.51% (theory: 7.39 %). Thermal decomposition temperature (TGA), N₂, at *ca.* <5 % (w/w) loss = 244 °C. The theoretical chemical structure of compound 38 is shown in Figure 28B.

### Compound 39

Compound 39 was synthesized by a 2-step convergent method according to scheme 2. Briefly, the polyisocyanate desmodur 4470 (11.45 g, 11 mmol) was reacted with capstone C6-FOH (7.65 g, 21 mmol) in anhydrous THF in the presence of Bismuth Carboxylate catalyst at 25 °C for 10 minutes. After the dropwise addition of the fluoroalcohol to the polyisocyanate, stirring was continued for 4 hour at 40°C. These steps lead to the formation of a partially fluorinated intermediate that is then coupled with the PLN8K diol (40 g, 5 mmol) at 70 °C over a period of 14 hours to provide compound 39. Because the reactions are moisture sensitive, they are carried out under an inert atmosphere (N₂) and anhydrous conditions. The temperature profile is also maintained carefully, especially during the partial fluorination, to avoid unwanted side reactions. Over the course of the reaction, the reaction mixture becomes very viscous, and continuous stirring must be maintained to prevent localized heating.

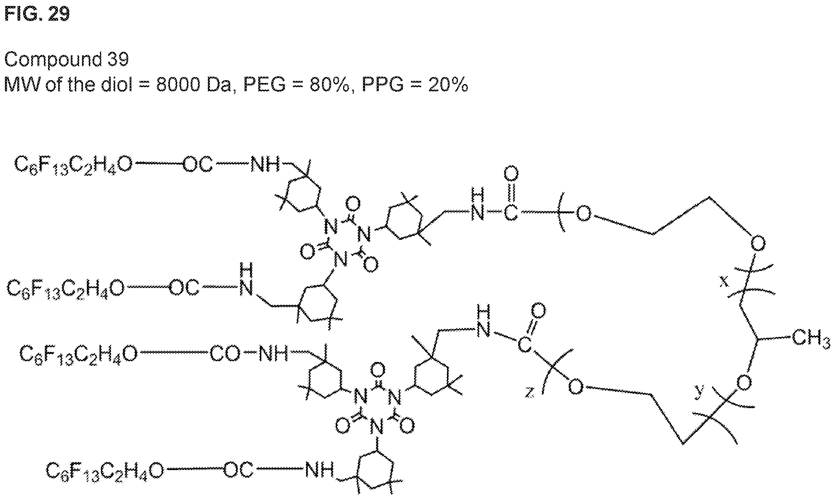

After the reaction, the THF solvent was evaporated on a rotary evaporator to yield the crude product. The product was purified by dissolving in chloroform and adding the EDTA solution (pH ~ 9.0). The mixture was then transferred to a separatory funnel, and the catalyst residues were separated with the aqueous layer. The organic layer was concentrated, and the product was dissolved in isopropanol and precipated in hexanes to yield a white chunky solid which was dried under vacuum (yield: 66 %). The purified product was characterized by GPC (Molecular Weight based on Polystyrene Standards), elemental analysis for fluorine, and TGA. Appearance: White chunky solid. Weight Average molecular weight (polystyrene equivalent) = 31806 g/mol. Polydispersity: 1.32. Elemental Analysis: F: 3.6% (theory: 8.0 %). Thermal decomposition temperature (TGA), N₂, at *ca.* <5 % (w/w) loss = 295 °C. The theoretical chemical structure of compound 39 is shown in Figure 29.

### Compound 40

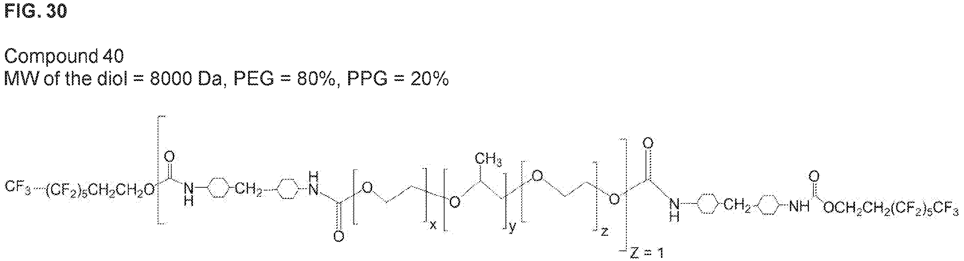

Compound 40 was synthesized following a procedure similar to that which was used in the preparation of compound 37. Thus, 50.0 g (5.7 mmol) of PLN8K diol were reacted with 4.5 g (17.1 mmol) of HMDI in THF in the presence of bismuth carboxylate catalyst to form the prepolymer. The prepolymer was then endcapped with 7.28 g (20 mmol) capstone C6-FOH (fluoroalcohol) to yield the crude product. The EDTA washes to eliminate the catalyst residues were similar. Final purification was performed by dissolving in isopropanol and precipitating with hexanes to yield a white solid (Yield, 86 %). The purified product was characterized by GPC (Molecular Weight based on Polystyrene Standards), elemental analysis for fluorine, and TGA. Appearance: While solid, Weight Average molecular weight (polystyrene equivalent) = 9253 g/mol. Polydispersity: 1.28. Elemental Analysis: F: 3.14% (theory: 4.94 %). Thermal decomposition temperature (TGA), N₂, at *ca.* <5 % (w/w) loss = 303 °C. The theoretical chemical structure of compound 40 is shown in Figure 30.

### Compound 41

Compound 41 was synthesized following a procedure similar to that which was used in the preparation of compound 27. The theoretical chemical structure of compound 41 is shown in Figure 21A, with the exception that the middle triblock copolymer is formed from a C10-Diol.

The purified product was characterized by GPC (Molecular Weight based on Polystyrene Standards), elemental analysis for fluorine, and TGA. Appearance: colorless viscous liquid, Weight Average molecular weight (polystyrene equivalent) = 5858 g/mol. Polydispersity: 1.21. Elemental Analysis: F: 18.39 % (theory: 15.08 %). Thermal decomposition temperature (TGA), N₂, at *ca.* <10 % (w/w) loss = 310 °C.

Examples 2 to 9 containing any of compounds 1 to 36 are only comparative examples not according to the invention.

### Example 2. Preparation of a Semipermeable Biointerface Membrane

A semipermeable biointerface film of the invention may be cast from a liquid mixture. In one example, the liquid mixture is prepared by mixing a dimethylacetamide (DMAc) solution of a biostabilizing additive (e.g., a compound of any one of formulae (I)-(XVII) or any one of compounds 1-40; targeted dry weight percentage of a biostabilizing additive in the final semipermeable biointerface film is from 0.05% (w/w) to 15% (w/w)) with a solution of polyetherurethaneurea (e.g., Chronothane H (Cardiotech International, Inc., Woburn, MA), a higher viscosity polymer solution (e.g., about 30000 cP). To this mixture may be added another polyetherurethaneurea (e.g., Chronothane 1020 (Cardiotech International, Inc., Woburn, MA), a lower viscosity polymer solution (e.g., about 6500 cP). The bowl is then fitted to a planetary mixer with a paddle-type blade and the contents are stirred for 30 minutes at room temperature. Coatings solutions prepared in this manner are then coated at a temperature from room temperature to about 70° C onto a PET release liner using a knife-over-roll set at a gap providing about 40 µm of dry thickness. The film is continuously dried at a temperature from about 120 °C to about 150 °C.

### Example 3. Evaluation of Wettability

The membrane of Example 2 may be tested for wettability by applying a predetermined quantity (e.g., 10 µL) of a fluid (e.g., distilled or deionized water (which may contain a dye for improved visualization) for the assessment of aqueous wettability) to the membrane and measuring the diameter or area of the resulting wet surface after a predetermined dwelling time (e.g., 5 s).

### Example 4. Determination of Glucose Permeability

A commercially available glucose meter with corresponding testing strips may be used to assess glucose permeability of the membrane of Example 2. In this experiment, a membrane of Example 3 may be placed over a testing strip and a predetermined volume of glucose solution (e.g., from 1 µL to 10 µL) may be pushed through the membrane with a pipette. The amount of glucose reaching the strip may be then determined using the appropriate commercially available glucose meter.

### Example 5. Determination of Oxygen Permeability

A commercial oxygen electrode capable of measuring dissolved oxygen may be used in this experiment.

The oxygen probe is immersed in PBS solution, which is purged with nitrogen until a reading of 0% oxygen was obtained. Then, after further nitrogen purging (e.g., for about 2 min), the nitrogen flow is stopped, and the diffusion of the atmospheric oxygen into the PBS solution is recorded using the oxygen meter for a predetermined time period (e.g., 30 min) at predetermined time intervals (e.g., every 10 s) under stirring.

The experiment is then repeated by wrapping the semipermeable biointerface film of the invention (e.g., as produced in Example 3) around the tip of the oxygen probe. All oxygen is then removed from the testing solution by nitrogen purging, and the nitrogen flow is then stopped. The diffusion of the atmospheric oxygen is measured for a predetermined time period (e.g., 30 min) at predetermined time intervals (e.g., every 10 s) under stirring.

The experiments may then be repeated with a reference film that differs from the semipermeable biointerface film used earlier only in that the reference film lacks the biostabilizing additive.

### Example 6. Determination of Hydrogen Peroxide Permeability

Hydrogen peroxide permeability of the semipermeable biointerface films of the invention (e.g., a semipermeable biointerface film of Example 2) may be assessed using procedures known in the art. For example, one approach for the measurement of hydrogen peroxide permeability of a film is described in Vaddiraju et al., Biosensors and Bioelectronics, 24:1557-1562, 2009, the disclosure of the hydrogen peroxide permeability measurement procedure is incorporated herein by reference in its entirety.

### Example 7. BCA Assay for Protein Deposition

A reference film and a semipermeable biointerface film of the invention are prepared (e.g., as described in Example 2) and incubated in protein solutions of varying concentrations. Examples of proteins that may be used in this assay include fibrinogen, albumin, and lysozyme. The concentrations of proteins typically fall within the range from 1 mg/mL to 5 mg/mL. The incubation time is typically from about 2 h to about 3 h. After the incubation is complete, the film samples are rinsed with PBS. Protein adhesion onto the samples may then be quantified using methods known in the art, e.g., a bicinchoninic acid (BCA) assay kit (Pierce, Rockford, IL). Briefly, the samples are incubated in a solution of sodium dodecyl sulfate (SDS) solution for up to about 24 h (with sonication if needed) in order to remove the proteins from the surfaces. A working solution is then prepared using the kit that facilitates the reduction of copper ions and interaction with the BCA. The sample protein solutions are added to the working solution, and the proteins from the sample solutions form a purple complex that is quantifiable using a spectrophotometer at a wavelength of 570 nm. A calibration curve of known protein concentrations is prepared in a similar manner for quantification. Based on the sample surface area, the results are typically reported as µg/cm².

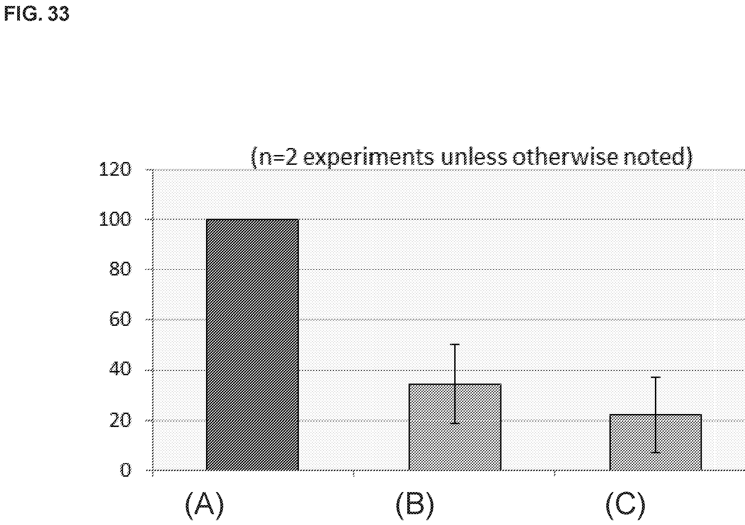

The results for an exemplary BCA assay on carbothane 85A rods prepared with and without biostabilizing additives are provided in Figure 33. The reference rod does not contain a biostabilizing additive. In Figure 33, the column labeled (A) is for the reference rod, the column labeled (B) is for the rod containing 2% (w/w) of compound 16, and the column labeled (C) is for the rod containing 1% (w/w) of compound 41.

### Example 8. Assay for Deposition in Blood

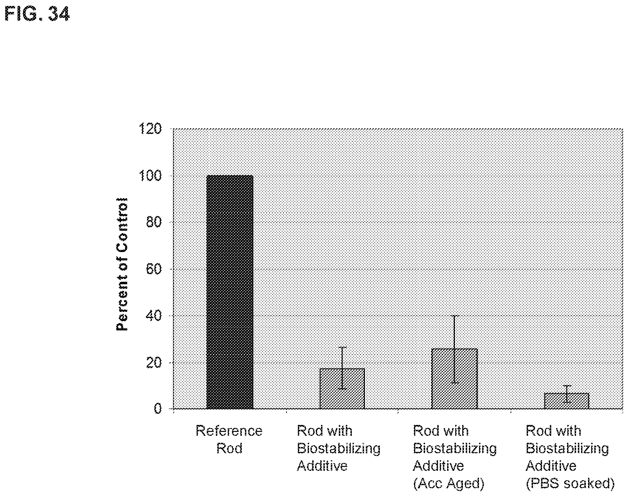

Carbothane polyurethane rods were prepared with and without compound 1. The rod sample prepared without compound 1 was used in this experiment as a reference rod. Fresh bovine blood with a heparin concentration of 0.75 to 1 U/ml was used in a circulating blood loop. To quantify thrombosis on the sample rods or tubes, the autologous platelets were radiolabeled with ¹¹¹In oxyquinoline (oxine) prior to the commencement of the experiment. Rod or tubing samples (15-20 cm) were placed inside a segment of circuit tubing and both ends of the circuit was placed in the blood reservoir. The blood was then circulated at a flow rate of 200 mL/min, and the temperature kept at 37 °C. The blood circulation was maintained for 60 to 120 minutes. When the experiment was terminated, the tubing section containing the sample rods or tubes was detached from the test circuit and rinsed gently with saline. The sample rods or tubes were removed from the tubing and further analyzed for visual and radioactive count. The percentage differences, which normalize the variations in platelet count and the uptake of ¹¹¹In in multiple experiments, are used as indicators of thrombosis. The results of this experiment are shown in Figure 34.

### Example 9. Wettability of Modified Hydrophilic Polyurethane Films.

Hydrophilic polyurethane films can be utilized in continuous glucose monitoring sensors of the invention. Biostabilizing additives can be added to improve the performance of the sensors. This example demonstrates the effect of certain hydrophilic biostabilizing additives on two commercially available hydrophilic base polymer resins, HydroThane^{®} and Tecophilic^{®}, as solvent cast films. The films were evaluated for (i) surface modification using XPS measurements, and (ii) hydration properties using percentage water uptake (also a measure of wettability)
*Film Preparation:* HydroThane^{®} (AL 25 80A from AdvanSource) and Tecophilic^{®} (SP-60D-60 from Lubrizol) were modified with 2 wt. % of a hydrophilic biostabilizing additive selected from compound 37, compound 38, compound 40, compound 39, and compound 22.

Control films were prepared by weighing 2.4 g of base polymer into 40 mL glass vials. To the vial was added 30 g of dimethylacetamide (DMAC) to give solutions of 8% base material (w/w). The solutions were mixed on a shaker at 100 rpm in warm room (37°C) for five days. Films were cast on 7 cm aluminum weighing pans at volumes of 1 - 3 mL, and then dried for 48 hours at 40°C.

Modified films were prepared by weighing 2.4 g of base polymer into 40 mL glass vials. To the vial was added 28 g of dimethylacetamide (DMAC) to each vial. The base polymers were dissolved by shaking overnight at 37°C, then heating in an air-flow oven at 65°C for 72 hours. Biostabilizing additive solutions were prepared by combining 48 mg of each additive in 2 g of DMAC. Each 2 g solution of biostabilizing additive was added to a separate vial containing the base polymer solution. This provided a final mixture of 8% base polymer with respect to solvent and 2% biostabilizing additive with respect to base polymer. The resulting mixtures were heated for a further 48 hours at 65°C. Five films (one for each hydrophilic biostabilizing additive) were cast for each of the base polymers in pre-weighed 7 cm aluminum weighing pans, which were then dried at 37°C for at least 48 hours. After casting and drying the films, selected films were removed from their pans and thicknesses were measured. The thicknesses of the unmodified films were similar between the two base materials and fell between 0.03 and 0.07 mm, depending on the position of the calipers on the film.

*XPS:* The cast films were analyzed by X-Ray Photoelectron Spectroscopy (XPS) to determine the chemical composition of the surface at depths < 10 nm and confirm the presence of the biostabilizing additives. For each biostabilizing additive two films samples were analyzed from 2 different portions. XPS analysis was performed on a Thermo Scientific K-Alpha x-ray photoelectron spectrometer. Survey spectra were performed on a maximum spot size of 400 µm at a take-off angle of 90°.

XPS showed that all films modified with biostabilizing additive, except those of compound 38 had Fluorine on the surface ca. 15-40 atm.% in Tecophilic films and HydroThane films, respectively, indicating the surfaces had been modified. Compound 38 is a silicon based hydrophilic additive that exhibited low surface fluorine, but has high silicon on the surface ca. 8 atm. %, which could be an indication of some form of surface re-orientation of the additive. XPS data suggests all of the additives have migrated and are capable of modifying the polymer surface.

*Hydration Testing:* Before testing, each dried film was weighed in its pan to determine the initial film mass. Fisher 417 filter papers were cut into circles 5 cm in diameter. Each filter paper was saturated with MilliQ water by immersion, then removed using tweezers and shaken gently to remove excess water droplets. The soaked filter papers were then placed on top of each film and exposed for 30 minutes. The wet filter papers were then removed, after which the film was blotted with a dry filter paper to remove any water droplets on the surface, and re-weighed to determine the change in mass. This procedure was repeated twice for each of three films in each sample group.

The results of the film hydration tests are summarized in Table 1. Each hydration value was the average of six measurements completed on three films. The exceptions were: Hydrothane + compound 37 (four out of six measurements included), Tecophilic + compound 22 (five out of six measurements included), and Tecophilic + compound 39 (five out of six measurements included). In all three of these cases, the excluded measurements were statistical outliers.

**Table 1**

| **Base Material** | **Endexo Formulation** | **Hydration (%)** |
|---|---|---|
| Hydrothane | Control | 12.7±0.8 |
| | Compound 37 | 12±2 |
| | Compound 22* | 16.1±0.7 |
| | Compound 39 | 16.1±0.6 |
| | Compound 40 | 15±4 |
| | Compound 38 | 16±2 |
| Tecophilic | Control | 23±2 |
| | Compound 37 | 25±2 |
| | Compound 22* | 28±2 |
| | Compound 39 | 21±3 |
| | Compound 40 | 24±3 |
| | Compound 38 | 29±2 |

| | | |
|---|---|---|
| * = comparative example | | |

Control HydroThane^{®} and Tecophilic^{®} exhibited a water uptake of ca. 13 % and 23 %, respectively, as measured. The hydration data of modified films indicated no significant change in the hydration properties in the control base polymers with addition of 2 wt % biostabilizing additive. Thus, the biostabilizing additives preserved hydrophilicity, and wettability, of the hydrophilic base polymers. Importantly, materials modified with biostabilizing additive can exhibit reduced cell and/or protein deposition without significantly reducing the hydrophilic nature of the base polymer used to make the material.

## Claims

1. A compound of formula (XX):
F_{T}-[B-A]ₙ-B-F_{T} (XX)
wherein
(i) A comprises 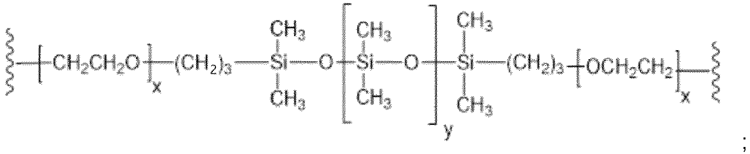
(ii) B is a segment including a urethane formed from 4,4'-methylene bis(cyclohexyl isocyanate);
(iii) F_{T} is a polyfluoroorgano group; and
(iv) x is an integer from 8 to 12, y is an integer from 6-9, and n is an integer from 1 to 10, optionally wherein n is 1 or 2;
a compound of formula (XXI):
F_{T}-[B-A]ₙ-B-F_{T} (XXI)
wherein
(i) A comprises a segment having the formula: 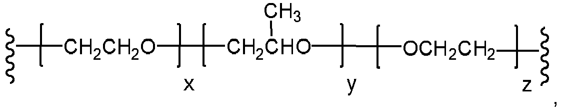 wherein said segment has a MW of 7,000 to 9,000 Da, comprises from 75% to 85% (w/w) polyethylene oxide, and comprises 15% to 25% (w/w) polypropylene oxide;
(ii) B is a segment including a urethane formed from 4,4'-methylene bis(cyclohexyl isocyanate);
(iii) F_{T} is a polyfluoroorgano group; and
(iv) n is an integer from 1 to 10,
optionally wherein n is 1 or 2; or
a compound of formula (XXII): 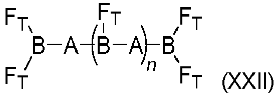 wherein
(i) A comprises a segment having the formula: 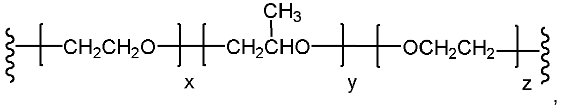 wherein said segment has a MW of 7,000 to 9,000 Da, comprises from 75% to 85% (w/w) polyethylene oxide, and comprises 15% to 25% (w/w) polypropylene oxide;
(ii) B is a segment including an isocyanurate trimer or biuret trimer formed from isophorone diisocyanate (IPDI) trimer;
(iii) F_{T} is a polyfluoroorgano group; and
(iv) n is an integer from 0 to 10.

2. The compound of claim 1, wherein the compound is the compound of formula (XX).

3. The compound of claim 1, wherein the compound is the compound of formula (XXI).

4. The compound of claim 1, wherein the compound is the compound of formula (XXII).

5. The compound of any one of claims 1-4, wherein F_{T} is selected from the group consisting of radicals of the general formula CHₘF₍₃₋ₘ₎(CF₂)ᵣCH₂CH₂- and CHₘF₍₃₋ₘ₎(CF₂)ₛ(CH₂CH₂O)_{χ}-, wherein
m is 0, 1, 2, or 3; optionally wherein m is 0 or 1;
χ is an integer between 1-10;
r is an integer between 2-20; and
s is an integer between 1-20;
optionally wherein said compound has a theoretical molecular weight of less than 40,000 Da.

6. An implantable glucose sensor comprising: a glucose detector and an enclosure defining a boundary between an internal space and an external space, said enclosure comprising a semipermeable biointerface film comprising a base polymer and a biostabilizing additive comprising the compound of any one of claims 1-5;
wherein said semipermeable biointerface film has a biostable surface and is permeable to glucose; wherein said glucose detector is disposed inside said internal space, and said biostable surface faces said external space or both said internal space and said external space.

7. The implantable glucose sensor of claim 6, wherein
(a) said semipermeable biointerface film has a thickness of from 1 to 1000 microns; and/or
(b) said semipermeable biointerface film comprises from 0.05% (w/w) to 15% (w/w) of said biostabilizing additive.

8. The implantable glucose sensor of claim 6 or 7, wherein
(a) said base polymer is a silicone, polyolefin, polyester, polycarbonate, polysulfone, polyamide, polyether, polyurea, polyurethane, polyetherimide, or cellulosic polymer, or a copolymer thereof or a blend thereof; or
(b) said base polymer is a silicone, polycarbonate, polypropylene (PP), polyvinylchloride (PVC), polyvinyl alcohol (PVA), polyvinylpyrrolidone (PVP), polyacrylamide (PAAM), polyethylene oxide, polyethylene oxide)-*b*-poly(propylene oxide)-*b*-poly(ethylene oxide), poly(hydroxyethylmethacrylate) (polyHEMA), polyethylene terephthalate (PET), polybutylene terephthalate (PBT), polymethylmethacrylate (PMMA), polyether ether ketone (PEEK), polyamide, polyurethane, cellulosic polymer, polysulfone, or a copolymer thereof or a blend thereof, optionally wherein said base polymer is a thermoplastic.

9. The implantable glucose sensor of claim 8, wherein said base polymer is polyvinylpyrrolidone (PVP), polyacrylamide (PAAM), polyethylene oxide, polyethylene oxide)-*b*-poly(propylene oxide)-*b*-poly(ethylene oxide), poly(hydroxyethylmethacrylate) (polyHEMA), polyether-*b*-polyamide, or polyurethane.

10. The implantable glucose sensor of any one of claims 6-9, wherein said biostabilizing additive is a hydrophilic biostabilizing additive and/or a fluorinated biostabilizing additive.

11. The implantable glucose sensor of any one of claims 6-10, wherein said semipermeable biointerface film further comprises one or more biologically active agents selected from the group consisting of anti-inflammatory agents, anti-infective agents, anesthetics, inflammatory agents, growth factors, angiogenic factors, growth factors, immunosuppressive agents, antiplatelet agents, anticoagulants, ACE inhibitors, cytotoxic agents, anti-sense molecules, and mixtures thereof.

12. The implantable glucose sensor of any one of claims 6-11, wherein said implantable glucose sensor is an implantable electrochemical glucose sensor, and wherein said glucose detector is a working electrode; optionally wherein said semipermeable biointerface film has a biostable surface and is permeable to oxygen.

13. The implantable glucose sensor of claim 12, further comprising a glucose-oxidizing enzyme layer disposed between said working electrode and said semipermeable biointerface film.

14. The implantable glucose sensor of any one of claims 6-11, wherein said implantable glucose sensor is an implantable optical glucose sensor, and wherein said glucose detector is a glucose recognition element comprising a glucose-binding fluorophore.

15. The implantable glucose sensor of any one of claims 6-11, wherein said semipermeable biointerface film is
(a) a bilayer film comprising a biointerface coating and a membrane, wherein said biointerface coating comprises said biostable surface, and wherein said biointerface coating comprises said biostabilizing additive;
optionally wherein said biointerface coating comprises said base polymer; wherein said membrane comprises a second base polymer that is same or different as said base polymer in said coating; and/or
wherein said membrane comprises a biostabilizing additive;
or
(b) a monolayer membrane comprising said base polymer and said biostabilizing additive.

## Patentansprüche

1. Verbindung mit der Formel (XX):
F_{T}-[B-A]ₙ-B-F_{T} (XX)
wobei
(i) A Folgendes umfasst: 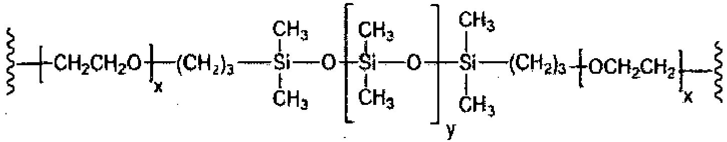
(ii) B ein Segment ist, das ein Urethan einschließt, welches aus 4,4'-Methylenbis(cyclohexylisocyanat) gebildet ist;
(iii) F_{T} eine Polyfluororganogruppe ist und
(iv) x eine ganze Zahl von 8 bis 12 ist, y eine ganze Zahl von 6 bis 9 ist, und n eine ganze Zahl von 1 bis 10 ist, wobei n gegebenenfalls 1 oder 2 ist;
Verbindung mit der Formel (XXI):
F_{T}-[B-A]ₙ-B-F_{T} (XXI)
wobei
(i) A ein Segment mit der folgenden Formel umfasst: 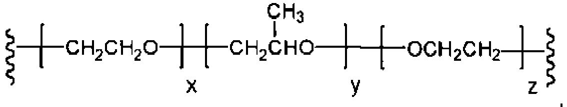 wobei das Segment ein MW von 7000 bis 9000 Da hat, 75 % bis 85 % (Gew./Gew.) Polyethylenoxid umfasst und 15 % bis 25 % (Gew./ Gew.) Polypropylenoxid umfasst;
(ii) B ein Segment ist, das ein Urethan einschließt, welches aus 4,4'-Methylenbis(cyclohexylisocyanat) gebildet ist;
(iii) F_{T} eine Polyfluororganogruppe ist; und
(iv) n eine ganze Zahl von 1 bis 10 ist,
wobei gegebenenfalls n 1 oder 2 ist; oder Verbindung mit der Formel (XXII): 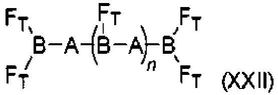 wobei
(i) A ein Segment mit der folgenden Formel umfasst: 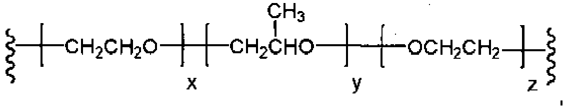 wobei das Segment ein MW von 7000 bis 9000 Da hat, 75 % bis 85 % (Gew./Gew.) Polyethylenoxid umfasst und 15 % bis 25 % (Gew./ Gew.) Polypropylenoxid umfasst;
(ii) B ein Segment ist, das ein Isocyanurattrimer oder Biurettrimer einschließt, das aus Isophorondiisocyanat- (IPDI)-Trimer gebildet ist;
(iii) F_{T} eine Polyfluororganogruppe ist und
(iv) n eine ganze Zahl von 0 bis 10 ist.

2. Verbindung nach Anspruch 1, wobei die Verbindung die Verbindung mit der Formel (XX) ist.

3. Verbindung nach Anspruch 1, wobei die Verbindung die Verbindung mit der Formel (XXI) ist.

4. Verbindung nach Anspruch 1, wobei die Verbindung die Verbindung mit der Formel (XXII) ist.

5. Verbindung nach einem der Ansprüche 1 bis 4, wobei F_{T} ausgewählt ist aus der Gruppe bestehend aus Resten mit der allgemeinen Formel CHₘF₍₃₋ₘ₎(CF₂)ᵣCH₂CH₂- und CHₘF₍₃₋ₘ₎(CF₂)ₛ(CH₂CH₂O)_{X}-, wobei
m 0, 1, 2 oder 3 ist; wobei m gegebenenfalls 0 oder 1 ist;
X eine ganze Zahl zwischen 1 und 10 ist;
r eine ganze Zahl zwischen 2 und 20 ist; und
s eine ganze Zahl zwischen 1 und 20 ist;
wobei die Verbindung gegebenenfalls ein theoretisches Molekulargewicht kleiner als 40.000 Da hat.

6. Implantierbarer Glucosesensor, umfassend: einen Glucosedetektor und eine Einhausung, die eine Grenze zwischen einem Innenraum und einem Außenraum definiert, wobei die Einhausung einen semipermeablen Biogrenzflächenfilm (Biointerfacefilm) umfasst, der ein Basispolymer und ein biostabilisierendes Additiv umfasst, das die Verbindung gemäß einem der Ansprüche 1 bis 5 umfasst;
wobei der semipermeable Biointerfacefilm eine biostabile Oberfläche aufweist und für Glucose permeabel ist; wobei der Glucosedetektor innerhalb des Innenraums angeordnet ist und die biostabile Oberfläche zu dem Außenraum oder sowohl dem Innenraum als auch dem Außenraum weist.

7. Implantierbarer Glucosesensor nach Anspruch 6, wobei
(a) der semipermeable Biointerfacefilm eine Dicke von 1 bis 1000 Mikrometern hat; und/oder
(b) der semipermeable Biointerfacefilm 0,05 % (Gew./ Gew.) bis 15 % (Gew./Gew.) des biostabilisierenden Additivs umfasst.

8. Implantierbarer Glucosesensor nach Anspruch 6 oder 7, wobei
(a) das Basispolymer ein Silikon, Polyolefin, Polyester, Polycarbonat, Polysulfon, Polyamid, Polyether, Polyharnstoff, Polyurethan, Polyetherimid oder cellulosisches Polymer oder ein Copolymer davon oder ein Gemisch davon ist; oder
(b) das Basispolymer ein Silikon, Polycarbonat, Polypropylen (PP), Polyvinylchlorid (PVC), Polyvinylalkohol (PVA), Polyvinylpyrrolidon (PVP), Polyacrylamid (PAAM), Polyethylenoxid, Poly(ethylenoxid)-*b*-poly(propylenoxid)-*b*-poly(ethylenoxid), Poly(hydroxyethylmethacrylat) (polyHEMA), Polyethylenterephthalat (PET), Polybutylenterephthalat (PBT), Polymethylmethacrylat (PMMA), Polyetheretherketon (PEEK), Polyamid, Polyurethan, cellulosisches Polymer, Polysulfon oder ein Copolymer davon oder ein Gemisch davon ist, wobei das Basispolymer gegebenenfalls ein Thermoplast ist.

9. Implantierbarer Glucosesensor nach Anspruch 8, wobei das Basispolymer Polyvinylpyrrolidon (PVP), Polyacrylamid (PAAM), Polyethylenoxid, Poly(ethylenoxid)-*b*-poly(propylenoxid)-*b*-poly(ethylenoxid), Poly(hydroxyethylmethacrylat) (polyHEMA), Polyether-*b*-polyamid oder Polyurethan ist.

10. Implantierbarer Glucosesensor nach einem der Ansprüche 6 bis 9, wobei das biostabilisierende Additiv ein hydrophiles biostabilisierendes Additiv und/oder ein fluoriertes biostabilisierendes Additiv ist.

11. Implantierbarer Glucosesensor nach einem der Ansprüche 6 bis 10, wobei der semipermeable Biointerfacefilm des Weiteren ein oder mehrere biologisch aktive Mittel ausgewählt aus der Gruppe bestehend aus antientzündlichen Mitteln, Antiinfektiva, Anästhetika, entzündungsauslösenden Mitteln, Wachstumsfaktoren, angiogenen Faktoren, Wachstumsfaktoren, Immunosuppressiva, Thrombozytenaggregationshemmern, Antikoagulantien, ACE-Inhibitoren, zytotoxischen Mitteln, Antisense-Molekülen und Mischungen davon umfasst.

12. Implantierbarer Glucosesensor nach einem der Ansprüche 6 bis 11, wobei der implantierbare Glucosesensor ein implantierbarer elektrochemischer Glucosesensor ist, und wobei der Glucosedetektor eine Arbeitselektrode ist; wobei der semipermeable Biointerfacefilm eine biostabile Oberfläche hat und sauerstoffpermeabel ist.

13. Implantierbarer Glucosesensor nach Anspruch 12, des Weiteren umfassend eine Glucose oxidierende Enzymschicht, die zwischen der Arbeitselektrode und dem semipermeablen Biointerfacefilm angeordnet ist.

14. Implantierbarer Glucosesensor nach einem der Ansprüche 6 bis 11, wobei der implantierbare Glucosesensor ein implantierbarer optischer Glucosesensor ist, und wobei der Glucosedetektor ein Glucoseerkennungselement ist, das ein Glucose bindendes Fluorophor umfasst.

15. Implantierbarer Glucosesensor nach einem der Ansprüche 6 bis 11, wobei der semipermeable Biointerfacefilm wie folgt ist:
(a) ein Zweischichtfilm, umfassend eine Biointerfacebeschichtung und eine Membran, wobei die Biointerfacebeschichtung die biostabile Oberfläche umfasst, und wobei die Biointerfacebeschichtung das biostabilisierende Additiv umfasst;
wobei die Biointerfacebeschichtung gegebenenfalls das Basispolymer umfasst; wobei die Membran ein zweites Basispolymer umfasst, welches das gleiche wie das Basispolymer in der Beschichtung oder von diesem verschieden ist; und/oder
wobei die Membran ein biostabilisierendes Additiv umfasst; oder
(b) eine Monoschichtmembran, umfassend das Basispolymer und das biostabilisierende Additiv.

## Revendications

1. Composé de formule (XX) :
F_{T}-[B-A]ₙ-B-F_{T} (XX)
(i) A comprenant 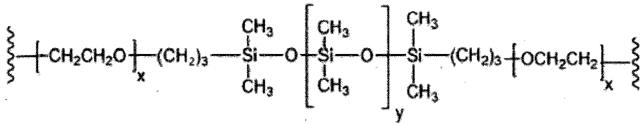 ;
(ii) B étant un segment comprenant un uréthane formé à partir de bis(isocyanate de cyclohexyle)-4,4'-méthylène ;
(iii) F_{T} étant un groupe polyfluoroorgano ; et
(iv) x étant un entier de 8 à 12, y étant un entier de 6 à 9, et n étant un entier de 1 à 10, éventuellement, n étant 1 ou 2 ;
composé de formule (XXI) :
F_{T}-[B-A]ₙ-B-F_{T} (XXI)
(i) A comprenant un segment ayant la formule : 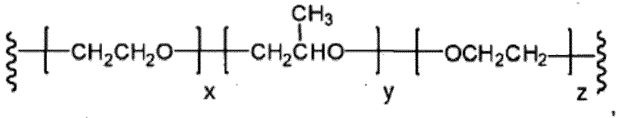 ledit segment ayant un MW de 7 000 à 9 000 Da, comprenant de 75 % à 85 % (p/p) de poly(oxyde d'éthylène), et comprenant 15 % à 25 % (p/p) de poly(oxyde de propylène) ;
(ii) B étant un segment comprenant un uréthane formé à partir de bis(isocyanate de cyclohexyle)-4,4'-méthylène ;
(iii) F_{T} étant un groupe polyfluoroorgano ; et
(iv) n étant un entier de 1 à 10,
éventuellement, n étant 1 ou 2 ; ou composé de formule (XXII) : 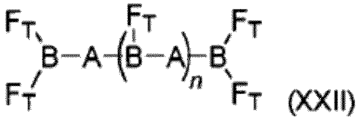
(i) A comprenant un segment ayant la formule : 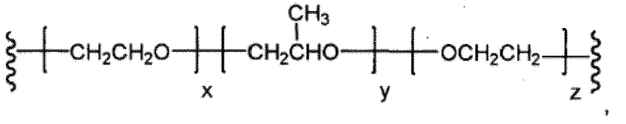 ledit segment ayant un MW de 7 000 à 9 000 Da, comprenant de 75 % à 85 % (p/p) de poly(oxyde d'éthylène), et comprenant 15 % à 25 % (p/p) de poly(oxyde de propylène) ;
(ii) B étant un segment comprenant un trimère d'isocyanurate ou un trimère de biuret formé à partir d'un trimère de diisocyanate d'isophorone (IPDI) ;
(iii) F_{T} étant un groupe polyfluoroorgano ; et
(iv) n étant un entier de 0 à 10.

2. Composé selon la revendication 1, le composé étant le composé de formule (XX).

3. Composé selon la revendication 1, le composé étant le composé de formule (XXI).

4. Composé selon la revendication 1, le composé étant le composé de formule (XXII).

5. Composé selon l'une quelconque des revendications 1 à 4, F_{T} étant choisi dans le groupe constitué par des radicaux de la formule générale 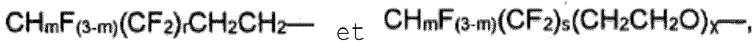
m étant 0, 1, 2 ou 3 ; éventuellement m étant 0 ou 1 ;
X étant un entier entre 1 et 10 ;
r étant un entier entre 2 et 20 ; et
s étant un entier entre 1 et 20 ;
éventuellement, ledit composé ayant un poids moléculaire théorique de moins de 40 000 Da.

6. Capteur de glucose implantable comprenant : un détecteur de glucose et un boîtier définissant une limite entre un espace interne et un espace externe, ledit boîtier comprenant un film de biointerface semi-perméable comprenant un polymère de base et un additif biostabilisant comprenant le composé selon l'une quelconque des revendications 1 à 5 ;
ledit film de biointerface semi-perméable ayant une surface biostable et étant perméable au glucose ; ledit détecteur de glucose étant disposé à l'intérieur dudit espace interne, et ladite surface biostable faisant face audit espace externe ou à la fois audit espace interne et audit espace externe.

7. Capteur de glucose implantable selon la revendication 6,
(a) ledit film de biointerface semi-perméable ayant une épaisseur allant de 1 à 1 000 microns ; et/ou
(b) ledit film de biointerface semi-perméable comprenant de 0,05 % (p/p) à 15 % (p/p) dudit additif biostabilisant.

8. Capteur de glucose implantable selon la revendication 6 ou 7,
(a) ledit polymère de base étant une silicone, une polyoléfine, un polyester, un polycarbonate, une polysulfone, un polyamide, un polyéther, une polyurée, un polyuréthane, un polyétherimide, ou un polymère cellulosique, ou un copolymère correspondant ou un mélange correspondant ; ou
(b) ledit polymère de base étant une silicone, un polycarbonate, un polypropylène (PP), un poly(chlorure de vinyle) (PVC), un poly(alcool vinylique) (PVA), une polyvinylpyrrolidone (PVP), un polyacrylamide (PAAM), un poly(oxyde d'éthylène), un poly(oxyde d'éthylène)-*b-*poly(oxyde de propylène)-*b*-poly(oxyde d'éthylène), un poly(méthacrylate d'hydroxyéthyle) (polyHEMA), un poly(téréphtalate d'éthylène) (PET), un poly(téréphtalate de butylène) (PBT), un poly(méthacrylate de méthyle) (PMMA), une polyétheréthercétone (PEEK), un polyamide, un polyuréthane, un polymère cellulosique, une polysulfone, ou un copolymère correspondant ou un mélange correspondant, éventuellement, ledit polymère de base étant un thermoplastique.

9. Capteur de glucose implantable selon la revendication 8, ledit polymère de base étant une polyvinylpyrrolidone (PVP), un polyacrylamide (PAAM), un poly(oxyde d'éthylène), un poly(oxyde d'éthylène)-*b*-poly(oxyde de propylène)-*b*-poly(oxyde d'éthylène), un poly(méthacrylate d'hydroxyéthyle) (polyHEMA), un polyéther-*b*-polyamide ou un polyuréthane.

10. Capteur de glucose implantable selon l'une quelconque des revendications 6 à 9, ledit additif biostabilisant étant un additif biostabilisant hydrophile et/ou un additif biostabilisant fluoré.

11. Capteur de glucose implantable selon l'une quelconque des revendications 6 à 10, ledit film de biointerface semi-perméable comprenant en outre un ou plusieurs agents biologiquement actifs choisis dans le groupe constitué par des agents anti-inflammatoires, des agents antiinfectieux, des anesthésiques, des agents inflammatoires, des facteurs de croissance, des facteurs angiogéniques, des facteurs de croissance, des agents immunosuppresseurs, des agents antiplaquettaires, des anticoagulants, des inhibiteurs d'ACE, des agents cytotoxiques, des molécules antisens et des mélanges correspondants.

12. Capteur de glucose implantable selon l'une quelconque des revendications 6 à 11, ledit capteur de glucose implantable étant un capteur de glucose électrochimique implantable, et ledit détecteur de glucose étant une électrode de travail ; éventuellement ledit film de biointerface semi-perméable ayant une surface biostable et étant perméable à l'oxygène.

13. Capteur de glucose implantable selon la revendication 12, comprenant en outre une couche d'enzyme d'oxydation de glucose disposée entre ladite électrode de travail et ledit film de biointerface semi-perméable.

14. Capteur de glucose implantable selon l'une quelconque des revendications 6 à 11, ledit capteur de glucose implantable étant un capteur de glucose optique implantable, et ledit détecteur de glucose étant un élément de reconnaissance de glucose comprenant un fluorophore liant le glucose.

15. Capteur de glucose implantable selon l'une quelconque des revendications 6 à 11, ledit film de biointerface semi-perméable étant
(a) un film bicouche comprenant un revêtement de biointerface et une membrane, ledit revêtement de biointerface comprenant ladite surface biostable, et ledit revêtement de biointerface comprenant ledit additif biostabilisant ; éventuellement, ledit revêtement de biointerface comprenant ledit polymère de base ; ladite membrane comprenant un deuxième polymère de base qui est identique ou différent dudit polymère de base dans ledit revêtement ; et/ou
ladite membrane comprenant un additif biostabilisant ;
ou
(b) une membrane monocouche comprenant ledit polymère de base et ledit additif biostabilisant.
